# EUROPEAN PATENT APPLICATION

(11) **EP 4 358 088 A1**
(43) Date of publication of application: **24.04.2024**
(21) Application number: 22825081.7
(22) Date of filing: 17.06.2022
(51) Int. Cl.: G16H 10/40

(54) **INFORMATION PROCESSING METHOD AND INFORMATION PROCESSING SYSTEM**

(30) Priority: 17.06.2021 JP 2021100752; 30.07.2021 JP 2021125242; 29.11.2021 JP 2021193353
(71) Applicant: TOPPAN INC., Tokyo 110-0016 (JP)
(72) Inventor: KATSUMATA, Masaru, Tokyo 110-0016 (JP); MIYATA, Kenichi, Tokyo 110-0016 (JP); YOSHIMURA, Yuki, Tokyo 110-0016 (JP)
(74) Representative: Plasseraud IP
(86) International application number: PCT/JP2022/024284
(87) International publication number: WO 2022/265093

(57) **Abstract**

An information processing method is an information processing system that includes a test piece, a first device, and a second device communicably connected with the first device and processes information concerning a test using the test piece. The test piece develops a color depending on whether a test object is present. The first device transmits individual identification information provided to the test piece and a captured digital image including a coloring area to a second device. The second device determines whether the test object is present and stores the digital image in a state of being associated with the individual identification information.

## Description

### [Technical Field]

The present invention relates to an information processing method and an information processing system.

The present application claims the benefit of priority from earlier Japanese Patent Applications No. 2021-100752 filed with the Japan Patent Office on June 17, 2021, No. 2021-125242 filed with the Japan Patent Office on July 30, 2021, and No. 2021-193353 filed with the Japan Patent Office on November 29, 2021, all of the descriptions of which are incorporated herein by reference.

### [Background Art]

Remarkable advances in medical science have led to the development of excellent therapeutic drugs and advanced medical technologies. However, due to various factors such as diet and living environment, human beings are constantly exposed to a wide variety of diseases such cancer, autoimmune diseases, infectious diseases, and circulatory diseases. Specifically, regarding infectious diseases, increased movement of persons and goods causes epidemics not only within a country but also on a global scale, which enormously influences social and economic activity. Hence, it is required to bring the infectious diseases under control promptly.

The novel coronavirus is still fresh in our memories. When a novel infectious disease that has never been experienced before spreads, it is difficult to take multifaceted and comprehensive measures such as prevention, treatment, and control of the spread of the disease, which makes it extremely difficult to suppress the increase in the number of infected persons. Vaccination is one of the most effective ways to prevent infection. However, for a novel infectious disease, it is required to start vaccine development, which requires time. In addition, it is assumed that time is required for the development and confirmation of efficacy of therapeutic drugs for infectious diseases.

Hence, prevention measures against the spread of the disease are extremely important in the early stages of the infection. For example, regarding the novel coronavirus, increase in the number of infected persons thereof causes a strain on medical institutions, which increases the load on medical personnel. As a result, treatment for patients with infectious diseases and patients with other diseases may be difficult. Thus, it is required to take reliable prevention measures against the spread, to suppress the increase in the number of patients and reduce the time that medical personnel are required to spend treating patients with infectious diseases.

In order to handle patients at medical institutions, first, examination and diagnosis are required. However, in the case of patients suspected of a novel infectious disease, the preparation time tends to increase, as medical personnel must wear personal protective equipment (PPE) to prevent infection themselves. Due to the increase in such preparation time, the burden of face-to-face examination and diagnosis at medical institutions tends to increase. Considering such a situation, relaxation of regulations concerning telemedicine is progressing, and approaches to further reduce the load on medical personnel are also progressing.

However, telemedicine still has problems. For example, although clinical test reagents can be used as materials for determining whether an infection is present, in the case of telemedicine, it is difficult for medical personnel to directly test a patient using the clinical test reagents. As a measure against this problem, a method of mailing a patient's specimen and performing an accurate clinical test at a specialized medical institution can be considered. However, since physical distribution requires time, it takes time until determination results of the test are available, and prompt diagnosis cannot be performed.

As a conventional technique, for example, a technique has been disclosed in which a patient themself uses dedicated testing equipment to test for the presence of pathogens (e.g., refer to Patent Literature 1). In addition, as methods that can perform testing relatively easily, test strips (test piece) and immunochromatography devices are known (e.g., refer to Patent Literature 2).

In addition, from the viewpoint of preventing infectious diseases from spreading while maintaining social activity, at an event site or the like, there is a trend to require presentation of information for proving that they are not infected with an infectious disease at an entrance of the site. For example, Patent Literature 3 discloses a technique for storing test results for a plurality of test items in a database in a state in which the test results are associated with user identification information, and outputting information concerning the test results read from the database.

In addition, there is a system that performs testing using images captured by a user (e.g., refer to Patent Literature 4). In such a system, the user supplies a specimen (e.g., saliva or the like) to a test strip to perform testing. The test strip develops a color depending on whether the supplied specimen includes a test object such as a virus. The user transmits an image of the tested test strip to a remote server from a smartphone or the like. The server subjects the received image to image processing to determine whether the specimen includes the test object.

### [Citation List]

### [Patent Literature]

Patent Literature 1: JP-A-2020-080083
Patent Literature 2: JP-A-2009-133813
Patent Literature 3: JP-A-2021-7000
Patent Literature 4: JP-T-2019-520548

### [Summary of the invention]

### [Technical Problem]

In order to suppress increase in the number of patients and reduce time required for medical personnel to treat patients with infectious diseases, the above techniques in Patent Literatures 1 to 4 have been disclosed. Since the above systems may cause a hindrance to the user, there is room for improvement in the following points.

That is, in the systems in Patent Literatures 1 and 2, since dedicated testing equipment is required to be prepared, application thereof to telemedicine is not realistic. This because it is unlikely that people in general will have the dedicated testing equipment. Even if the people have the equipment, expertise and effort are required to appropriately manage and operate complicated testing equipment, which is not suitable for public use. Hence, there is difficulty in obtaining widespread adoption of dedicated testing equipment by people in general. In addition, in a clinical test, determination of the presence or absence of infection based on test results should not be based on the patient's judgment, but on a reliable method.

Thus, the result of determination of presence or absence of infection or the like based on test results is desired to be shared more rapidly and easily by doctors and the like. That is, it is required to determine the presence or absence of infection by an even more reliable method and ensure that hospital beds and medical systems are available, to prevent freedom of social and economic activity from being limited.

In contrast, the result of the determination of presence or absence of infection or the like is highly confidential information. Hence, how to keep such highly confidential information secret is an important viewpoint.

In addition, in Patent Literature 3, identification information and test results for a user are stored in a database in a state in which they are associated with each other. Hence, information indicating, for example, persons who are positive may be leaked from the database, so there is a problem that it cannot necessarily be said to be highly secure.

That is, if personal information is leaked, it may cause a hindrance to the user.

In addition, in Patent Literature 4, a user captures images in various environments (imaging environments). For example, images are captured in environments in which color resolutions of imaging devices of respective users, lighting units that light subjects (test strips), relative positional relationships among the subjects, the lighting units, and the imaging devices are different. Verification conducted by the inventors found that, in different environments, different determination results may be obtained even when images of the same subject are captured. It is desirable that, even in different imaging environments, the same determination result is obtained if images of the same subject are captured. That is, if the same determination result cannot be obtained though images of the same subject are captured, accuracy in the determination result becomes lowered, which may cause a hindrance to the user.

From the above, the inventors found a problem that it is required to establish a method by which when a test is performed using images captured by a user, even if the images are captured in different imaging environments, if the images include the same subject, a determination is made with high accuracy so that the same determination result can be obtained.

The present invention has been made in light of such circumstances as stated above and has for its object the provision of an information processing method and an information processing system that can perform testing in a manner having high reliability by a mode that is difficult to leak information having high confidentiality.

Furthermore, the present invention has for its object the provision of an information processing system and an information processing method that can increase safety of data when data concerning a test is utilized.

Furthermore, the present invention has for its object the provision of an information processing method and an information processing system that can judge, when a determination is made using an image captured by a user, images captured in various imaging environments with high accuracy.

### [Solution to Problem]

An information processing method according to the present invention is performed by an information processing system including a test piece, a first device, and a second device communicably connected with the first device and processes information concerning a test using the test piece. The method includes: a coloring step of applying individual identification information for identifying an individual of the test piece to the test piece, and supplying a biological sample, which is collected from a tested person, to the test piece, to develop a color depending on whether the biological sample includes a test object; an imaging step of capturing, by the first device, a digital image including at least an area colored with the color of the test piece in a state of being colored with the color; a first communication step of transmitting, by the first device, the digital image and the individual identification information to the second device communicably connected with the first device; a determination step of determining, by the second device, whether color information indicating the color in the digital image has the test object, based on the digital image received from the first device and determination criterion information for determining presence or absence of the test object; and a storage control step of storing, by the second device, the digital image received from the first device in a state of being associated with the individual identification information.

An information processing system according to the present invention includes a test piece, a first device, and a second device communicably connected with the first device and processes information concerning a test using the test piece. The test piece has a coloring unit that, when a biological sample collected from a tested person is supplied to the test piece, develops a color depending on whether the biological sample includes a test object. The test piece is applied with individual identification information for identifying an individual of the test piece. The first device has: an imaging unit that captures a digital image including at least an area colored with the color of the test piece in a state of being colored with the color; and a first communication unit that transmits the digital image and the individual identification information to an information processing server. The second device has: a determination unit that determines whether color information indicating the color in the digital image has the test object, based on the digital image received from the first device and determination criterion information for determining presence or absence of the test object; and a storage control unit that causes the digital image received from the first device to be stored in a stated of being associated with the individual identification information.

An information processing system according to the present invention includes a test terminal, a user terminal, and an information processing server and processes information concerning a test using a test piece for testing whether a biological sample includes a test object. The test terminal has: a first acquisition unit that acquires individual identification information for identifying an individual of the test piece used for a test of a tested person and test information indicating a test result of the tested person; and a registration unit that transmits the individual identification information and the test information acquired by the first acquisition unit to the information processing server. The user terminal has: a second acquisition unit that acquires the individual identification information of the test piece used for the test of the tested person and user information concerning the tested person; and a user registration unit that transmits the individual identification information and the user information acquired by the second acquisition unit to the information processing server. The information processing server has a registration control unit that causes a test result database to store information in which the test information is associated with the individual identification information based on the information received from the test terminal, and stores information in which the user information is associated with the individual identification information in a user database based on the information received from the user terminal.

An information processing method according to the present invention is an information processing system that includes a test terminal, a user terminal, and an information processing server and processes information concerning a test using a test piece for testing whether a biological sample includes a test object. The test terminal acquires individual identification information for identifying an individual of the test piece used for a test of a tested person and test information indicating a test result of the tested person, and transmits the acquired individual identification information and test information to the information processing server. The user terminal acquires the individual identification information of the test piece used for the test of the tested person and user information concerning the tested person, and transmits the acquired individual identification information and user information to the information processing server. The information processing server causes a test result database to store information in which the test information is associated with the individual identification information based on the information received from the test terminal, and stores information in which the user information is associated with the individual identification information in a user database based on the information received from the user terminal.

An information processing method according to the present invention is performed by an information processing system that processes information concerning a test using a test piece that develops a color depending on whether a specimen includes a test object. The method includes: capturing, by an imaging unit, an image of the test piece that is a subject so as to include a coloring area, which is colored with the color, of the test piece; and determining, by a determination unit, whether the image captured by the imaging unit indicates a positive result indicating that the coloring area has the test object, by using a determination model. The determination model is a learned model that learns a correspondence relationship between an image and the positive by performing machine learning using a training data set, which is obtained by appending a sign, which indicates whether the coloring area in a training image, which has been captured so as to include the coloring area in the unspecified test piece, indicates the positive, to the training image, and that estimates a degree of positive indication in an input image based on the correspondence relationship, to output a result of the estimation. The training image includes a plurality of images of the same subject captured in different imaging environments.

An information processing system according to the present invention processes information concerning a test using a test piece that develops a color depending on whether a specimen includes a test object. The system includes: an imaging unit that captures an image of the test piece that is a subject so as to include a coloring area, which is colored with the color, of the test piece; and a determination unit that determines whether the image captured by the imaging unit indicates a positive result indicating that the coloring area has the test object, by using a determination model. The determination model is a learned model that learns a correspondence relationship between an image and the positive by performing machine learning using a training data set, which is obtained by appending a sign, which indicates whether the coloring area in a training image, which has been captured so as to include the coloring area in the unspecified test piece, indicates the positive, to the training image, and that estimates a degree of positive indication in an input image based on the correspondence relationship, to output a result of the estimation. The training image includes a plurality of images of the same subject captured in different imaging environments.

### [Advantageous Effects of the Invention]

According to the present invention, a test using a manner having high reliability can be performed by an aspect that is difficult to leak information having high confidentiality.

Furthermore, according to the present invention, safety of data can be increased when a test result is utilized. Specifically, a tested person who has undergone a test and the test result thereof are stored in separate databases. Hence, the test result and a person cannot be easily associated with each other, whereby privacy of the tested person can be protected.

Furthermore, according to the present invention, when a determination is made using an image captured by a user, images captured in various imaging environments can be determined with high accuracy.

### [Brief Description of the Drawings]

FIG. 1 is a block diagram illustrating a configuration example of an information processing system according to an embodiment;
FIG. 2 is a diagram illustrating an example of a test strip according to the embodiment;
FIG. 3 is a block diagram illustrating a configuration example of a user terminal according to the embodiment;
FIG. 4 is a block diagram illustrating a configuration example of an information processing server according to the embodiment;
FIG. 5 is a block diagram illustrating a configuration example of an institution server according to the embodiment;
FIG. 6 is a diagram illustrating an example of individual information according to the embodiment;
FIG. 7 is a diagram illustrating an example of tested person information according to the embodiment;
FIG. 8 is a diagram illustrating an example of test result information according to the embodiment;
FIG. 9 is a sequence diagram illustrating a flow of processing performed by the information processing system according to the embodiment;
FIG. 10 is a sequence diagram illustrating a flow of processing performed by the information processing system according to a first modification of the embodiment;
FIG. 11 is a sequence diagram illustrating a flow of processing performed by the information processing system according to a second modification of the embodiment;
FIG. 12 is a block diagram illustrating a configuration example of an information processing system according to the embodiment;
FIG. 13 is a sequence diagram illustrating a flow of processing performed by the information processing system according to the embodiment;
FIG. 14 is a sequence diagram illustrating a flow of processing performed by the information processing system according to the embodiment;
FIG. 15 is a diagram illustrating an example of a test strip according to the embodiment;
FIG. 16 is a block diagram illustrating a configuration example of a test terminal according to the embodiment;
FIG. 17 is a block diagram illustrating a configuration example of an information processing server according to the embodiment;
FIG. 18 is a block diagram illustrating a configuration example of a user terminal according to the embodiment;
FIG. 19 is a block diagram illustrating a configuration example of an event site terminal according to the embodiment;
FIG. 20 is a diagram illustrating an example of a test according to the embodiment;
FIG. 21 is a diagram illustrating an example of test results according to the embodiment;
FIG. 22 is a diagram illustrating an example of users according to the embodiment;
FIG. 23 is a diagram illustrating an example of an entry condition information unit according to the embodiment;
FIG. 24 is a diagram for describing processing performed by the test terminal according to the embodiment;
FIG. 25 is a diagram for describing the processing performed by the test terminal according to the embodiment;
FIG. 26 is a diagram for describing the processing performed by the test terminal according to the embodiment;
FIG. 27 is a diagram for describing the processing performed by the test terminal according to the embodiment;
FIG. 28 is a diagram for describing the processing performed by the test terminal according to the embodiment;
FIG. 29 is a diagram for describing the processing performed by the test terminal according to the embodiment;
FIG. 30 is a diagram for describing the processing performed by the test terminal according to the embodiment;
FIG. 31 is a diagram for describing processing performed by the user terminal according to the embodiment;
FIG. 32 is a diagram for describing the processing performed by the user terminal according to the embodiment;
FIG. 33 is a diagram for describing the processing performed by the user terminal according to the embodiment;
FIG. 34 is a diagram for describing the processing performed by the user terminal according to the embodiment;
FIG. 35 is a diagram for describing the processing performed by the user terminal according to the embodiment;
FIG. 36 is a block diagram illustrating a configuration example of an information processing system according to the embodiment;
FIG. 37 is a diagram illustrating an example of a test strip according to the embodiment;
FIG. 38 is a block diagram illustrating a configuration example of a user terminal according to the embodiment;
FIG. 39 is a block diagram illustrating a configuration example of an information processing server according to the embodiment;
FIG. 40 is a sequence diagram illustrating a flow of processing performed by the information processing system according to the embodiment;
FIG. 41 is a sequence diagram illustrating a flow of processing performed by the information processing system according to a first modification of the embodiment;
FIG. 42 is a sequence diagram illustrating a flow of processing performed by the information processing system according to a second modification of the embodiment;
FIG. 43 is a sequence diagram illustrating a flow of processing performed by the information processing system according to a third modification of the embodiment; and
FIG. 44 is a sequence diagram illustrating a flow of processing performed by the information processing system according to a fourth modification of the embodiment.

### [Description of the Embodiments]

Hereinafter, embodiments of the present invention will be described with reference to the drawings.

### <Regarding information processing system 1>

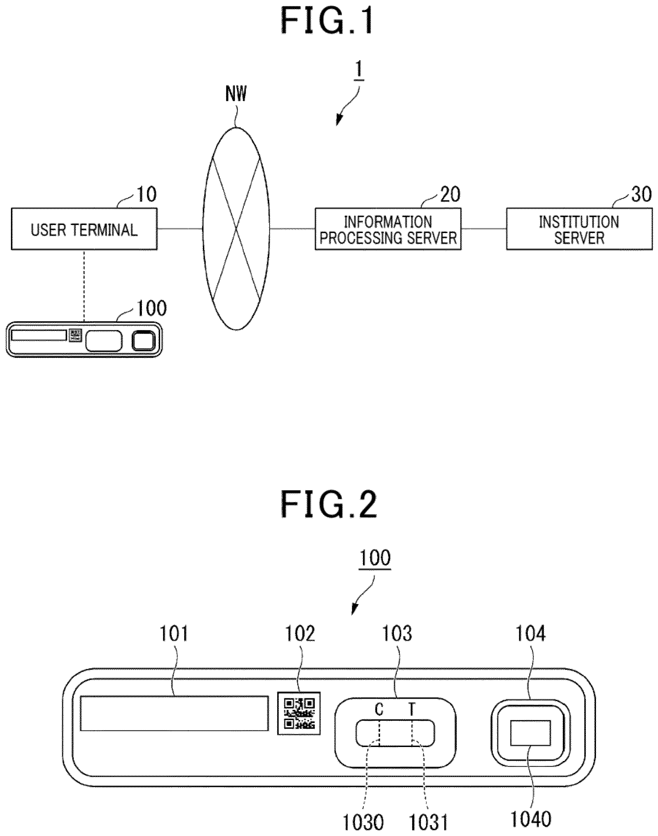

FIG. 1 is a block diagram illustrating a configuration example of an information processing system 1 according to an embodiment. The information processing system 1 includes, for example, a test strip 100, a user terminal 10, an information processing server 20, and an institution server 30. The user terminal 10 and the information processing server 20 are communicably connected via a communication network NW. The information processing server 20 and the institution server 30 are communicably connected via a dedicated line or a universal line.

In order to transmit and receive information, the communication network NW, and the dedicated line or the universal line may use a LAN (Local Area Network), a WAN (Wide Area Network), a telephone network (mobile telephone network, fixed-line telephone network, or the like), a regional IP (Internet Protocol) network, the Internet, or the like.

In the information processing system 1 according to the present embodiment, a test using the test strip 100 is performed by a tested person. For example, the test is performed by dropping a biological sample (mucus or the like) from the tested person onto a supply part 104 (refer to FIG. 2) of the test strip 100. The test strip 100 is configured, for example, so that when the biological sample includes a test object (specific virus or the like), a line having a specific color appears in a coloring part 103 (refer to FIG. 2). The tested person captures an image of the post-test test strip 100. The user terminal 10 transmits an image of the post-test test strip 100 (referred to as a test image G) to the information processing server 20. The information processing server 20 analyzes the test image G to determine whether the test strip 100 indicates that the biological sample includes the test object. The information processing server 20 informs the user terminal 10 of the determination result. The determination of whether the test strip indicates that the biological sample includes the test object may be quantitatively performed.

In the information processing system 1, determination results or the like from the information processing server 20 may be applied to telemedicine via a system other than the present system. For example, the information processing server 20 may transmit the test image G and the determination results to the institution server 30.

For example, when the institution server 30 is a server of a medical institution, if the tested person wishes to be diagnosed by a doctor or if the doctor has requested the test image G of the tested person or the like, the information processing server 20 transmits the test image G and the determination result to the institution server 30. In this case, the institution server 30 displays the received test image G and determination result so that the doctor can perform a diagnosis. The doctor views the displayed image to perform a diagnosis and inputs a diagnostic result to the institution server 30. In this case, for example, the institution server 30 informs the information processing server 20 of the diagnostic result. The information processing server 20 informs the user terminal 10 of the determination result together with the diagnostic result. Alternatively, the institution server 30 may directly inform the user terminal 10 of the diagnostic result. In this case, although the information processing server 20 transmits the test image G and the determination result to the institution server 30, the information processing server 20 does not receive the diagnostic result from the institution server 30.

### <Regarding test strip 100>

The test strip 100 is a test piece serving as a constituent element of a clinical test reagent. The clinical test reagent is used for verifying presence or absence of a disease. For example, a lateral flow assay (lateral flow method), an ELISPOT method, or the like is used to determine the presence or absence of a test object for diagnosing presence or absence of the disease. The lateral flow assay to which immunity assay is applied may be specifically referred to as immunochromatography.

FIG. 2 is a diagram illustrating an example of the test strip 100 according to the embodiment. FIG. 2 illustrates an example of a test strip used in a lateral flow assay. The test strip 100 includes, for example, a name tag 101, a two-dimensional code 102, a coloring part 103, and a supply part 104.

The name tag 101 is a column in which the full name or the like of a tested person is written. When a group medical test is performed in a hospital or the like, information such as the full name of the tested person is written on the name tag 101 with a pen, or a sticker on which the information such as the full name of the tested person is printed is stuck on the name tag 101, by, for example, medical personnel such as a nurse or a laboratory technician. Hence, the identity of the tested person whose biological body has been tested by the test strip 100 is clarified, whereby the risk of confusion in the group medical test is reduced.

The two-dimensional code 102 is a two-dimensional code image in which individual identification information IDA of the test strip 100 is embedded. The two-dimensional code may be, for example, a QR code (registered trademark) or a bar code. The individual identification information IDA is information for uniquely identifying the test strip 100, for example, information indicating a manufacturing number of the test strip 100.

In the present embodiment, a test using the test strip 100 is performed by the tested person themself, and a captured image of the test strip 100 after the test (hereinafter, referred to as a test image G) is transmitted to the information processing server 20. In the present embodiment, from the viewpoint of protecting personal information of the tested person, an image in which the full name or the like of the tested person is not written on the name tag 101 is used as the test image G. Alternatively, the individual identification information IDA or the like embedded in the two-dimensional code 102 may be used to perform management so as not to cause confusion between test strips 100. A method of managing the test strips 100 by using the individual identification information IDA or the like will be described in detail later.

The coloring part 103 is an area on which a result of a color reaction of the test strip 100 is displayed. The color reaction is a chemical reaction that causes change in color or color development when the biological sample includes the test object. The coloring part 103 includes, for example, a control line 1030 and a test line 1031. The control line 1030 is an area in which a line appears when the biological sample supplied to the supply part 104 described later migrates to the coloring part 103 normally. The test line 1031 is an area in which a line appears when a virus, an antibody, or the like (test object) to be targeted is present in the biological sample.

The supply part 104 is an area to which the biological sample is supplied. The supply part 104 includes, for example, a biological sample supply window 1040. For example, dropping the biological sample from the tested person onto the biological sample supply window 1040 supplies the biological sample to the supply part 104.

It is noted that the clinical test reagents may include a specimen collection jig for collecting a specimen, an extraction liquid, a detection reagent, a correction indicator, and the like.

### (Regarding biological sample)

The biological sample in the present embodiment is a specimen collected from a tested person, for example, mucus collected by rubbing a throat using a cotton swab or the like. The biological sample is desirably a liquid, for example, peripheral blood, blood serum, blood plasma, ascites fluid, urine, cerebrospinal fluid, sputum, saliva, bone marrow, synovial fluid, aqueous humor, amniotic fluid, earwax, mother milk, bronchoalveolar lavage fluid, semen, prostatic fluid, Cowper's fluid or pre-ejaculation fluid, sweat, feces, hair, tears, cyst fluid, pleural fluid or ascites, pericardial fluid, lymph, chyme, chyle, bile, interstitial fluid, menstrual secretion, pus, sebum, vomit, vaginal secretion, secretion from mucous membranes, watery stools, pancreatic fluid, secretions from the nasal cavity, secretions from the pharynx, nasal cavity swab fluid, pharynx swab fluid, fluid from nasal irrigation, bronchoalveolar aspirates, blastocyst cavity fluid, umbilical cord blood, or the like, and includes a substance serving as an indicator when diseases are diagnosed.

### (Regarding test object)

The test object in the present embodiment is a substance to be tested with a clinical test reagent. For example, the test object may be a cell, a bacterium, a virus, an exosome, a nucleic acid, polypeptide (including an antigen and an antibody), polynucleotide, lipid, phospholipids, carbohydrates, polysaccharide, glycoprotein, a low molecular weight compound, a metabolite from a cell or a bacterium, or a single substance (object) such as a fragment of a bacterium, a virus, or an exosome, or a compound substance (object) thereof.

### (Regarding individual identification information IDA)

The individual identification information IDA in the present embodiment is information that can uniquely specify at least the test strip 100. For example, the individual identification information IDA is information that indicates a manufacturing number (serial number) or the like. The two-dimensional code 102 may include, in addition to the individual identification information IDA, information describing the test strip 100 ("test piece description information"). The information describing the test strip 100 is information that indicates, for example, a manufacturer, a test object, diseases corresponding to the test object, disease items thereof, and the like.

### (Regarding diseases)

The diseases are illnesses such as, as representative classifications, cancer, hypertension, diabetes, heart disease, cerebrovascular disease, neuropsychiatric disease, immunological/allergy disease, and infectious disease.

### (Regarding disease items)

Disease items further subdivide the above diseases and include a cause, a metabolite, and a phenomenon of the disease. For example, the disease items include coronavirus, influenza, adenovirus, RS virus, rotavirus, hepatitis B virus, hepatitis C virus, HIV, herpesvirus, norovirus, human metapneumovirus, group A beta haemolytic streptococcus, helicobacter pylori, treponema pallidum, mycoplasma, clostridium difficile, mycobacterium, escherichia coli 0157, escherichia coli verocytotoxin, pneumococcus, legionella, procalcitonin, chlamydia, gonococcus, allergic conjunctivitis, interstitial-cell-stimulating hormone (LH), human chorionic gonadotropin (HCG), BNP, NT-proBNP, CK-MB, myoglobin, troponin, D-dimer, H-FABP, granulocyte elastase, carcinoembryonic antigen (CEA), fecal occult blood, insulin-like growth factor-binding protein, fFN, allergy test, CRP, and anti-CCP antibodies.

### (Regarding user terminal 10)

The user terminal 10 is a computer device of the tested person (user) and is operated by the user. The user may be a patient subjected to telemedicine. The user terminal 10 is implemented by a smartphone, a tablet terminal, a cellular phone, a PC (Personal Computer), or the like. In the user terminal 10, for example, an application program for telemedicine (hereinafter, telemedicine application) is installed. The telemedicine application is a program for implementing telemedicine by the information processing system 1.

It is noted that, as a matter of course, functions corresponding to the telemedicine application may be implemented by a Web browser. In this case, the user terminal 10 accesses a site specified by a predetermined URL (Uniform Resource Locator) or the like for performing telemedicine via the Web browser. Then, operations concerning telemedicine are performed according to guidance on a screen provided from the accessed site.

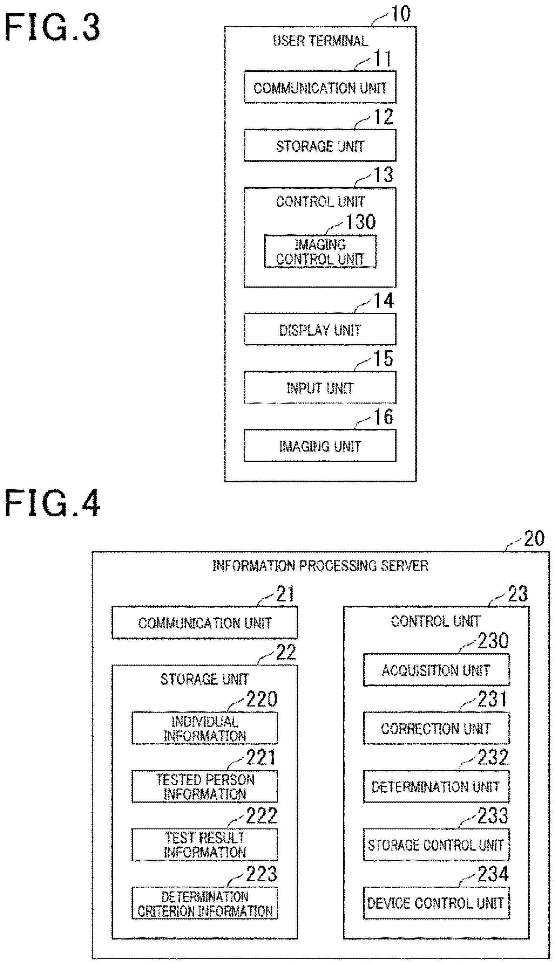

FIG. 3 is a block diagram illustrating a configuration example of the user terminal 10 according to the embodiment. The user terminal 10 includes, for example, a communication unit 11, a storage unit 12, a control unit 13, a display unit 14, an input unit 15, and an imaging unit 16. The communication unit 11 communicates with the information processing server 20.

The storage unit 12 is configured by a storage medium such as an HDD (Hard Disk Drive), a flash memory, an EEPROM (Electrically Erasable Programmable Read Only Memory), a RAM (Random Access read/write Memory) and, a ROM (Read Only Memory) or a combination thereof. The storage unit 12 stores a program for performing various processes of the user terminal 10 (e.g., a program concerning the telemedicine application) and temporary data used when the various processes are performed.

The display unit 14 includes, for example, a display device such as a liquid crystal display and displays images according to control performed by the control unit 13. The input unit 15 includes, for example, an input device such as a mouse and a keyboard and outputs information input by operation by the user such as the tested person to the control unit 13. The imaging unit 16 includes, for example, an imaging device such as a digital camera and captures images according to control performed by the control unit 13.

The control unit 13 is implemented by causing a CPU (Central Processing Unit), included in the user terminal 10 as hardware, to execute the program. The control unit 13 performs overall control of the user terminal 10. The control unit 13 controls, for example, the display unit 14 and the imaging unit 16 according to the program concerning the telemedicine application.

The control unit 13 performs processes according to the program of the telemedicine application. The control unit 13 starts the telemedicine application, for example, in response to an input operation by the user such as the tested person, for example, when an icon of the telemedicine application is tapped. Hence, for example, an operation screen of the telemedicine application is displayed on the display unit 14. On the operation screen, for example, a plurality of operation buttons which are labelled "Capture test image G", "Input account information", and the like are displayed. When "Capture test image G" is tapped by the user, the control unit 13 activates the imaging unit 16 to cause the imaging unit 16 to display an imaging screen for capturing the test image G.

The control unit 13 includes, for example, an imaging control unit 130. The imaging control unit 130 controls imaging when capturing the test image G. Specifically, when the test image G is captured, the imaging control unit 130 causes an image for supporting the imaging to be displayed. For example, the imaging control unit 130 causes the display unit 14 to display a guide representing an outline of the test strip 100. Hence, orientations and sizes of the test strips 100 in the test images G can be made to coincide with each other, whereby the information processing server 20 can analyze images with high accuracy.

In addition, when attempting to capture an image of the test strip 100, the imaging control unit 130 may determine whether there is an obstruction within the angle of view. The obstruction is an object present between the user terminal 10 and the test strip 100 and is an object hiding the test strip 100 when an image is captured. If an obscuring object is present, for example, the imaging control unit 130 causes the display unit 14 to display a warning. After capturing an image of the test strip 100, the imaging control unit 130 may determine whether there is an obstruction in the image. If there is an obstruction, for example, the imaging control unit 130 causes the display unit 14 to display a message prompting capture of the image again. In addition, after capturing an image of the test strip 100, even when color information is so biased that the captured image cannot be corrected by the method described later (at least one of color correction and angular correction), for example, the imaging control unit 130 may similarly cause the display unit 14 to display a message prompting capture of the image again.

If the test image G is captured, the control unit 13 causes operation buttons, which are labelled "Send image", "Capture image gain", and the like, to be displayed. If "Send image" is tapped by the user, the control unit 13 transmits the test image G to the information processing server 20 via the communication unit 11. If "Capture image again" is tapped by the user, the control unit 13 returns to the imaging screen of the imaging unit 16 and captures an image again.

When transmitting the test image G, the control unit 13 transmits the individual identification information IDA together with the test image G to the information processing server 20. The individual identification information IDA is embedded in the two-dimensional code 102. In the control unit 13, the imaging control unit 130 captures the test image G, for example, so as to include the coloring part 103 and the two-dimensional code 102. The control unit 13 transmits the test image G including the coloring part 103 and the two-dimensional code 102 to the information processing server 20, thereby transmitting the individual identification information IDA to the information processing server 20.

When transmitting the test image G, the control unit 13 transmits the individual identification information IDA together with the test image G to the information processing server 20. The individual identification information IDA is embedded in the two-dimensional code 102. In the control unit 13, the imaging control unit 130 captures the test image G, for example, so as to include the coloring part 103 and the two-dimensional code 102. The control unit 13 transmits the test image G including the coloring part 103 and the two-dimensional code 102 to the information processing server 20, thereby transmitting the individual identification information IDA to the information processing server 20.

In addition, the control unit 13 transmits personal identification information IDB to the information processing server 20. The personal identification information IDB is information for uniquely identifying a tested person, for example, information indicating a full name or the like. For example, the control unit 13 displays an input screen for inputting the personal identification information IDB to the telemedicine application and transmits the information input by the user according to guidance on the screen to the information processing server 20 as the personal identification information IDB. The control unit 13 may set, as the personal identification information IDB, information previously registered by the user, for example, when an account is created in the telemedicine application. The control unit 13 transmits the personal identification information IDB together with the test image G including the coloring part 103 and the two-dimensional code 102 to the information processing server 20.

### (Regarding Personal identification information IDB)

The personal identification information IDB may be information that at least can identify a person. The personal identification information IDB may be a code for identifying a person (e.g., an individual number, a license number, or an insurance number). The individual number or the like is stored in a database in a state in which it is linked with a full name, an address, and the like. By using the code such as an individual number linked with a full name, an address, and the like, it is not required of the user to input a specific full name and address to the telemedicine application. Hence, anonymity can be increased so that a person cannot be identified from the appearance. The personal identification information IDB may be downloaded and input from another system.

The personal identification information IDB may include attribute information, location information, and used equipment information. The attribute information is information that indicates an attribute of a tested person, specifically, information required when a diagnosis is performed by a doctor. The attribute information is, for example, information that indicates answers for questions addressed in a medical interview. The attribute information for example, information that indicates, for example, age, sex, occupation, medical histories of the person in question and their family, current health condition, presence or absence of allergies, travel history, and the like. The location information is information that indicates a location at which a test has been performed. The location information may be information input by the user or information automatically acquired by GPS (Global Positioning System) or the like included in the user terminal 10. The location information is used, for example, in a region in which an infection has spread, when statistical information is created which indicates the number of persons who have undergone a test. The used equipment information is information for identifying an identity of the user terminal 10 used by the tested person. The used equipment information is, for example, a serial number, a terminal number, a MAC address, or the like of a smartphone.

### (Regarding information processing server 20)

The information processing server 20 is a computer device of the tested person. The information processing server 20 is implemented by, for example, a cloud device, a server device, a PC, or the like. The information processing server 20 is, for example, a server device that manages a site concerning the telemedicine application.

FIG. 4 is a block diagram illustrating a configuration example of the information processing server 20 according to the embodiment. The information processing server 20 includes, for example, a communication unit 21, a storage unit 22, and a control unit 23. The communication unit 21 communicates with the user terminal 10 and the institution server 30.

The storage unit 22 is configured by a storage medium such as an HDD, a flash memory, an EEPROM, a RAM, and a ROM or a combination thereof. The storage unit 22 stores a program for performing various processes of the information processing server 20 and temporary data used when the various processes are performed. The storage unit 22 stores individual information 220, tested person information 221, test result information 222, and determination criterion information 223.

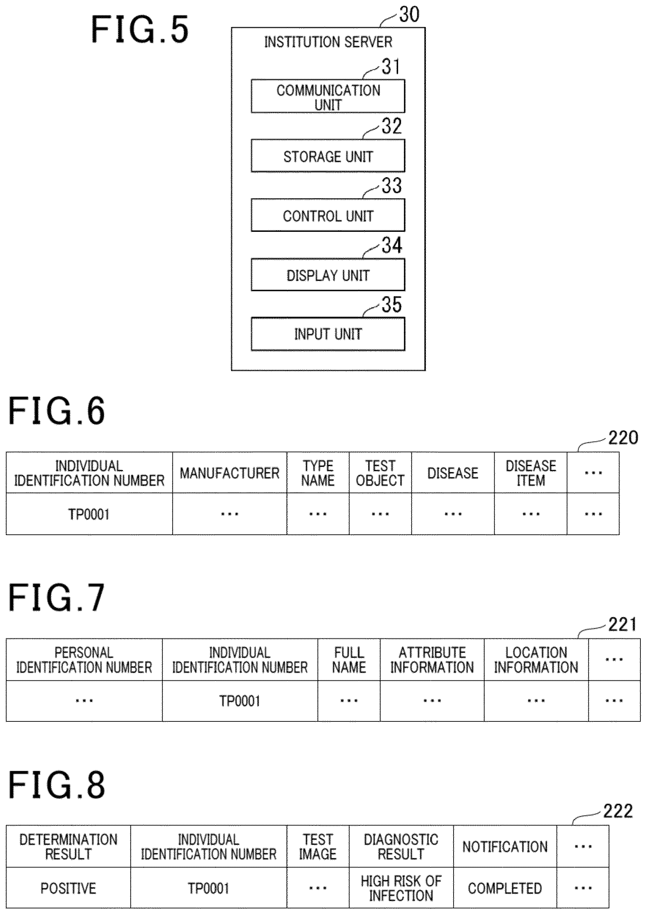

FIG. 6 is a diagram illustrating an example of the individual information 220 according to the embodiment. The individual information 220 is information that includes at least the individual identification information IDA and is information that is embedded in the two-dimensional code 102. The individual information 220 includes, for example, an individual identification number, a manufacturer, a type name, a test object, diseases, and disease items. The individual identification number is identification information such as a manufacturing number uniquely specifying the test strip 100. The manufacturer is information that indicates a manufacturer that produced the test strip 100. The type name is information that indicates a type name of the test strip 100.

The test object is a substance to be tested for by the test strip 100. The diseases are information that indicates diseases that can be indicated by the presence of the test object of the test strip 100. The disease items are information on the subdivided diseases.

FIG. 7 is a diagram illustrating an example of the tested person information 221 according to the embodiment. The tested person information 221 is information that includes at least the personal identification information IDB of the tested person and information that has been provided from the user terminal 10. The tested person information 221 includes, for example, a personal identification number, an individual identification number, a full name, attribute information, and location information. The personal identification number is identification information such as an individual number for uniquely identifying a person. The individual identification number is an individual identification number of the individual information 220. The full name is a full name of the tested person specified by the personal identification number. The attribute information is information that indicates an attribute of the tested person, specifically, information required when a diagnosis is performed by a doctor. The location information is information indicating a location at which the tested person is tested.

FIG. 8 is a diagram illustrating an example of the test result information 222 according to the embodiment. The test result information 222 is information that indicates a determination result based on the test image G. The determination result is provided by analyzing the test image G using a determination unit 232 described later and is a result of a quantitative determination of whether the test strip 100 indicates that the biological sample includes the test object.

The storage unit 22 includes items, for example, the determination result, the individual identification number, the test image G, the diagnostic result, and a notification. The determination result is information indicating a determination result based on the test image G, for example, positive or negative, presence or absence of a suspicion of a disease, and the like. The individual identification number is an individual identification number of the individual information 220. The test image G is information indicating the test image G provided from the user terminal 10. The diagnostic result is information that indicates a result of a diagnosis performed by a doctor based on the determination result and the test image G and is, for example, information indicating "possibility of infection is high" or the like.

The notification is information that indicates whether the determination result and the diagnostic result are provided to the user terminal 10.

The determination criterion information 223 is information that indicates a color serving as a determination criterion (e.g., a pixel value) when the determination is made based on the test image G. The determination criterion information 223 is created, for example, for each type of the test strips 100.

Returning to the description of FIG. 4, the control unit 23 is implemented by causing a CPU that the information processing server 20 includes as hardware to execute the program. The control unit 23 performs overall control of the information processing server 20. The control unit 23 includes, for example, an acquisition unit 230, a correction unit 231, a determination unit 232, a storage control unit 233, and a device control unit 234.

The acquisition unit 230 acquires the test image and the personal identification information IDB provided from the user terminal 10 via the communication unit 21. The acquisition unit 230 outputs the test image to the correction unit 231.

The acquisition unit 230 outputs the test image and the personal identification information IDB to the storage control unit 233.

The correction unit 231 corrects the test image. For example, the correction unit 231 corrects the color of the test image. In this case, for example, the test strip 100 is provided with a color correction indicator (e.g., a patch for color correction, a color code, or the like), and an image is captured so that the color correction indicator is included in the test image G. The correction unit 231 creates a color conversion table by which the pixel value of the captured color correction indicator of the test image G becomes a pixel value of a color concerning a predetermined color correction indicator. The correction unit 231 converts the pixel value of the test image G using the created color conversion table to correct the color of the test image.

The color correction indicator is not limited to being directly provided on the test strip 100. When a test image is acquired by the imaging unit, a color correction indicator whose image is included in the same test image may be used. For example, when an image of the test strip is captured, paper on which the color correction indicator is printed may be prepared, and an image of the color correction indicator on the paper may be also simultaneously captured, to perform color correction.

In addition, as the color code used as the color correction indicator, for example, cyan, magenta, yellow, black, blue, green, red, gray scale, or the like is used. Not only a single color but also a combination of a plurality of color codes may be used. Furthermore, a color gradient may be used and compared with light and shade levels of the control line 1030 and the test line 1031 to determine the density of the control line 1030 and the test line 1031.

The correction unit 231 may correct an angle of the test image. In this case, for example, the test strip 100 is provided with an angle correction indicator. The angle correction indicator is a line parallel to a reference line (e.g., the control line 1030 or the test line 1031) provided to the test strip 100 or a line perpendicular to the reference line. Then, an image is captured so that the angle correction indicator is included in the test image G. The correction unit 231 changes the angle of the test image so that the line of the angle correction indicator whose image is included in the test image G is along the vertical direction or the horizontal direction of the image to correct the angle of the test image. The correction unit 231 outputs the corrected test image G to the determination unit 232.

The angle correction indicator is not limited to be directly provided to the test strip 100. When a test image is acquired by the imaging unit, the color correction indicator whose image is included in the same test image may be used. For example, when an image of the test strip is captured, paper on which the color correction indicator is printed may be prepared, and an image of the color correction indicator on the paper may be also simultaneously captured, to perform color correction.

In addition, as a component to be prepared in addition to the test strip 100, other than paper, a masking jig (a jig for covering or holding the test strip 100 to mask an area other than the display area whose image is required to be captured) or a holding jig may be used.

In the above description, a case is exemplified in which the correction unit 231 performs correction using the color correction indicator or the angle correction indicator. However, the correction unit 231 is not limited to the case in which correction is performed using the color correction indicator or the angle correction indicator. The correction unit 231 may use, instead of the color correction indicator and the angle correction indicator, the two-dimensional code 102 to perform correction. Specifically, the correction unit 231 may use a color of the two-dimensional code 102 to correct the color of the test image G. In addition, the correction unit 231 corrects the angle of the test image G depending on an angle between the vertical direction or the horizontal direction of the test image G and the vertical direction or the horizontal direction of the two-dimensional code 102 whose image is included in the test image G.

The determination unit 232 analyzes the (corrected) test image G to determine whether the test object is present. For example, if the pixel value of the pixel of the coloring part 103 indicates a color close to the color depending on the determination criterion information 223, the determination unit 232 determines that the test object is present. In contrast, if the pixel value of the pixel of the coloring part 103 indicates a color different from the color depending on the determination criterion information 223, the determination unit 232 determines that the test object is not present. Hence, presence or absence of the test object can be quantitatively determined compared with a case in which the tested person makes self-judgement. The determination unit 232 outputs the determination result to the storage control unit 233.

When the quantitative determination is made, the color correction indicator may be utilized. Furthermore, when a test image is acquired by the imaging unit, the color correction indicator whose image is included in the same test image may be used. For example, when an image of the test strip is captured, paper on which the color correction indicator is printed may be prepared, and an image of the color correction indicator on the paper may be also simultaneously captured, to calculate a quantitative value utilizing the color correction indicator.

The storage control unit 233 controls information to be stored in the storage unit 22. The storage control unit 233 extracts an area of the two-dimensional code 102 from the test image acquired from the acquisition unit 230. The storage control unit 233 analyzes an image of the extracted area to acquire information embedded in the two-dimensional code 102. The storage control unit 233 causes the storage unit 22 to store the acquire information as the individual information 220.

The storage control unit 233 generates information in which the personal identification information IDB acquired from the acquisition unit 230 is associated with the individual identification information IDA and causes the storage unit 22 to store the generated information as the tested person information 221.

In the present embodiment, in the tested person information 221, the personal identification information IDB is stored in a state of not being associated with the test image G but being associated with the individual identification information IDA. The individual identification information IDA is information unrelated to the tested person and is, for example, a character string indicating a manufacturing number or the like. That is, in the present embodiment, the personal identification information IDB is not associated with the test image G but is associated with a character string or the like unrelated to the tested person. Hence, a person and the test image G cannot be easily associated with each other, whereby anonymity can be increased.

The storage control unit 233 causes an item of the test image G of the test result information 222 to store the test image. The storage control unit 233 causes an item of the determination result of the test result information 222 to store the determination result acquired from the determination unit 232.

In addition, when the information processing server 20 and the institution server 30 have communicated with each other, and some sort of response to the determination result has been acquired, the storage control unit 233 associates the response result with the determination result and causes the test result information 222 to store them.

For example, when a diagnosis is performed by a doctor based on the determination result in response to a request by the tested person, the storage control unit 233 causes an item of the diagnostic result of the test result information 222 to store a diagnostic result provided from the institution server 30 of the medical institution. A method of undergoing a diagnosis performed by a doctor via the institution server 30 of the medical institution will be described later.

In addition, when the determination result and the diagnostic result are provided to the user terminal 10, the storage control unit 233 causes an item of the provision of the test result information 222 to store an indication that the provision has been made.

In the present embodiment, as in the tested person information 221, in the test result information 222, the determination result is stored in a state of not being associated with the personal identification information IDB but being associated with the individual identification information IDA. Hence, the person and the determination result cannot be easily associated with each other, whereby anonymity can be increased.

The device control unit 234 performs overall control of the information processing server 20. For example, the device control unit 234 outputs the test image G and the like received by the communication unit 21 to the acquisition unit 230. When transmitting the test image G to the institution server 30, the device control unit 234 refers to the storage unit 22 and extracts the test result information 222 of the tested person. The device control unit 234 acquires the test image G and the like from the extracted test result information 222 and transmits the acquired test image G and the like to the institution server 30.

In this case, the device control unit 234 associates the test image G with the individual identification information IDA and transmits them to the institution server 30. That is, when transmitting the test image G and the like, the device control unit 234 does not transmit the personal information of the tested person to the institution server 30. Hence, the test image G is not easily associated with the personal information.

If a response to the determination result is provided from the institution server 30, the device control unit 234 outputs the response to the storage control unit 233 to store the response in a state of being associated with the determination result.

For example, a case will be considered in which a diagnosis by a doctor is performed based on the test image G and the determination result as a result of a request by the tested person. The device control unit 234 acquires the test image G and the determination result from the test result information 222. The device control unit 234 acquires the individual identification information IDA from the individual information 220. The device control unit 234 transmits the test image G and the determination result together with individual identification information IDA to the institution server 30 of the medical institution. The device control unit 234 may provide the diagnostic result provided from the institution server 30 to the user terminal 10. After providing the diagnostic result, the device control unit 234 may cause the storage control unit 233 to store an indication that the diagnostic result has been provided.

### (Regarding institution server 30)

The institution server 30 is a computer device of various institutions such as medical institutions and administrative institutions. The institution server 30 includes server devices of general institutions such as a PC of an event promoter installed in an event site. The institution server 30 is operated by medical personnel, that is, doctors and the like, persons in charge in an administrative institution, personnel managing an event, and the like. The institution server 30 is implemented by, for example, a cloud device, a server device, a PC, or the like. Hereinafter, a case will be exemplified in which the institution server 30 is a server device of a medical institution managed by a hospital or the like that performs diagnoses with telemedicine.

FIG. 5 is a block diagram illustrating a configuration example of the institution server 30 according to the embodiment. The institution server 30 includes, for example, a communication unit 31, a storage unit 32, a control unit 33, a display unit 34, and an input unit 35. The communication unit 31 communicates with the information processing server 20. The storage unit 32 is configured by a storage medium such as an HDD, a flash memory, an EEPROM, a RAM, and a ROM or a combination thereof. The storage unit 32 stores a program for performing various processes of the institution server 30 and temporary data used when the various processes are performed.

The display unit 34 includes, for example, a display device such as a liquid crystal display and displays images according to control performed by the control unit 33. The input unit 35 includes, for example, an input device such as a mouse and a keyboard and outputs information input by operation by the user such as the tested person to the control unit 33.

The control unit 33 is implemented by causing a CPU that the institution server 30 includes as hardware to execute the program. The control unit 33 performs overall control of the institution server 30. The control unit 33 causes the display unit 14 to display the test image G and the determination result received by the communication unit 31 from the information processing server 20. A doctor observes the displayed test image G and considers the determination result to perform a diagnosis, and inputs the diagnostic result to the institution server 30. The control unit 33 acquires the diagnostic result input by the doctor via the input unit 35. The control unit 33 associates the diagnostic result with the individual identification information IDA and transmits them to the information processing server 20.

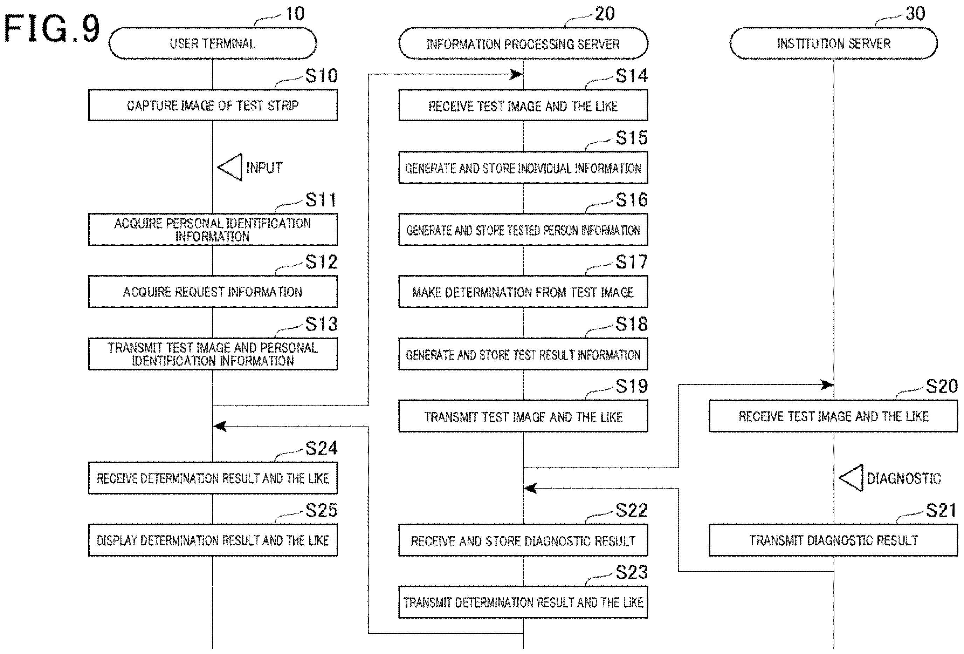

FIG. 9 is a sequence diagram illustrating a flow of processing performed by the information processing system 1 according to the embodiment. The user terminal 10 captures an image of the test strip 100 (step S10). Herein, the test strip 100 is a post-test test strip to which a biological sample of the tested person is supplied. The user terminal 10 acquires the personal identification information IDB (step S11). The user terminal 10 displays a screen to which the personal identification information IDB is input, and a user performs input operation according to the display to acquire the personal identification information IDB.

In addition, the user terminal 10 acquires request information (step S12). The request information is information indicating a request for a diagnosis or the like, by the user, using test image G. For example, the request information is information that indicates whether a diagnosis by a doctor using the test image G or the like is required, the doctor from which a diagnosis is requested, and the like. The user terminal 10 displays, for example, in addition to a message such as "Do you require diagnosis by doctor?", selection buttons such as "Required" and "Not required". If the user operates the selection button "Required" according to the display, in addition to a message such as "Select doctor from which diagnosis is requested", names of hospitals and doctors that can perform a telediagnosis are displayed on a list. The user searches the displayed list by scrolling or the like and performs an operation for selecting a hospital and a doctor from which to request a diagnosis. Hence, the user terminal 10 acquires the request information.

The user terminal 10 transmits the test image G, the personal identification information IDB, and the request information to the information processing server 20 (step S13). The test image G transmitted herein is an image including images of the two-dimensional code 102 and the coloring part 103 captured in step S10.

It is noted that the order of steps S10 to S12 described above is optional. In step S13, before transmitting information to the information processing server 20, the user terminal 10 is required to acquire the information (the test image G, the personal identification information IDB, and the request information). In the example of this figure, step S11 and step S12 are executed in series, whereby the number of changes of input screens is minimized, which increases efficiency.

The information processing server 20 receives the test image G and the like (step S14). The information processing server 20 generates the individual information 220 and causes the individual information 220 to be stored (step S15). The information processing server 20 reads the information embedded in the two-dimensional code 102 whose image is included in the test image G to acquire the individual identification information IDA. The information processing server 20 generates the tested person information 221 and causes the tested person information 221 to be stored (step S16). The information processing server 20 does not associate the personal identification information IDB provided from the user terminal 10 with the test image G but associates it with the individual identification information IDA to generate the tested person information 221.

The information processing server 20 makes a determination from the test image G (step S17). The information processing server 20 analyzes an image of the coloring part 103 of the test image G to determine whether the test object is present. The information processing server 20 generates the test result information 222 and causes the test result information 222 to be stored (step S18). The information processing server 20 does not associate the determination result and the test image G with the personal information but associates the determination result and the test image G with the individual identification information IDA to generate the test result information 222. If the tested person has requested diagnosis by a doctor, the information processing server 20 specifies a hospital or a doctor from which the diagnosis is requested based on the request information and transmits the test image G and the determination result to the institution server 30 of the medical institution corresponding to the specified hospital or the like (step S19).

The institution server 30 receives the test image G and the determination result from the information processing server 20 (step S20). The institution server 30, for example, displays the test image G and the determination result, whereby diagnosis by a doctor is performed and a diagnostic result is input to the institution server 30. The institution server 30 transmits the diagnostic result to the information processing server 20 (step S21).

The information processing server 20 receives the diagnostic result from the institution server 30 and causes the test result information 222 to store the diagnostic result in (step S22). The information processing server 20 transmits the determination result together with the diagnostic result to the user terminal 10 (step S23).

The user terminal 10 receives the determination result and the diagnostic result from the information processing server 20 (step S24). The user terminal 10 displays the received determination result and diagnostic result (step S25).

It is noted that in the above flow, the case has been exemplified in which the information processing server 20 transmits the test image G and the like to the institution server 30 based on the request information. However, this is not limiting. The information processing server 20 may be configured so as to transmit the test image G and the like to the institution server 30 in response to a request from the institution server 30.

For example, the information processing server 20 can be applied to a case in which while telemedicine is performed in a system different from the information processing system 1, the individual identification information IDA is conveyed from a user to a doctor, and the doctor requests the information processing server 20 to transmit the determination result corresponding to the individual identification information IDA. Alternatively, the information processing server 20 can be applied to a case in which at a later date after the information processing system 1 performs the test, at the time of a face-to-face diagnosis, the individual identification information IDA is supplied from a user to a doctor, the doctor requests the information processing server 20 to transmit the determination result associated with the individual identification information IDA.

### (First modification of embodiment)

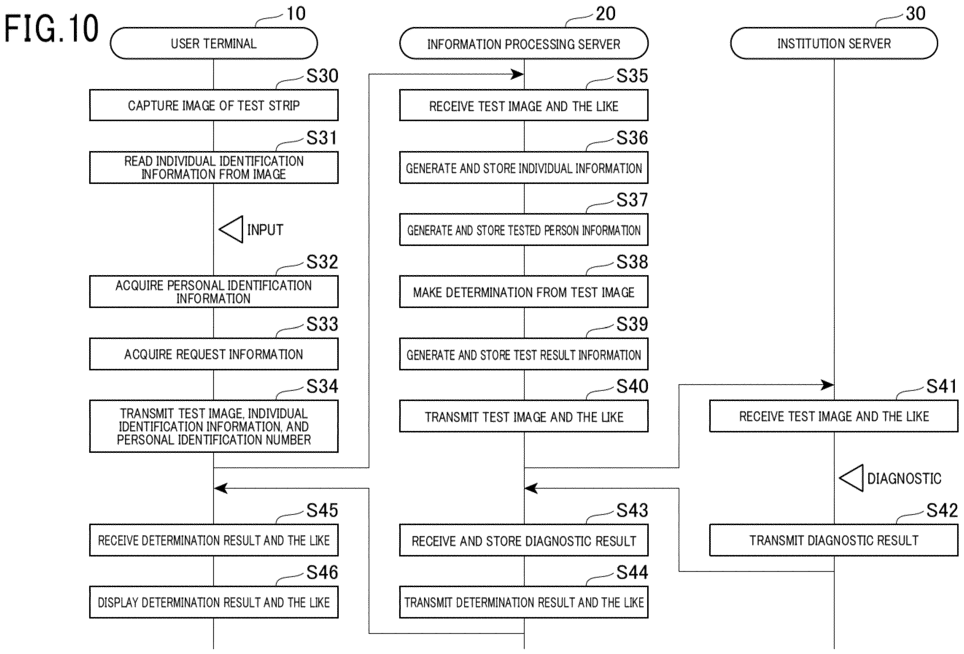

FIG. 10 is a sequence diagram illustrating a flow of processing performed by the information processing system 1 according to a first modification of the embodiment. Since steps S32, S33, S37 to S46 are similar to step S11, S12, S16 to S25 illustrated in FIG. 9, description thereof is omitted.

The user terminal 10 reads the individual identification information IDA from an area of the two-dimensional code 102 in a captured image (step S31). The user terminal 10 reads the two-dimensional code 102 with a code reader to acquire the individual identification information IDA. The code reader is installed in the user terminal 10 as, for example part of a function of the telemedicine application. The user terminal 10 transmits the read individual identification information IDA together with the test image G and the personal identification information IDB to the information processing server 20 (step S34). In this case, the user terminal 10, for example, trims an image captured in step S30 to generate an image including at least the coloring part 103, sets the generated image as the test image G, and transmits the test image G.

The information processing server 20 receives the test image G, the individual identification information IDA, and the personal identification information IDB from the user terminal 10 (step S35). The information processing server 20 generates the individual information 220 and causes the individual information 220 to be stored (step S36). The information processing server 20 causes the individual identification information IDA received from the user terminal 10 to be stored as the individual information 220.

In the first modification of the embodiment, the user terminal 10 reads the individual identification information IDA from the two-dimensional code 102. Then, the user terminal 10 transmits the test image G including only the coloring part 103 and the individual identification information IDA to the information processing server 20. Herein, the individual identification information IDA transmitted to the information processing server 20 is not an image (image data) of the two-dimensional code but character string (character data) information. The test image G transmitted to the information processing server 20 is an image in which only the coloring part 103 has been trimmed, and is information having a data quantity smaller than that of the image including the two-dimensional code 102 and the coloring part 103. Hence, in the present modification, compared with the embodiment, the data quantity transmitted to the information processing server 20 can be reduced.

In addition, the test image G transmitted to the information processing server 20 may be an image in which the coloring part 103 is enlarged. In this case, it can be expected that accuracy in a determination by the information processing server 20 is improved.

It is noted that, instead of reading the individual identification information IDA from an image, the individual identification information IDA may be manually input by the user. In this case, for example, in the test strip 100, the individual identification information IDA is displayed as a character string, symbols, or the like. The user inputs the character string or the symbols indicating the individual identification information IDA displayed on the test strip 100 to the user terminal 10.

### (Second modification of embodiment)

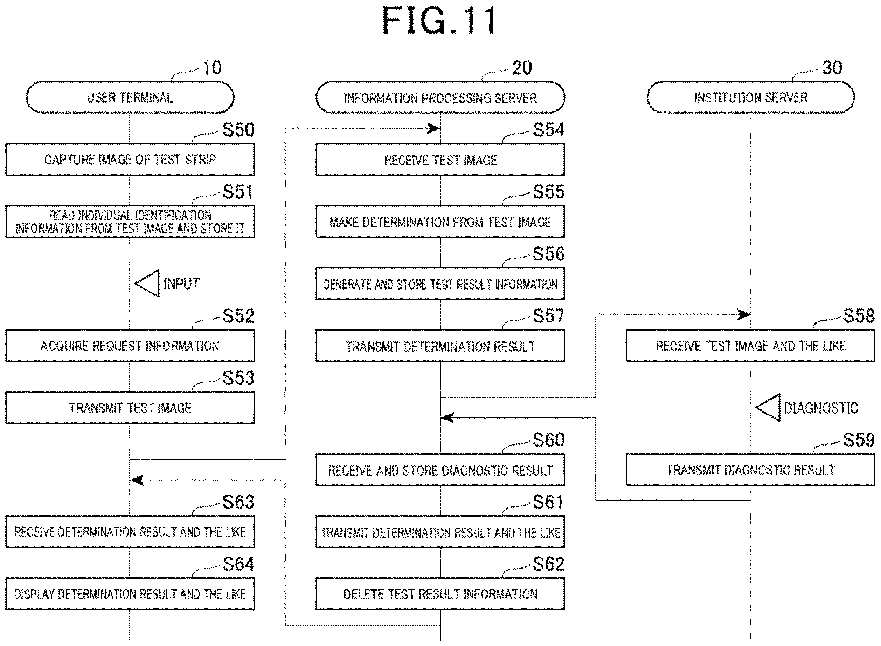

FIG. 11 is a sequence diagram illustrating a flow of processing performed by the information processing system 1 according to a second modification of the embodiment. Since steps S52, S55, S57 to S60, S63 are similar to S12, S17, S19 to S22, S24 illustrated in FIG. 9, description thereof is omitted.

The user terminal 10 captures an image of the test strip 100 so as to include the two-dimensional code 102 and the coloring part 103 (step S50). The user terminal 10 reads the individual identification information IDA from the two-dimensional code 102 of the test image G and causes the storage unit 12 to store the individual identification information IDA (step S51). The user terminal 10 transmits only the test image G and the request information to the information processing server 20 (step S53). Herein, the user terminal 10 does not transmit the read individual identification information IDA to the information processing server 20.

The information processing server 20 receives the test image G from the user terminal 10 (step S54). The information processing server 20 generates the test result information 222 and causes the storage unit 22 to store the test result information 222 (step S56). The information processing server 20 causes the test result information 222 to store the determination result. The information processing server 20 reads the individual identification information IDA from the two-dimensional code 102 of the test image G and causes the test image of the test result information 222 to store the read individual identification information IDA. The information processing server 20 transmits the determination result and the diagnostic result to the user terminal 10 in a state in which the determination result and the diagnostics result are associated with the individual identification information IDA (step S61). The information processing server 20 deletes the test result information 222 (step S62). The user terminal 10 compares the individual identification information IDA associated with the determination result and the diagnostics result received from the information processing server 20 with the individual identification information IDA stored in the storage unit 12, and displays the determination result and the diagnostics result if both the pieces of individual identification information IDA agree with each other (step S64).

In the second modification of the embodiment, the user terminal 10 reads the individual identification information IDA from the two-dimensional code 102 and causes a storage unit (storage unit 12) of the user terminal 10 to store the individual identification information IDA. The user terminal 10 transmits only the test image G to the information processing server 20. The user terminal 10 does not transmit the personal identification information IDB. In addition, after informing the user terminal 10 of the determination result and the like, the information processing server 20 deletes the test result information 222. Hence, information leakage is suppressed, whereby safety in security can be increased.

It is noted that as in the first modification described above, instead of reading the individual identification information IDA from an image, the individual identification information IDA may be manually input by the user also in the second modification.

As described above, the information processing system 1 of the embodiment includes the test strip 100, the user terminal 10, and the information processing server 20. The information processing system 1 is a system managing information on tests performed using the test strip 100. The test strip 100 has the coloring part 103. When a biological sample taken from the tested person is supplied to the test strip 100, the coloring part 103 develops a color depending on whether the biological sample includes the test object. The test strip 100 is provided with the individual identification information IDA. The user terminal 10 has the imaging unit 16 and the communication unit 11. The imaging unit 16 captures the test image G. The test image G is a digital image including at least the coloring part 103 of the post-test test strip 100. The communication unit 11 transmits the test image G and the individual identification information IDA to the information processing server 20. The information processing server 20 includes the determination unit 232 and the storage control unit 233. The determination unit 232 determines whether the coloring part 103 in the test image G indicates the presence of the test object based on the test image G and the determination criterion information 223. The storage control unit 233 causes the test image G to be stored in a state of being associated with the individual identification information IDA.

Hence, the information processing system 1 of the embodiment can transmit the test image G to the information processing server 20. The test image G includes an image of the coloring part 103. Hence, the information processing server 20 can determine whether it is positive based on the coloring part 103 in the test image G. Hence, it can be quantitatively determined whether the post-test test strip 100 indicates a positive result. In addition, since the test image G is transmitted as electronic information, the determination can be made quickly compared with a case in which a specimen or the post-test test strip 100 is mailed. In addition, the test image G is stored in a state of being associated with the individual identification information IDA that is unrelated to the personal identification information IDB. Hence, a person and the test image G cannot be easily associated with each other, whereby anonymity can be increased.

Herein, the test strip 100 is an example of a "test piece". The user terminal 10 is an example of a "first device". The communication unit 11 is an example of a "first communication unit". The information processing server 20 is an example of a "second communication unit".

In addition, in the information processing system 1 of the embodiment, the user terminal 10 includes the input unit 15.

The function of the input unit 15 is an example of an "acquisition step". To the input unit 15, the personal identification information IDB is input. The personal identification information IDB is information concerning the tested person and including at least one of information for uniquely identifying a person, attribute information, and location information.

The communication unit 11 transmits the personal identification information IDB to the information processing server 20. The storage control unit 233 causes the personal identification information IDB to be stored in a state of being associated with the individual identification information IDA. Hence, in the information processing system 1 of the embodiment, the personal identification information IDB can be managed separately from the test image G and the determination result. Hence, a person cannot be easily associated with the test image G and the determination result, whereby anonymity can be increased.

In addition, in the information processing system 1 of the embodiment, the communication unit 21 of the information processing server 20 informs the user terminal 10 of the determination result. The user terminal 10 displays the determination result. Hence, in the information processing system 1 of the embodiment, the determination result can be quickly recognized by the user compared with a case in which the determination result is provided by mail.

In addition, in the information processing system 1 of the embodiment, the communication unit 21 of the information processing server 20 informs the institution server 30 of the determination result. The institution server 30 is an example of a "third device". The institution server 30 transmits the diagnostic result (an example of a verification result) produced by a doctor (an example of a verifying person) to the information processing server 20. Hence, according to the information processing system 1 of the embodiment, not only determination by image analysis but also diagnosis by a doctor or the like can be performed.

In addition, in the information processing system 1 of the embodiment, the storage control unit 233 of the information processing server 20 stores the determination result in a state of not being associated with the tested person information 221 but being associated with the individual information 220. The storage control unit 233 stores the personal identification information IDB in a state of not being associated with the determination result but being associated with the individual information 220. Hence, according to the information processing system 1 of the embodiment, a person cannot be easily associated with the determination result, whereby anonymity can be increased.

In addition, in the information processing system 1 of the embodiment, the personal identification information IDB is embedded in the two-dimensional code 102. The imaging unit 16 captures an image so as to include the two-dimensional code 102 and the coloring part 103. The communication unit 11 transmits the test image G including the two-dimensional code 102 and the coloring part 103 to the information processing server 20. Hence, in the information processing system 1 of the embodiment, the personal identification information IDB can be transmitted by transmitting the test image G.

In addition, in the information processing system 1 of the embodiment, the two-dimensional code 102 is information indicating at least one selected from a group including a type of the test object, a type of the disease, and a manufacturer of the test piece. Hence, according to the information processing system 1 of the embodiment, when a determination is made, the determination can be made based on the individual identification information IDA, considering various pieces of information such as the test object and the disease. Furthermore, the types and the number of the diseases for which a determination is made can be acquired based on the individual identification information IDA, whereby statistical processing can be easily performed.

In addition, in the information processing system 1 of the embodiment, the imaging unit 16 may capture an image so that a plurality of pieces of information different from each other included in the individual identification information IDA are included in one image.

For example, when the name of a manufacturer is displayed on the test strip 100, the imaging unit 16 captures an image of the two-dimensional code 102 and an area on which the name of the manufacturer is displayed. Hence, in the information processing system 1 of the embodiment, even when information indicating the manufacturer is not embedded in the two-dimensional code 102, the information can be acquired from the test image G.

In addition, in the information processing system 1 of the embodiment, the test strip 100 is a lateral flow test strip. The test strip used for immunochromatography illustrated in FIG. 2 is an example of the "lateral flow test strip". Since the lateral flow assay can easily and quickly detect a target substance (tested substance) in a specimen liquid, the lateral flow assay is widely used in the clinical field, food field, environmental inspection field, and the like. Hence, according to the information processing system 1 of the embodiment, information concerning the test using the test strip 100 to which a method having an actual achievement and reliability is applied can be used.

In addition, in the information processing system 1 of the embodiment, the communication unit 11 associates the information on the manufacturer of the test strip 100 with the test image G and transmits them to the information processing server 20. Hence, according to the information processing system 1 of the embodiment, a determination can be made based on the information on the manufacturer of the test strip 100, considering test accuracy and the like of the test strip 100 as a product.

In addition, in the information processing system 1 of the embodiment, when capturing an image of the test strip 100, the imaging unit 16 causes the display unit 14 to display a guide representing an outline of the test strip 100. Hence, according to the information processing system 1 of the embodiment, since orientations and sizes of the test strips 100 included in the test images G can be made to coincide with each other, the information processing server 20 can analyze images with high accuracy.

In addition, in the information processing system 1 of the embodiment, the test strip 100 is provided with the color correction indicator. The imaging unit 16 captures the test image G including the color correction indicator. The information processing server 20 further includes the correction unit 231. The correction unit 231 uses the color correction indicator to correct the color of the test image G. Hence, according to the information processing system 1 of the embodiment, since the color of the test image G can be corrected, the information processing server 20 can analyze images with high accuracy.

In addition, in the information processing system 1 of the embodiment, the test strip 100 is provided with the angle correction indicator. The imaging unit 16 captures the test image G including the angle correction indicator. The correction unit 231 uses the angle correction indicator to correct the angle of the test image G. Hence, according to the information processing system 1 of the embodiment, since the angle of the test image G can be corrected, the information processing server 20 can analyze images with high accuracy.

In addition, in the information processing system 1 of the embodiment, the correction unit 231 uses the two-dimensional code 102 to correct at least one of the color and the angle of the test image G. Hence, according to the information processing system 1 of the embodiment, even if the test strip 100 is not provided with a specific indicator, at least one of the color and the angle of the test image G can be corrected.

In addition, in the information processing system 1 of the embodiment, if an obscuring object is present when capturing an image of the test strip 100, the imaging unit 16 causes the display unit 14 to display it. Hence, according to the information processing system 1 of the embodiment, all or part of the test strip 100 can be suppressed from being obscured in the test image G, whereby accuracy in image analysis by the information processing server 20 can be prevented from lowering.

### (First variation of institution server 30)

In the embodiment described above, a case has been exemplified in which a doctor performs a diagnosis via the institution server 30. However, this is not limiting. In the information processing system 1, instead of the institution server 30, or together with the institution server 30, a server device of a manufacturer (hereinafter, referred to as a manufacturer server) may be applied. Herein, the manufacturer is the manufacturer of the test strip 100. For example, the information processing server 20 sends a list of the individual identification information items IDA to be provided to the test strip 100 to the manufacturer. The manufacturer produces the test strip 100 to which the individual identification information IDA received from the information processing server 20 is provided. The information processing server 20 compares the individual identification information IDA provided from the user terminal 10 with the individual identification information IDA provided to the manufacturer server. If the individual identification information IDA provided from the user terminal 10 is included in the list provided to the manufacturer server, the information processing server 20 makes a determination for the test image G. In contrast, if the individual identification information IDA provided from the user terminal 10 is not included in the list provided to the manufacturer server, since the test strip 100 in the test image G is highly likely not to be a product of the manufacturer, the information processing server 20 does not make the determination. Hence, determination considering whether the test strip 100 is a product of the manufacturer can be made.

### (Second variation of institution server 30)

The institution server 30 may issue a prescription. For example, the institution server 30 acquires the issued prescription together with the diagnostic result obtained from a doctor and provides them to the information processing server 20. In the future, due to an amendment of the law or the like, if prescriptions are allowed to be issued based on a test result obtained by using a reliable test strip 100 of a specific manufacturer, the institution server 30 or the information processing server 20 may issue a prescription based on the determination result.

### (Third variation of institution server 30)

In the information processing system 1, instead of the institution server 30, or together with the institution server 30, a server device of a company (hereinafter, referred to as a company server) may be applied. A case can be assumed in which a company obliges employees of the company to be tested. The company server acquires the determination result associated with the individual identification information IDA from the information processing server 20. The company server can manage the test result in a highly anonymous manner.

### (Fourth variation of institution server 30)

In the information processing system 1, instead of the institution server 30, or together with the institution server 30, a server device of the government or a municipality (hereinafter, referred to as a municipality server) may be applied. A case can be assumed in which the government or the municipality is responsible for monitoring an infection status. The municipality server acquires the determination result associated with the individual identification information IDA from the information processing server 20. The municipality server can manage the test result in a highly anonymous manner.

All or part of the information processing system 1 and the information processing server 20 in the embodiment described above may be implemented by a computer. This case may be implemented by storing a program for implementing the functionality in a computer readable storage medium and causing a computer system to read the program stored in the storage medium to execute the program. The "computer system" herein includes an OS and hardware such as peripheral devices. In addition, the "computer readable storage medium" refers to a storage medium such as a portable medium, such as a flexible disk, a magneto-optical disk, a ROM, and a CD-ROM, and a hard disk and the like installed in the computer system. Furthermore, the "computer readable storage medium" may include a medium that dynamically holds a program for a short time, such as a communication line that transmits a program via a network such as the Internet or a communication line such as a telephone line, and a medium that holds a program for a certain time, such as a volatile memory inside a computer system serving as a server or a client in that case. In addition, the program may be a program for implementing part of the function described above, furthermore, a program that can implement the function described above by the combination with the program previously stored in the computer system, or a program that is implemented by using a programmable logic device such as an FPGA.

### (Second embodiment)

Hereinafter, an embodiment of the present invention will be described with reference to the drawings. In the following embodiment, a different reference sign may be appended to even a similar component to that of the first embodiment.

### <Regarding information processing system 1>

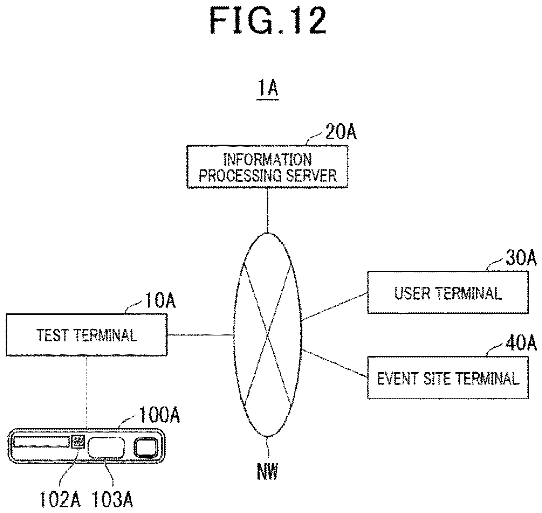

FIG. 12 is a block diagram illustrating a configuration example of the information processing system 1 according to the embodiment. An information processing system 1A includes, for example, a test strip 100A, a test terminal 10A, an information processing server 20A, a user terminal 30A, and an event site terminal 40A. The information processing system 1A is a system that presents a test result at an event site or the like.

The event site of the present embodiment is a site at which presenting a test result is a condition established for entering the site. The event site is, for example, a site at which various events such as a concert and an exhibition are held. If presenting a test result is a condition at a facility such as a school, a medical institution, a welfare facility, a public facility, a public transportation facility, a theme park, a play facility, a company, a shop, a retail store, and a restaurant, these facilities can be included as event sites of the present embodiment.

The test in the present embodiment is a test performed using the test strip 100A. The test is performed, for example, at a medical institution or the like, and supplies a biological sample taken from the tested person to the test strip 100A to determine whether the biological sample includes the test object.

The test terminal 10A is a computer, for example, a smartphone, a cellular phone, a tablet terminal, a PC (Personal Computer), a reader of the test strip, or the like. The test terminal 10A is operated by a person in charge of the test or the like in a test institution such as a medical institution and a health center. To the test terminal 10A, information on the test (test information) is input by the person in charge of testing or the like. The test information is, for example, information indicating a test result, a location at which the test is performed, the date and time when the test has been performed, and the like.

The user terminal 30A is a computer, for example, a smartphone, a cellular phone, a tablet terminal, a PC, or the like. The user terminal 30A is operated by a tested person. To the user terminal 30A, information on the tested person (user information) is input by the tested person. The user information is, for example, information indicating a unique identifier assigned to the tested person, the full name, the address, or the telephone number serving as contact information of the tested person, a photograph of the tested person's facial image, and the like.

The information processing server 20A is a computer, for example, a PC, a server device, a cloud server, or the like. The information processing server 20A communicates with the test terminal 10A and the information processing server 20A via a communication network NW. The communication network NW transmits and receives information. As the communication network NW, for example, a LAN (Local Area Network), a WAN (Wide Area Network), a telephone network (mobile telephone network, fixed-line telephone network), a regional IP (Internet Protocol), the Internet, or the like is used.

The event site terminal 40A is a computer, for example, a smartphone, a cellular phone, a tablet terminal, a PC, or the like. The event site terminal 40A, for example, captures an image of a test result displayed on the user terminal 30A to acquire the test result. The event site terminal 40A determines whether to grant entrance based on the acquired test result.

The test strip 100A is a test piece used for a test. The test strip 100A includes a coloring part 103A. The coloring part 103A shows a color depending on whether the biological sample includes the test object. Depending on the color indicated on the coloring part 103A of the post-test test strip 100A, it can be determined whether the biological sample includes the test object.

In addition, the test strip 100A is provided with the two-dimensional code 102A. In the two-dimensional code 102A, the individual identification information is embedded. The individual identification information is information for uniquely identifying the test strip 100A.

In the present embodiment, the individual identification information of the test strip 100A used for a test is utilized as a test ID. The test ID is information for uniquely identifying the test.

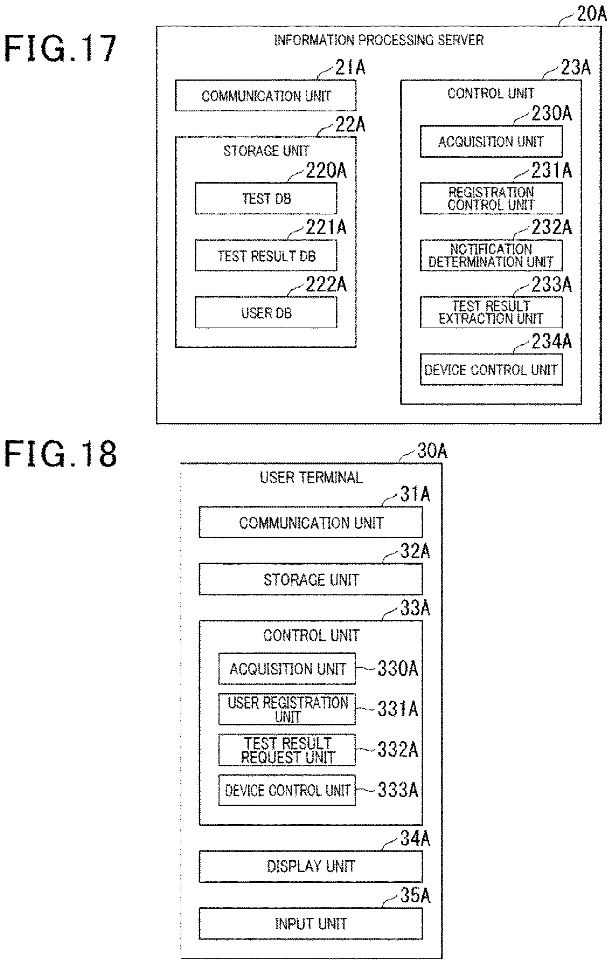

Specifically, in the present embodiment, test information is stored in a test result DB 221A (refer to FIG. 17) in a state in which the test information is associated with the test ID. In addition, user information is stored in a user DB 222A (refer to FIG. 17) in a state in which the user information is associated with the test ID. The test result DB 221A and the user DB 222A are databases different from each other. That is, in the present embodiment, the test information and the user information are stored in separate databases. Hence, the test result and a person are not easily associated with each other. Even if information has leaked from the test result DB 221A, it cannot be known whose test result has leaked. Even if information has leaked from the user DB 222A, it is not possible to determine the test result for the tested person whose information has leaked. As described above, causing the data, by which the test result concerning the tested person can be easily known, not to be stored can increase safety of data.

### (Regarding flow of processing performed by information processing system 1A)

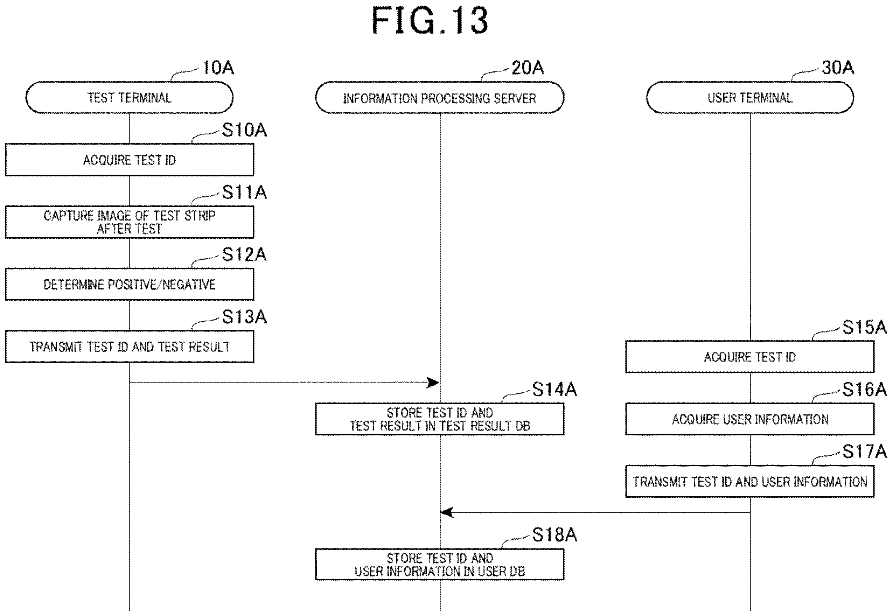

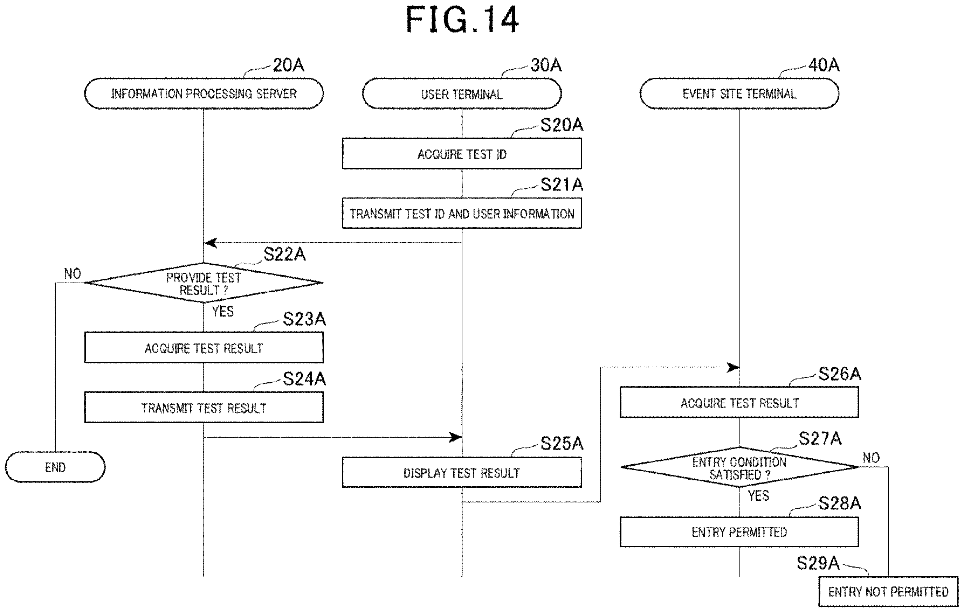

Herein, a flow of processing performed by the information processing system 1A according to the embodiment will be described with reference to FIG. 13 and FIG. 14. FIG. 13 illustrates a flow of processing performed when the test is performed. FIG. 14 illustrates a flow of processing performed in a case of entering an event site.

In the following description, a case will be exemplified in which the information processing server 20A provides a service through an application program (hereinafter, referred to as a test management application) to the test terminal 10A, the user terminal 30A, and the event site terminal 40A, whereby the processing performed by the information processing system 1A is implemented.

The test management application is an application program for implementing a system that holds a test result obtained when the test is performed and that presents the test result at an event site or the like. In the test management application, for example, test information on the test performed at a medical institution or the like can be registered. In the test management application, user information on the tested person can be registered.

In the test management application, a test result of a test that a tested person has undergone can be presented in response to a request from the tested person.

Specifically, by installing the test management application, the test terminal 10A can register the test information on the test performed at the medical institution or the like by utilizing the test management application. In addition, by installing the test management application, the user terminal 30A registers the user information on the tested person by utilizing the application, whereby the user terminal 30A can present the test result of the test that the tested person has undergone at the event site or the like.

It is noted that, as a matter of course, the function corresponding to the test management application may be implemented not by an application but by a Web browser. In this case, the test terminal 10A accesses a site specified by a predetermined URL (Uniform Resource Locator) via the Web browser. At the site, for example, images related to a negative certification are displayed for a test institution, and a person in charge of the test or the like performs input operations according to guidance in the image, whereby the test result is registered.

As illustrated in FIG. 13, when a test is performed, the test terminal 10A acquires the test ID (step S10A). The test ID is identification information for uniquely identifying a test and is individual identification information of the test strip 100A used for the test. The test terminal 10A acquires, for example, an image of the two-dimensional code 102A captured by a person in charge of the test or the like. The test terminal 10A, for example, analyses the acquired image to acquire the individual identification information embodied in the two-dimensional code 102A. Alternatively, the test terminal 10A may acquire the individual identification number directly input by the person in charge of testing. The test terminal 10A sets the acquired individual identification information as the test ID.

The test strip 10A acquires, as test information, an image of the post-test test strip 100A (step S11A). The test terminal 10A acquires an image including an image of the coloring part 103A of the test strip 100A. The coloring part 103A indicates a color depending on whether the biological sample includes the test object. For example, if the biological sample includes the test object, part of an area of the coloring part 103A is colored with a color different from that of the background and develops that color. In contrast, if the biological sample does not include the test object, that part of the area of the coloring part 103A is not colored, and the area of the coloring part 103A develops the color of the background of the coloring part 103A. One type or a plurality of types of colors may be developed on the coloring part 103A. The test terminal 10A acquires, for example, an image of the coloring part 103A captured by the person in charge of testing or the like.

It is noted that step S10A and step S11A may be performed based on the same image. In this case, an overall image of the test strip 100A is captured only once, and step S10A and step S11A are performed by subj ecting the overall image to image processing. The overall image herein is an image including an image of an area including the two-dimensional code 102A and the coloring part 103A of the test strip 100A. Specifically, in step S10A, the test strip 100A acquires the overall image of the post-test test strip 100A and acquires the test ID based on the acquired overall image. In step S11A, the test terminal 10A acquires an image of an area in which an image of the coloring part 103A is included from the overall image acquired in step S10A.

The test terminal 10A determines whether it is positive or negative based on the image acquired in step S11A (step S12A). Positive indicates, for example, that the biological sample includes the test object. In this case, negative indicates that the biological sample does not include the test object. The test terminal 10A, for example, compares information indicating a color represented in the coloring part 103A (e.g., a pixel value) with information indicating a color serving as a determination criterion (e.g., a pixel value) to determine whether the color represented in the coloring part 103A indicates that the biological sample includes the test object. If the color represented in the coloring part 103A indicates that the biological sample includes the test object, the test terminal 10A determines that it is positive. In contrast, if the color represented in the coloring part 103A indicates that the biological sample does not include the test object, the test terminal 10A determines that it is negative.

The test terminal 10A transmits the test ID and the test result to the information processing server 20A (step S13A). The test ID herein is the test ID acquired in step S10A. The test result is the test result acquired in step S12A.

The information processing server 20A stores the test ID and the test result received from the test terminal 10A in the test result DB 221 A (step S14A). The test result DB 221 A is information in which the test ID and the test result are associated with each other.

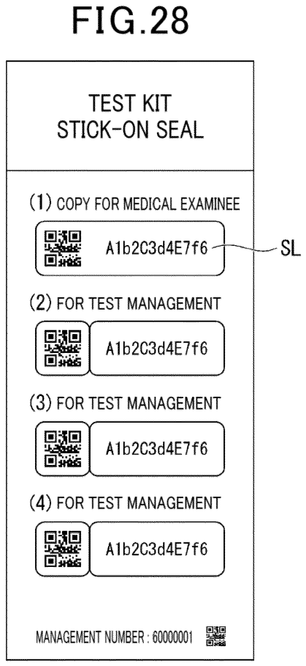

The user terminal 30A acquires the test ID (step S15A). For example, when a test is performed, the tested person obtains printed matter SL (refer to FIG. 28) such as a sticker, on which the individual identification information of the test strip 100A used for their own test is printed, as a copy for the medical examinee from the person in charge of testing. When the individual identification information indicated in the printed matter is input to the user terminal 30A by input operation by the tested person, the user terminal 30A acquires the individual identification information. Alternatively, when a test is performed, the user terminal 30A may read a bar code or the like in which the individual identification information is embedded to acquire the individual identification information. The user terminal 30A sets the acquired individual identification information as the test ID.

Alternatively, the user terminal 30A may receive the individual identification information transmitted from the test terminal 10A to acquire the individual identification information. In this case, for example, the tested person designates an email address or the like when the test is performed, and requests the test terminal 10A to provide the individual identification information of the test strip 100A used for the test to the designated email address. In response to the request, for example, the test terminal 10A transmits the individual identification information to the designated email address. The tested person acquires the individual identification information transmitted to the email address by using the user terminal 30A. Hence, the user terminal 30A acquires the individual identification information.

Alternatively, for example, when the test is performed, the tested person may cause the test terminal 10A and the user terminal 30A to perform short-range communication via Bluetooth (registered trademark) or the like to acquire the individual identification information of the test strip 100A used for the test.

The user terminal 30A acquires user information (step S16A). For example, the user information is input to the user terminal 30A by input operation by the tested person. Hence, the user terminal 30A acquires the user information.

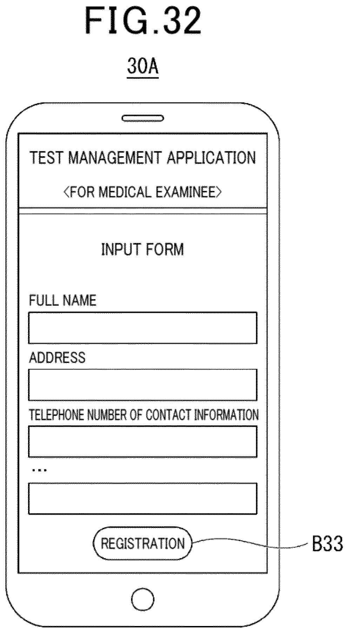

It is noted that before step S16A, the user terminal 30A may log in to the test management application. If logging in to the test management application succeeds, for example, an input form for inputting user information (refer to FIG. 32) is displayed on the user terminal 30A. The tested person inputs user information according to the input form. Hence, the user terminal 30A acquires the user information.

The user terminal 30A transmits the test ID and the user information to the information processing server 20A (step S17A). The test ID herein is the test ID acquired in step S15A. The user information is the user information acquired in step S16A. Herein, the user terminal 30A may transmit all or only part of the acquired user information to the information processing server 20A. For example, when information indicating each of the full name of the tested person and a tested person's facial image is acquired as the user information, the user terminal 30A transmits the full name of the tested person to the information processing server 20A and does not transmit the tested person's facial image to the information processing server 20A. In this case, for example, the user terminal 30A stores information on the tested person's facial image, which has not been transmitted to the information processing server 20A, in a storage unit 32A.

The information processing server 20A causes the user DB 222A to store the test ID and the user information received from the user terminal 30A (step S18A). Hence, the test management application can refer to the user information. The user DB 222A is information in which the test ID and the user information are associated with each other.

As illustrated in FIG. 14, in a case of entering an event site, the user terminal 30A acquires the test ID (step S20A). The test ID is the test ID of the test that is selected, from among the tests that the tested person has undergone, for a test result presented for entering the event site. For example, when the test is selected by the tested person, and the selected test ID is input, the user terminal 30A acquires the test ID.

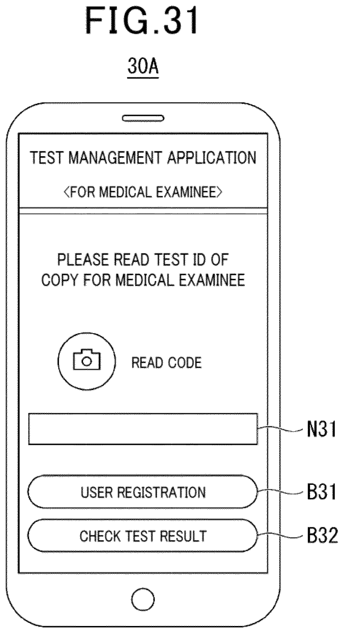

It is noted that before step S20A, the user terminal 30A may log in to the test management application. If logging in to the test management application succeeds, for example, an image for reading the test ID (refer to FIG. 31) is displayed on the user terminal 30A. The tested person inputs the test ID according to the displayed image. Hence, the user terminal 30A acquires the test ID.

The user terminal 30A transmits the test ID, which is acquired in step S20A, together with the user information to the information processing server 20A. For example, the user terminal 30A transmits the user information, which is input by the tested person, together with the test ID, which is acquired in step S20A, to the information processing server 20A.
Alternatively, the user terminal 30A may previously cause the user information acquired in step S16A to be stored. In this case, the user terminal 30A may transmit the stored user information together with the test ID, which is acquired in step S20A, to the information processing server 20A.

The information processing server 20A determines whether to provide the test result to the user terminal 30A based on the information received from the user terminal 30A (step S22A). The information processing server 20A refers to the user DB 222A based on the test ID to determine whether to provide the test result. For example, if the combination of the test ID and the user information received from the user terminal 30A was previously stored in the user DB 222A, the information processing server 20A determines to provide the test result. Alternatively, if the combination of the test ID and the user information received from the user terminal 30A is not stored in the user DB 222A, the information processing server 20A determines not to provide the test result. If determining not to provide the test result, the information processing server 20A transmits a message to that effect to the user terminal 30A, and ends the process.

If determined to provide the test result in step S22A, the information processing server 20A acquires the test result (step S23A). The information processing server 20A refers to the test result DB 221 A based on the test ID to acquire the test result. The information processing server 20A acquires, as the test result, the test information associated with the test ID in the test result DB 221A, which is received from the user terminal 30A. The information processing server 20A transmits the acquired test result to the user terminal 30A (step S24A).

It is noted that in step S21A, the user terminal 30A may provide only the test ID to the information processing server 20A. In this case, the user terminal 30A proceeds to step S23A without performing step S22A and acquires the test result associated with the test ID to transmit the acquired test result to the user terminal 30A. Even in such a case, since only the test result is provided to the user terminal 30A in step S24A, even if a third party who is not the tested person transmits the test ID of the test performed by the tested person to the information processing server 20A, and the test result is provided to the third party, the third party cannot specify the person who has undergone the test corresponding to the provided test result. Hence, privacy of the tested person can be protected. The test result and a person cannot be easily associated with each other.

The user terminal 30A displays the test result received from the information processing server 20A on a screen (step S25A). The tested person, for example, presents the test result displayed on the screen of the user terminal 30A to a verifying person who is verifying entrance at the event site. The verifying person, for example, captures an image of the test result displayed on the screen of the user terminal 30A by using a camera of the event site terminal 40A to acquire the test result (step S26A). In addition, the verifying person may visually verify the test result presented by the tested person without using the event site terminal 40A.

Alternatively, in step S24A, the information processing server 20A may transmit information indicating a bar code or the like, in which the test result is embedded, to the user terminal 30A. In this case, the user terminal 30A displays the bar code received from the information processing server 20A. The tested person, for example, presents the bar code displayed on a display unit 34A (refer to FIG. 18) of the user terminal 30A to the verifying person. The verifying person acquires the bar code displayed on the user terminal 30A by, for example, capturing an image of the bar code using the camera of the event site terminal 40A, and reads the embedded information from the acquired bar code to acquire the test result (step S26A).

The event site terminal 40A determines whether the acquired test result satisfies an entrance condition for the event site (step S27A). The entrance condition is, for example, that an antigen test performed up to three days before the start of the event is negative, or the like. In this case, the test result includes information indicating a distinction whether it is negative or positive and the date and time or the like when the test has been performed. It is noted that the entrance condition may be arbitrarily set depending on the size of the site, whether the event is performed indoors or outdoors, or the like, or depending on a state of infection in the region in which the event is performed or a view of the organizer of the event.

If determining that the entrance condition is satisfied, the event site terminal 40A admits the tested person (step S28A). In contrast, if determining that the entrance condition is not satisfied, the event site terminal 40A does not admit the tested person (step S29A).

### (Regarding test strip 100A)

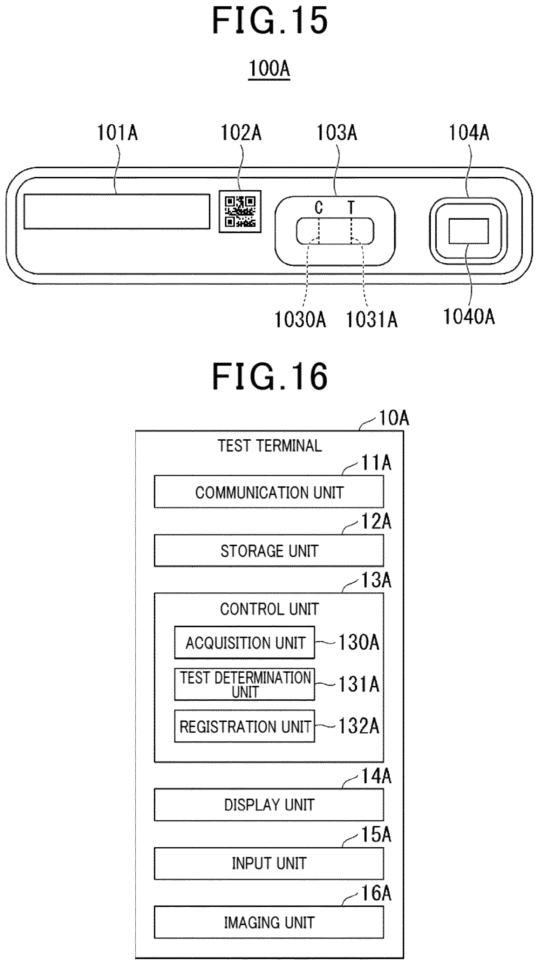

Herein, the test strip 100A will be described with reference to FIG. 15. FIG. 15 is a diagram illustrating an example of the test strip 100A according to the embodiment. The test strip 100A is a test piece serving as a constituent element of a clinical test reagent. The clinical test reagent is used for verify presence or absence of a disease, for example, a lateral flow assay (lateral flow method), an ELISPOT method, and the like, and is an agent for determining the presence or absence of a test object for diagnosing presence or absence of a disease. The lateral flow assay to which immunity assay is applied may be specifically referred to as immunochromatography. FIG. 15 illustrates an example of a test strip used for the lateral flow assay. The test strip 100A includes, for example, a name tag 101A, the two-dimensional code 102A, the coloring part 103A, and a supply part 104A.

The name tag 101A is a column to which the full name and the like of the tested person is written. When a group medical test is performed in a hospital or the like, information such as the full name of the tested person or the like is written on the name tag 101A with a pen, or a sticker on which the information such as the full name of the tested person or the like is printed is attached to the name tag 101A, by, for example, the person in charge of testing or the like. Hence, the tested person whose biological body has been tested by the test strip 100A is verified, whereby the risk of confusion in the group medical test is reduced.

The two-dimensional code 102A is a two-dimensional code image in which the individual identification information of the test strip 100A is embedded. The two-dimensional code may be, for example, a QR code (registered trademark) or a bar code. The individual identification information is information for uniquely identifying the test strip 100A, for example, information indicating a manufacturing number of the test strip 100A or the like.

The coloring part 103A is an area on which a result of a color reaction of the test strip 100A is displayed. The color reaction is a chemical reaction that causes change in color or color development when the biological sample includes the test object. The coloring part 103A includes, for example, a control line 1030A and a test line 1031A. The control line 1030A is an area in which a line appears when the biological sample supplied to the supply part 104A described later migrates to the coloring part 103A normally. The test line 1031A is an area in which a line appears when a virus, an antibody, or the like (test object) to be targeted is present in the biological sample.

The supply part 104A is an area to which the biological sample is supplied. The supply part 104A includes, for example, a biological sample supply window 1040A. For example, dropping the biological sample from the tested person onto the biological sample supply window 1040A supplies the biological sample to the supply part 104A.

It is noted that the clinical test reagents may include a specimen collection jig for collecting a specimen, an extraction liquid, a detection reagent, a correction indicator, or the like.

### (Regarding biological sample)

The biological sample in the present embodiment is a specimen collected from a tested person, for example, mucus collected by rubbing a throat using a cotton swab or the like. The biological sample is desirably a liquid, for example, peripheral blood, blood serum, blood plasma, ascites fluid, urine, cerebrospinal fluid, sputum, saliva, bone marrow, synovial fluid, aqueous humor, amniotic fluid, earwax, mother milk, bronchoalveolar lavage fluid, semen, prostatic fluid, Cowper's fluid or pre-ejaculation fluid, sweat, feces, hair, tears, cyst fluid, pleural fluid or ascites, pericardial fluid, lymph, chyme, chyle, bile, interstitial fluid, menstrual secretion, pus, sebum, vomit, vaginal secretion, secretion from mucous membranes, watery stools, pancreatic fluid, secretions from the nasal cavity, secretions from the pharynx, nasal cavity swab fluid, pharynx swab fluid, fluid from nasal irrigation, bronchoalveolar aspirates, blastocyst cavity fluid, umbilical cord blood, or the like, and includes a substance serving as an indicator when diseases are diagnosed.

### (Regarding diseases)

The diseases are illnesses such as, as representative classification, cancer, hypertension, diabetes, heart disease, cerebrovascular disease, neuropsychiatric disease, immunological/allergy disease, and infectious disease.

### (Regarding disease items)

Disease items further subdivide the above diseases and include a cause, a metabolite, and a phenomenon of the disease. For example, the disease items include coronavirus, influenza, adenovirus, RS virus, rotavirus, hepatitis B virus, hepatitis C virus, HIV, herpesvirus, norovirus, human metapneumovirus, group A beta haemolytic streptococcus, helicobacter pylori, treponema pallidum, mycoplasma, clostridium difficile, mycobacterium, escherichia coli 0157, escherichia coli verocytotoxin, pneumococcus, legionella, procalcitonin, chlamydia, gonococcus, allergic conjunctivitis, interstitial-cell-stimulating hormone (LH), human chorionic gonadotropin (HCG), BNP, NT-proBNP, CK-MB, myoglobin, troponin, D-dimer, H-FABP, granulocyte elastase, carcinoembryonic antigen (CEA), fecal occult blood, insulin-like growth factor-binding protein, fFN, allergy test, CRP, and anti-CCP antibodies.

### (Regarding test object)

The test object in the present embodiment is a substance to be tested with clinical test agents. For example, the test object may be a cell, a bacterium, a virus, an exosome, a nucleic acid, polypeptide (including an antigen and an antibody), polynucleotide, lipid, phospholipids, carbohydrates, polysaccharide, glycoprotein, a low molecular weight compound, a metabolite from a cell or a bacterium, or a single substance (object) such as a fragment of a bacterium, a virus, or an exosome, or a compound substance (object) thereof. In the test items concerning infectious diseases, the test in a case in which the test object is a bacterium or a virus, or polypeptide (antigen) derived from these, lipid, phospholipids, carbohydrates, polysaccharide, or glycoprotein is specifically referred to as an "antigen test". When the "antigen test" is performed, it is determined whether the possibility of infection by a bacterium or a virus is high. In addition, the test in a case in which the test object is a bacterium or a virus, or polynucleotide derived from these is specifically referred to as a "nucleic acid detection lipid". When the "nucleic acid detection test" is performed, it is determined whether the possibility of infection by a bacterium or a virus is high. In addition, the test in a case in which the test object is an antibody is specifically referred to as an "antibody test". When the test is performed, it is determined whether the possibility of infection by a bacterium or a virus in the past or the possibility of production of an antibody in a body due to previous vaccination is high.

### (Regarding test terminal 10A)

Herein, the test terminal 10A will be described with reference to FIG. 16. FIG. 16 is a block diagram illustrating a configuration example of the test terminal 10A according to the embodiment. In the test terminal 10A, for example, the test management application is installed.

As illustrated in FIG. 16, the test terminal 10A includes, for example, a communication unit 11A, a storage unit 12A, a control unit 13A, a display unit 14A, an input unit 15A, and an imaging unit 16A. The communication unit 11A communicates with the information processing server 20A via the communication network NW.

The storage unit 12A is configured by a storage medium such as an HDD (Hard Disk Drive), a flash memory, an EEPROM (Electrically Erasable Programmable Read Only Memory), a RAM (Random Access read/write Memory) and a ROM (Read Only Memory) or a combination thereof. The storage unit 12A stores a program for performing various processes of the test terminal 10A (e.g., a program concerning the test management application) and temporary data used when the various processes are performed.

The control unit 13A is implemented by causing a CPU (Central Processing Unit) that the test terminal 10A includes as hardware to execute the program. The control unit 13A performs overall control of the test terminal 10A. The control unit 13A controls, for example, the display unit 14A and the imaging unit 16A according to the program concerning the test management application.

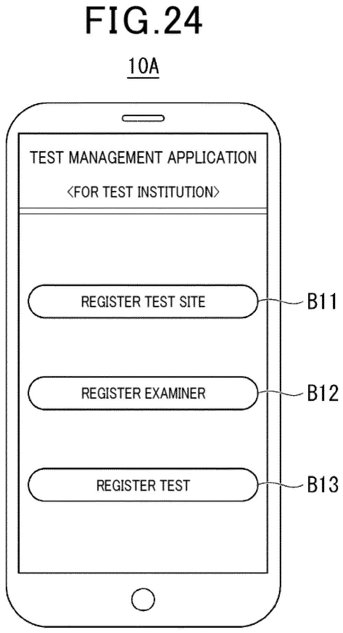

The control unit 13A performs processes according to the program of the test management application. The control unit 13A starts the test management application, for example, when an icon of the test management application is tapped. Hence, the test terminal 10A logs in to the test management application. If the login succeeds, for example, the display unit 14A displays a start image for test institutions of the test management application (refer to FIG. 24).

The control unit 13A includes, for example, an acquisition unit 130A, a test determination unit 131A, and a registration unit 132A. The acquisition unit 130A acquires various pieces of information. For example, the acquisition unit 130A acquires information input by the person in charge of testing or the like via the input unit 15A. For example, the acquisition unit 130A acquires image information on an image captured by the person in charge of testing or the like via the imaging unit 16A.

Processing performed by the acquisition unit 130A will be described with reference to FIG. 24 to FIG. 29. FIG. 24 to FIG. 29 are diagrams for describing processing performed by the test terminal 10A according to the embodiment. FIG. 24 illustrates an example of a start image for test institutions of the test management application.

As illustrated in FIG. 24, the start image for test institutions displays buttons B11 to B13, which are according to respective registration items, which are labelled, for example, "Register test site", "Register examiner", and "Register test". These operation buttons are operated by the person in charge of testing or the like. It is noted that the test terminal 10A may be configured to cause the storage unit 12A to store information on the test site and the examiner, for example, when the test is performed. Hence, information on the test result or the like can be registered without operating the operation buttons of the test management application.

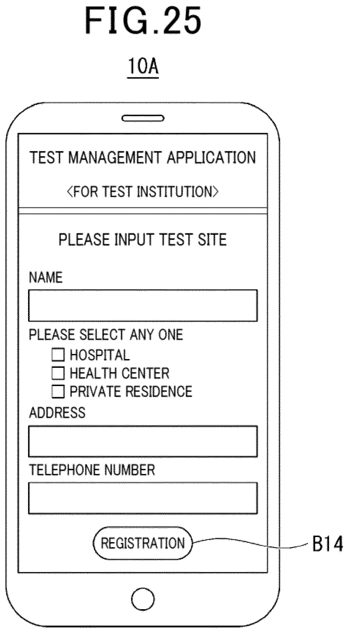

If an operation pressing the button B11 in FIG. 24 is performed, a test site registration image illustrated in FIG. 25 is displayed on 10. The test site registration image is an image for registering a test site. As illustrated in FIG. 25, the test site registration image includes respective input fields in which information corresponding to a name, an address, and a telephone number of the test site in input. In addition, the test site registration image may include check boxes for registering the test site that is any of a hospital, a health center, and a private residence. The person in charge of testing or the like inputs the name and the like of the test site to the input fields and checks a check box, and then performs an operation for pressing the button B14 indicating "Registration". Hence, the acquisition unit 130A acquires, as test site information, information indicated in the input fields. The acquisition unit 130A outputs the acquired test site information to the registration unit 132A.

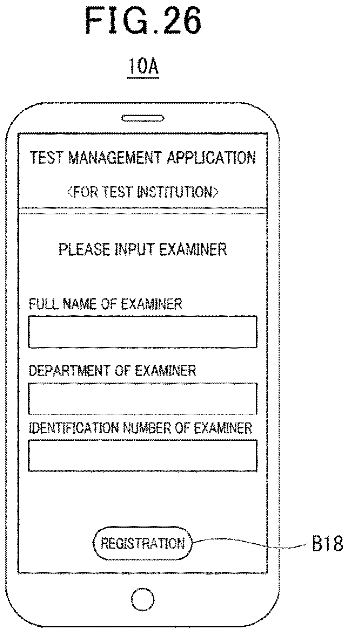

If an operation pressing the button B 12 in FIG. 24 is performed, an examiner registration image illustrated in FIG. 26 is displayed on 10. The examiner registration image is an image for registering an examiner. As illustrated in FIG. 26, the examiner registration image includes respective input fields in which information corresponding to a name, a department, and an identification number of the examiner is input. The person in charge of testing or the like inputs the name and the like of the examiner to the input fields and performs an operation for pressing the button B18 indicating "Registration". Hence, the acquisition unit 130A acquires, as information concerning the examiner, the information indicated in the input fields. The acquisition unit 130A outputs the acquired information to the registration unit 132A.

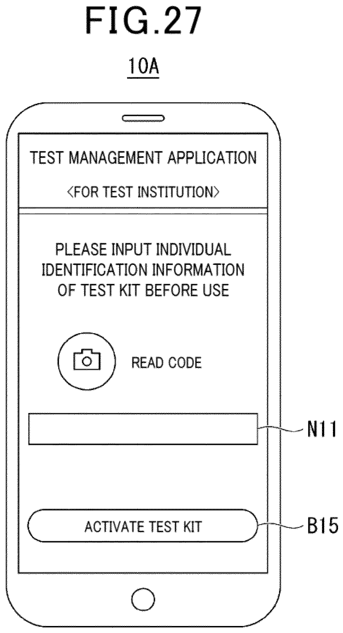

If an operation for pressing the button B13 in FIG. 24 is performed, an examiner image illustrated in FIG. 27 is displayed on the test terminal 10A. The examiner image is an image for registering a test. As illustrated in FIG. 27, the examiner image includes, in addition to such a message as "Please read individual identification information of test kit before use", an input field N11 in which individual identification information is input, and the button B15 indicating "Activate test kit". The test kit corresponds to the test strip 100A of the present embodiment. The person in charge of testing or the like inputs individual identification information to the input field N11. Alternatively, the person in charge of testing or the like operates an icon image to activate the imaging unit 16A and captures an image of the two-dimensional code 102A of test strip 100A with the imaging unit 16A. Hence, the individual identification information embedded in the two-dimensional code 102A is displayed on the input field N11. Then, the person in charge of testing or the like performs an operation for pressing the button B15. Hence, the acquisition unit 130A acquires the individual identification information displayed on the input field N11. The acquisition unit 130A outputs the acquired individual identification information to the registration unit 132A.

If the operation for pressing the button B15 in FIG. 27 is performed, the individual identification information is associated with information concerning the test institution registered in the test application. Specifically, the individual identification information is associated with information such as the test site, the examiner, the date and time of the test, and the test result.

It is noted that in the above, when the individual identification information of the test strip 100A is input, such printed matter as illustrated in FIG.28 may be used to input the individual identification information. In the example in FIG. 28, printed matter of a sticker for the test on which the individual identification information is displayed is illustrated. On the printed matter, the individual identification information or a serial number associated with the individual identification information is indicated as the test ID in each field indicated as a copy for the medical examinee, for test management, or the like. In addition, a two-dimensional code in which the test ID is embedded may be indicated together with the serial number, or instead of the serial number. For example, the person in charge of testing captures an image of the sticker for the test prepared for the test management to input the individual identification information to the test management application. The person in charge of testing pastes the captured image of the sticker for the test onto the test strip 100A. The person in charge of testing hands the tested person a copy, which is for the medical examinee, of the sticker for the test. Hence, the individual identification information is associated with the information concerning the test institution, and the test strip 100A is provided with the individual identification information, whereby the individual identification information is notified to the tested person.

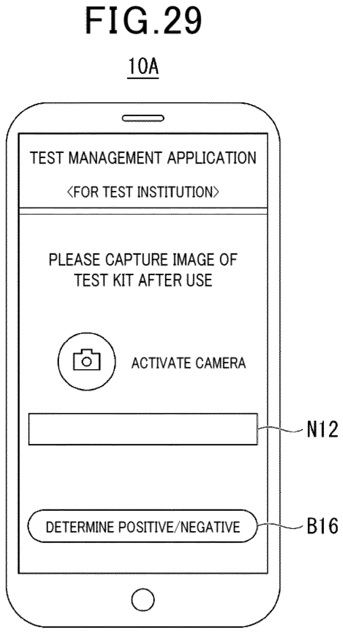

If the test using the test strip 100A is performed, such a reading image as illustrated in FIG. 29 is displayed on the test terminal 10A according to an operation by the person in charge of testing or the like. The reading image is an image for imaging a color indicated on the coloring part 103A of test strip 100A. As illustrated in FIG. 29, the reading image includes, in addition to such a message as "Please capture image of test kit after use", an icon image G11 for activating the imaging unit 16A, a display field N12 on which the test ID is displayed, and a button B 16 indicating "Determine positive/negative". The person in charge of testing or the like operates the icon image to activate the imaging unit 16A to capture an image of the coloring part 103A of the test strip 100A with the imaging unit 16A. The person in charge of testing or the like, for example, confirms whether the coloring part 103A is correctly included in the captured image, and performs an operation for pressing the button B 16. Hence, the acquisition unit 130A acquires image information of the image in which an image of the coloring part 103A is included. The acquisition unit 130A outputs the acquired image information to the test determination unit 131A.

Alternatively, instead of capturing an image of the coloring part 103A after the test, the person in charge of testing, a doctor, or the like who has observed a color state of the coloring part 103A of the test strip 100A may determine whether it is positive or negative. In this case, the acquisition unit 130A acquires information indicating the test result via the input unit 15A and outputs the acquired information to the registration unit 132A.

The test determination unit 131A analyzes the test image acquired from the acquisition unit 130A to determine whether the color indicated by the coloring part 103A indicates that the test object is present, that is, whether it is positive or negative. The test determination unit 131A, for example, compares a pixel value of a pixel of the coloring part 103A with a pixel value of a color serving as a determination criterion. If a difference between the both pixel values on a color space is smaller than a predetermined threshold value, the test determination unit 131A determines that the test object is present, that is, the result is positive. In contrast, if the difference between the both pixel values on the color space is the predetermined threshold value or greater, the test determination unit 131A determines that the test object is not present, that is, the result is negative. The test determination unit 131A outputs the result of the test regarding whether it is positive or negative to the registration unit 132A.

In this case, the test determination unit 131A may correct the test image acquired from acquisition unit 130A to perform the determination using the corrected test image. For example, the test determination unit 131A corrects the color of the test image. In this case, for example, the test strip 100A is provided with a color correction indicator (e.g., a patch for color correction, a color code, or the like), and an image is captured so that the color correction indicator is included in the test image GA. The test determination unit 131A creates a color conversion table by which the pixel value of the color correction indicator whose image is included in the test image GA becomes a pixel value of a color concerning a predetermined color correction indicator. The test determination unit 131A converts the pixel value of the test image GA using the created color conversion table to correct the color of the test image.

The color correction indicator is not limited to be directly provided to the test strip 100A. When a test image is acquired by the imaging unit, the color correction indicator whose image is included in the same test image may be used. For example, when an image of the test strip is captured, paper on which the color correction indicator is printed may be prepared, and an image of the color correction indicator on the paper may also be simultaneously captured, to perform color correction.

In addition, as the color code used as the color correction indicator, for example, cyan, magenta, yellow, black, blue, green, red, a gray scale, or the like may be used. Not only a single color but also a combination of a plurality of color codes may be used. Furthermore, a color gradient may be used and compared with tones of the control line 1030 and the test line 1031 to determine the density of the control line 1030 and the test line 1031.

Alternatively, the test determination unit 131A may correct an angle of the test image. In this case, for example, the test strip 100A is provided with an angle correction indicator. The angle correction indicator is a line parallel to a reference line (e.g., the control line 1030A or the test line 1031A) provided to the test strip 100 or a line perpendicular to the reference line. Then, an image is captured so that the angle correction indicator is included in the test image GA. The test determination unit 131A changes the angle of the test image so that the line of the angle correction indicator whose image is included in the test image is along the vertical direction or the horizontal direction of the image to correct the angle of the test image. It is noted that the case in which the color correction indicator or the angle correction indicator is used to perform the correction is not limiting. The test determination unit 131A may use the two-dimensional code 102A instead of the color correction indicator and the angle correction indicator to perform the correction. Specifically, the test determination unit 131A may use the color of the two-dimensional code 102A to correct the color of the test image. In addition, the test determination unit 131A may correct the angle of the test image depending on an angle between the vertical direction or the horizontal direction of the test image and the vertical direction or the horizontal direction of the two-dimensional code 102A whose image is included in the test image.

The angle correction indicator is not limited to be directly provided to the test strip 100A. When a test image is acquired by the imaging unit, the color correction indicator whose image is included in the same test image may be used. For example, when an image of the test strip is captured, paper on which the color correction indicator is printed is prepared, and an image of the color correction indicator on the paper is also simultaneously captured, to perform color correction.

In addition, as a component to be prepared in addition to the test strip 100A, other than the paper, a masking jig (a jig for covering or holding the test strip 100A to mask an area other than the display area whose image is required to be captured) may be used.

In addition, the test determination unit 131A may use AI (artificial intelligence) to determine whether it is negative or positive. In this case, the test determination unit 131A, for example, inputs the test image to a learned model to acquire an estimated result indicating whether it is negative or positive output from the learned model. The learned model herein is, for example, a model that uses a training data set, which is a set of a training test image and a label indicating whether the training test image indicates negative or positive, to learn a correspondence relationship between the test image and negative or positive. By learning the correspondence relationship, the learned model can accurately output an estimated result estimating whether the input test image indicates negative or positive based on the input test image.

The registration unit 132A transmits the test site information acquired from the acquisition unit 130A to the information processing server 20A. Hence, the test site is registered in a database (not shown) of the information processing server 20A. For example, in an event site, the test site at which a test is undergone may indicate an entrance condition. For example, such entrance conditions may be indicated as that entrance is allowed when the test undergone at a medical institution such as a hospital indicates negative or that entrance is allowed when the test result is negative even when the test is undergone at a residence. When such an entrance condition is indicated, the registered test site information is presented as a test result.

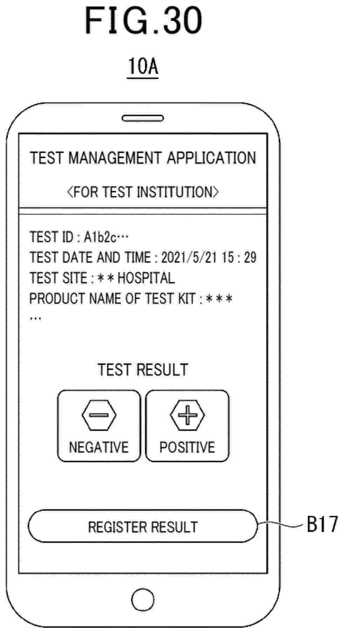

The registration unit 132A registers the test result. Processing for registering the test result by the registration unit 132A will be described with reference to FIG. 30. FIG. 30 is a diagram for describing the processing performed by the test terminal 10A according to the embodiment. FIG. 30 illustrates an example of an image displaying the test result. As illustrated in FIG. 30, for example, the image displaying the test result includes, for example, information indicating the test ID, the date and time of the test, the name of the test site, the product name of the test kit, and the like, result information indicating whether it is positive or negative as the test result, and a button B17 on which an indication "Register result" is shown.

The test ID indicates the individual identification information input in the image illustrated in FIG. 27 as the test ID. The date and time of the test indicates, for example, the date and time when the button B16 in the image illustrated in FIG. 29 was operated. The name of the test site indicates the name of the test site input to the image illustrated in FIG. 25. The product name of the test kit indicates, for example, the product name of the test strip 100A associated with the individual identification information. The result information indicates, as the test result, the determination result of the test determination unit 131A indicating whether it is negative or positive. Alternatively, if whether it is positive or negative is determined by the person in charge of testing, a doctor, or the like, the determination result of the person in charge of testing or the like indicating whether it is positive or negative is indicated as the test result.

If an operation for pressing the button B17 is performed by the person in charge of testing or the like, the registration unit 132A transmits the test ID and the test result to the information processing server 20A. Hence, the registration unit 132A registers the test result.

Returning to the description of FIG. 16, the display unit 14A includes, for example, a display device such as a liquid crystal display and displays images according to control performed by the control unit 13A. The input unit 15A includes, for example, an input device such as a mouse, a keyboard, and a touch panel and outputs information input by operation by the person in charge of testing to the control unit 13A. The imaging unit 16A includes, for example, an imaging device such as a digital camera and captures images according to control performed by the control unit 13A. The imaging unit 16A outputs image information of the captured image to the control unit 13A.

### (Regarding information processing server 20A)

Herein, the information processing server 20A will be described with reference to FIG. 17. FIG. 17 is a block diagram illustrating a configuration example of the information processing server 20A according to the embodiment. The information processing server 20A provides, for example, services concerning the test management application.

As illustrated in FIG. 17, the information processing server 20A includes, for example, a communication unit 21A, a storage unit 22A, and a control unit 23A. The communication unit 21A communicates with the test terminal 10A and the user terminal 30A via the communication network NW.

The storage unit 22A is configured by a storage medium such as an HDD, a flash memory, an EEPROM, a RAM, and a ROM or a combination thereof. The storage unit 22A stores a program for performing various processes of the information processing server 20A and temporary data used when the various processes are performed. The storage unit 22A stores, for example, a test DB 220A, the test result DB 221 A, and the user DB 222A.

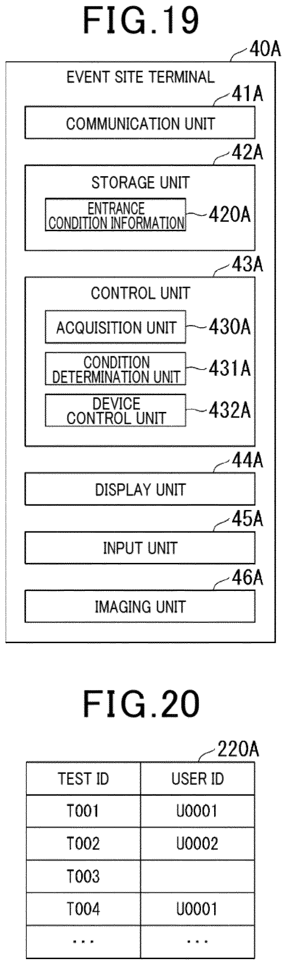

Herein, the information stored in the storage unit 22A will be described with reference to FIG. 20 to FIG. 22. FIG. 20 is a diagram illustrating an example of the test DB 220A according to the embodiment. As illustrated in FIG. 20, the test DB 220A stores user IDs associated with the test IDs. The user ID is identification information for uniquely identifying the user who has registered user information. The user ID is configured by a character string such as a number or a numerical value associated with the user information. The test DB 220A stores only the user IDs and the test IDs and does not store information indicating the full name of a tested person and information indicating a test result and the like.

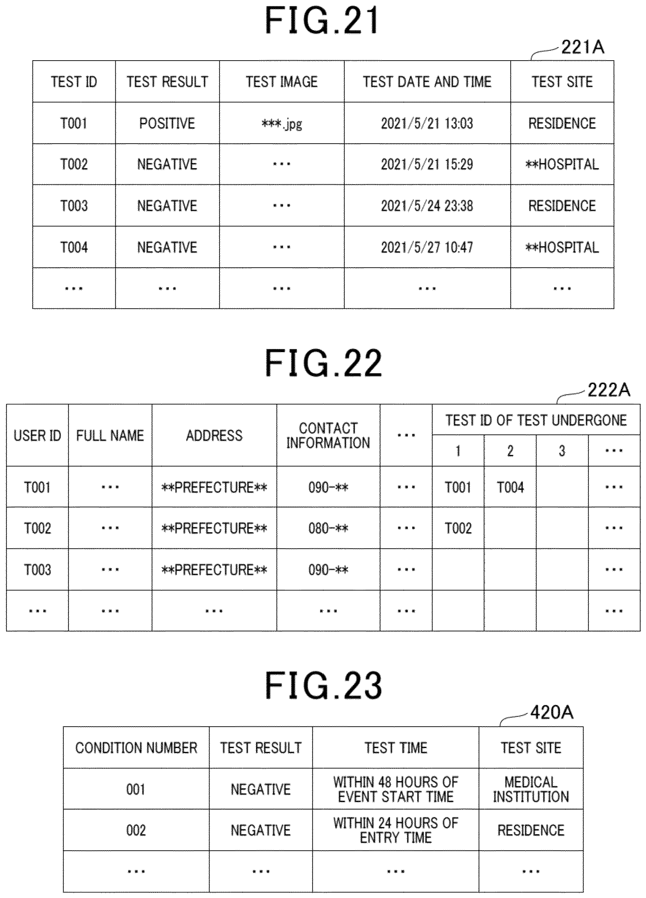

FIG. 21 is a diagram illustrating an example of the test result DB 221A according to the embodiment. As illustrated in FIG. 21, the test result DB 221A stores test information in a state of being associated with the test ID.

The test information includes, for example, information for a test result indicating whether it is negative or positive, a test image, the date and time of the test, a test site, and the like. The test image stores image information of an image including an image of the test coloring part 103A after the test. The date and time of the test stores information indicating the date and time when the test is performed. The test site stores information indicating the site at which the test is performed or the name of a test institution. The test results DB 221A stores only the test information and does not store information indicating the name of a tested person.

It is noted that the test result DB 221A may store each of a plurality of types of test results. For example, each of test results of a PCR test, an antigen test, presence or absence of vaccination, and the like may be stored in the test result DB 221A.

FIG. 22 is a diagram illustrating an example of the user DB 222A according to the embodiment. As illustrated in FIG. 22, the user DB 222A stores user information in a state of being associated with the test ID. The user information includes, for example, information including a user ID, a full name, an address, contact information, and the test ID of the undergone test. The user ID stores identification information uniquely identifying the user information. The full name, the address, and the contact information respectively stores information indicating a full name, an address, and contact information of a user (tested person). The test ID of the undergone test stores a test ID of the test undergone by the user. The user DB 222A stores only the test ID and does not store information indicating a test result or the like.

Returning to the description of FIG. 17, the control unit 23A is implemented by causing a CPU that the information processing server 20A includes as hardware to execute the program. The control unit 23A performs overall control of the information processing server 20A. The control unit 23A includes, for example, an acquisition unit 230A, a registration control unit 231A, a notification determination unit 232A, a test result extraction unit 233A, and a device control unit 234A.

The acquisition unit 230A acquires various pieces of information. For example, the acquisition unit 230A acquires the test result and the test ID provided from the test terminal 10A via the communication unit 21A. In this case, the acquisition unit 230A outputs the acquired information to the registration control unit 231A.

In addition, the acquisition unit 230A acquires the user information and the test ID provided from the user terminal 30A via the communication unit 21A. For example, when user registration is performed, the user information and the test ID are provided from the user terminal 30A. In this case, the acquisition unit 230A outputs the acquired information to the registration control unit 231A. Alternatively, in the case of entering an event site, the user information and the test ID are provided from the user terminal 30A. In this case, the acquisition unit 230A outputs the acquired information to the notification determination unit 232A.

The registration control unit 231A stores the information acquired from the acquisition unit 230A in the storage unit 12A. Specifically, the registration control unit 231A causes the test result DB 221A to store the test result in a state of being associated with the test ID. The registration control unit 231A causes the user DB 222A to store the user information in a state of being associated with the test ID. In this case, the registration control unit 231A provides a user ID to the combination of the user information and the test ID stored in the user DB 222A. The registration control unit 231A causes the test DB 220A to store the user ID in a state of being associated with the test ID.

The notification determination unit 232A determines whether to provide the test result to the user terminal 30A, which is a source, based on the user information and the test ID acquired from the acquisition unit 230A. If the combination of the user information and the test ID provided from the user terminal 30A was previously stored in the user DB 222A, the notification determination unit 232A determines to provide the test result to the user terminal 30A. In contrast, if the combination of the user information and the test ID provided from the user terminal 30A is not stored in the user DB 222 A, the notification determination unit 232A determines not to provide the test result to the user terminal 30A. Hence, the user terminal 30A that has provided information that does not agree with the combination of the user information and the test ID, which were provided when registration was performed, cannot be provided with the test result. Hence, a third party that is not the tested person is not provided with the test result of the tested person, whereby safety of data can be increased.

It is noted that the user information may include a passphrase or a keyword set by the tested person. In this case, when the registration is performed, the user terminal 30A transmits, as the user information, the passphrase or the keyword set by the tested person together with the test ID to the information processing server 20A. The information processing server 20A causes the user DB 222A to store the provided passphrase or keyword in a state of being associated with the test ID. Thereafter, for example, in a case of entering an event site, the user terminal 30A transmits the test ID together with the passphrase or the keyword provided when the registration is performed to the information processing server 20A to request a test result of the test corresponding to the test ID. If the combination of the passphrase or keyword and the test ID provided from the user terminal 30A was previously stored in the user DB 222A, the notification determination unit 232A determines to provide the test result to the user terminal 30A.

The passphrase and the keyword are information set by the tested person when the registration is performed. Hence, a third party who is not the tested person will not know the passphrase and the keyword set by the tested person. Hence, the third party that is not the tested person is not provided with the test result of the tested person, whereby safety of data can be increased.

The notification determination unit 232A outputs the determination result of the determination of whether to inform the user terminal 30A of the test result, to the test result extraction unit 233A.

The test result extraction unit 233A extracts the test result provided to the user terminal 30A. If the notification determination unit 232A determines that the test result is to be provided to the user terminal 30A by the notification determination unit 232A, the test result extraction unit 233A refers to the test result DB 221A based on the test ID acquired from the acquisition unit 230A to acquire the test result associated with the test ID. Hence, the test result extraction unit 233A extracts the test result. The test result extraction unit 233A outputs the extracted test result to the device control unit 234A.

The device control unit 234A performs overall control of the information processing server 20A. For example, the device control unit 234A outputs information indicating the test ID and the like received by the communication unit 21A to acquisition unit 230A. The device control unit 234A outputs the test result extracted by the test result extraction unit 233A to the communication unit 21A, thereby transmitting the test result to the user terminal 30A.

In this case, the device control unit 234A may provide, instead of the test result, a one-time password for displaying the test result to the user terminal 30A. For example, the user terminal 30A provides the test ID and the one-time password to the event site terminal 40A. Such a configuration may be provided that when the test ID and the one-time password are input to, for example, a screen for checking the test result of the test application, the test result corresponding to the test ID is displayed.

### (Regarding user terminal 30A)

Herein, the user terminal 30A will be described with reference to FIG. 18. FIG. 18 is a block diagram illustrating a configuration example of the user terminal 30A according to the embodiment. In the user terminal 30A, for example, the test management application has been installed. Hence, the user terminal 30A can utilize a service provided by the test management application.

As illustrated in FIG. 18, the user terminal 30A includes, for example, a communication unit 31A, a storage unit 32A, a control unit 33A, a display unit 34A, and an input unit 35A. The communication unit 31A communicates with the information processing server 20A via the communication network NW.

The storage unit 32A is configured by a storage medium such as an HDD, a flash memory, an EEPROM, a RAM, and a ROM or a combination thereof. The storage unit 32A stores a program for performing various processes of the user terminal 30A (e.g., a program concerning the test management application) and temporary data used when the various processes are performed.

The control unit 33A is implemented by causing a CPU, which the user terminal 30A includes as hardware, to execute the program. The control unit 33A performs overall control of the user terminal 30A. The control unit 33A, for example, controls the display unit 34A according to the program concerning the test management application.

The control unit 33A performs a process according to the program of the test management application. The control unit 33A starts the test management application, for example, when an icon of the test management application is tapped. Hence, for example, a start image for a medical examinee of the test management application is displayed on the display unit 14A (refer to FIG. 31).

The control unit 33A includes, for example, an acquisition unit 330A, a user registration unit 331A, a test result request unit 332A, and a device control unit 333A.

The acquisition unit 330A acquires various types of information. The acquisition unit 330A acquires information input in the input field of various images displayed on the test management application and information indicating that buttons or the like of the various images have been operated. The acquisition unit 330A outputs the acquired information to at least one of the user registration unit 331A and the test result request unit 332A.

The user registration unit 331A performs user registration. Processing for performing the user registration by the user registration unit 331A will be described with reference to FIG. 31 and FIG. 32. FIG. 31 and FIG. 32 are diagrams for describing processing performed by the user terminal 30A according to the embodiment. In FIG. 31, a start image for a medical examinee in the test management application is displayed on the display unit 34A. In FIG. 32, a registration image for the user registration in the test management application is displayed on the display unit 34A.

As illustrated in FIG. 31, the start image for a medical examinee displays an input field N31 to which individual identification information is input, a button B31 indicating "User registration" and a button B32 indicating "Check test result". When user registration is performed, the tested person inputs individual identification information in the input field N31 and, for example, checks that the input individual identification information contains no errors, and thereafter performs an operation for pressing the button B31. Hence, the user registration unit 331A acquires the individual identification information indicated in the input field N31 as the test ID.

If the user information has been already registered, a configuration may be provided in which the start image for a medical examinee illustrated in FIG. 31 does not display the button B31 on which the indication "User registration" is indicated. In addition, a configuration may be provided in which even in a state in which the user information has not been registered, inputting the test ID can check the test result of the test corresponding to the input test ID.

If an operation for pressing the button B31 is performed, such a registration image as illustrated in FIG. 32 is displayed. The tested person inputs user information such as a full name and an address according to a displayed input form. If confirming that the input full name and address include no errors, the tested person performs an operation for pressing a button B33 indicating an indication that perform "Registration". Hence, the user registration unit 331A acquires the user information such as the full name and the address input in the input field. Herein, the user registration unit 331A may cause the storage unit 32A to store the acquired user information. Hence, when the same user uses the user terminal 30A to utilize the test management application, a step of inputting the user information again can be saved.

The user registration unit 331A transmits the acquired user information together with the test ID to the information processing server 20A. Hence, the user registration unit 331A performs the user registration.

The test result request unit 332A requests a test result. Processing for requesting the test result by the test result request unit 332A will be described with reference to FIG. 31. The tested person inputs individual identification information to the input field N31. When requesting the test result, the tested person inputs the individual identification information to the input field N31 and, for example, confirms that the input individual identification information includes no errors, and thereafter performs an operation for pressing the button B32. Hence, the test result request unit 332A acquires the individual identification information indicated in the input field N31 as the test ID. When the user information was previously stored in the storage unit 32A, the test result request unit 332A refers to the storage unit 12A to acquire the user information.
When no user information is stored in the storage unit 32A, the test result request unit 332A displays an image (not shown) for inputting user information. The test result request unit 332A acquires the user information input by the tested person.

The test result request unit 332A transmits the acquired user information together with the test ID to the information processing server 20A. Hence, the test result request unit 332A requests the test result of the test corresponding to the test ID.

The device control unit 333A performs overall control of the user terminal 30A. For example, if an indication that an operation for, for example, starting the test management application has been performed is input in the input unit 35A, the device control unit 333A provides information indicating that the operation has been performed to (a server providing various images of) the test management application.
In addition, if image information related to the start image is provided from the test management application, the device control unit 333A outputs the image information to the display unit 34A to cause the display unit 34A to display the start image. Alternatively, if the image information related to the registration image is provided from the test management application, the device control unit 333A outputs the information to the display unit 34A to cause the display unit 34A to display the registration image.

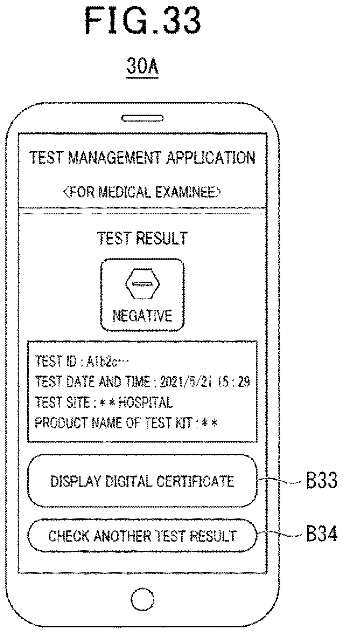

In addition, if the image information related to the image indicating a test result is provided from the test management application, the device control unit 333A outputs the information to the display unit 34A to cause the display unit 34A to display the test result. The image indicating the test result will be described with reference to FIG. 33 to FIG. 35. FIG. 33 to FIG. 35 are diagrams for describing processing performed by the user terminal 30A according to the embodiment.

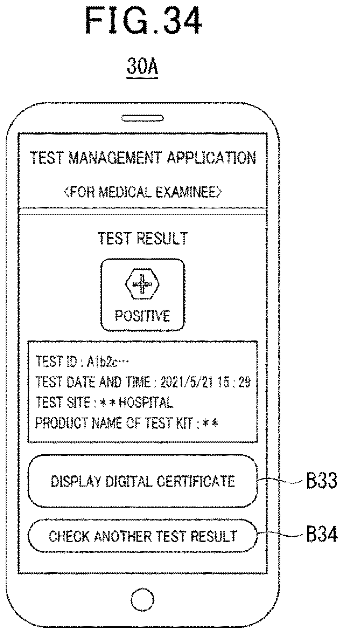

FIG. 33 illustrates an example of an image displayed on the user terminal 30A when the test result is negative. FIG. 34 illustrates an example of an image displayed on the user terminal 30A when the test result is positive. As illustrated in FIG. 33 and FIG. 34, for example, fields may be included which indicate, as the test result, information indicating the test result, for example, an icon image indicating negative or positive, a code corresponding to the test ID, the date and time of the test, the test site, the product name of the test kit, and the like. In addition, as images indicating the test result, the button B33 indicating "Display digital certificate", a button B34 indicating "Check another test result", and the like may be displayed together with the information indicating the test result. The digital certificate herein is a bar code or a two-dimensional code in which the test result is embedded.

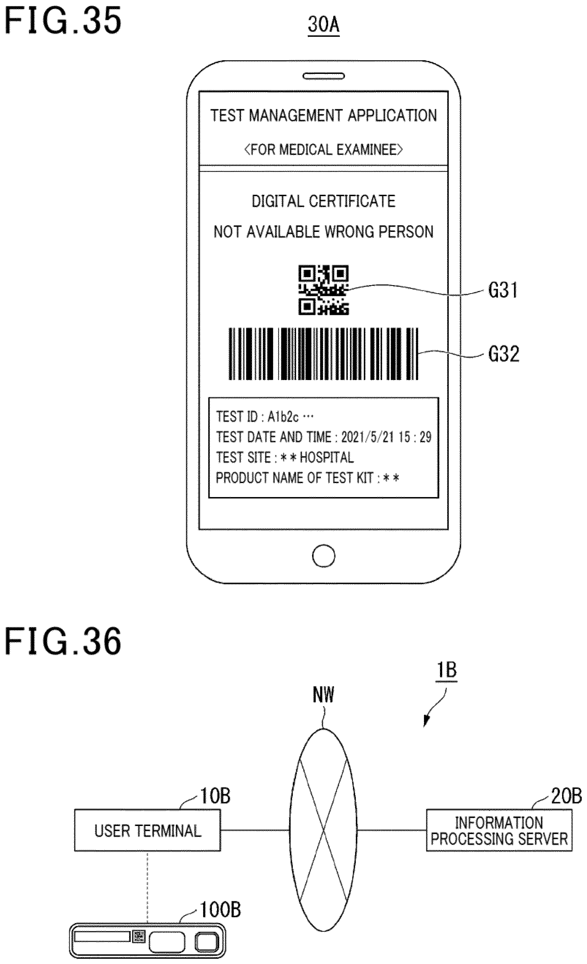

FIG. 35 illustrates an example of an image indicating the digital certificate displayed when an operation for pressing the button B33 has been performed. As illustrated in FIG. 35, the digital certificate is displayed, together with a message such as "not available wrong person", at least one of code images G31 and G32. In the code images G31 and G32, for example, the test result indicating negative or positive is embedded. The image indicating the digital certificate may include, together with the code images G31 and G32, fields indicating the date and time of the test, the test site, the product name of the test kit, and the like.

Returning to the description of FIG. 18, the display unit 34A includes, for example, a display device such as a liquid crystal display and displays images according to control performed by the control unit 33A. The input unit 35A includes, for example, an input device such as a mouse, a keyboard, and a touch panel and outputs information, which is input by operation by the person in charge of testing or the like, to the control unit 33A.

### (Regarding event site terminal 40A)

Herein, the event site terminal 40A will be described with reference to FIG. 19. FIG. 19 is a block diagram illustrating a configuration example of the event site terminal 40A according to the embodiment. The event site terminal 40A includes, for example, a communication unit 41A, a storage unit 42A, a control unit 43A, an input 45A, and an imaging unit 46A. The communication unit 41A communicates with the user terminal 30A via the communication network NW.

The storage unit 42A is configured by a storage medium such as an HDD, a flash memory, an EEPROM, a RAM, and a ROM or a combination thereof. The storage unit 42A stores a program for performing various processes of the event site terminal 40A and temporary data used when the various processes are performed.

The storage unit 42A stores, for example, an entry condition information section 420A. The entry condition information section 420A is information indicating a condition for entering the event site. FIG. 23 illustrates an example of information stored in the entry condition information section 420A according to the embodiment. As illustrated in FIG. 23, the entry condition information section 420A stores, for example, information corresponding to items such as a condition number, a test result, test time, and a test site. The condition number stores identification information such as a number for identifying a condition for entrance. The test result stores information indicating a test result serving as the condition for entrance, for example, information indicating that the test result is negative. The test time stores information indicating a test time serving as the condition for entrance, for example, information indicating that the test must be undergone within 48 hours of the event start time. The test site stores information indicating a test site serving as the condition for entrance, for example, information indicating that the test is undergone at a medical institution.

Returning to the description of FIG. 19, the control unit 43A is implemented by causing a CPU that the event site terminal 40A includes as hardware to execute the program. The control unit 43A performs overall control of the event site terminal 40A.

The control unit 43A includes, for example, an acquisition unit 430A, a condition judgment unit 431A, and a device control unit 432A. The acquisition unit 430A acquires information indicating the test result of the tested person who intends to enter the event site. For example, the acquisition unit 430A acquires an image including an image of the test result displayed on the user terminal 30A as the information indicating the test result of the tested person. Alternatively, the acquisition unit 430A reads a digital certificate displayed on the user terminal 30A to acquire information embedded in a bar code or the like corresponding to the digital certificate as the information indicating the test result of the tested person. The control unit 43A outputs the acquired information indicating the test result of the tested person to the condition judgment unit 431A.

The condition judgment unit 431A determines whether to permit the tested person entrance based on the information indicating the test result of the tested person. The condition judgment unit 431A refers to the entry condition information section 420A to extract the information corresponding to the condition for entering the event site. The condition judgment unit 431A determines whether the test result of the tested person satisfies the condition for entry based on the extracted information and the information indicating the test result acquired from the acquisition unit 430A.

For example, the condition judgment unit 431A refers to, for each item indicated in the entrance condition, the entrance condition and the test result to determine whether the test result satisfies the entrance condition. When the entrance condition indicates that "the test result is negative", if the test result is negative, the condition judgment unit 431A determines, for each item of the test result of the entrance condition, that the test result satisfies the entrance condition. When the entrance condition indicates that "the test must be undergone at a test time which is within 48 hours of the event start time", if the indicated date and time of the test is within 48 hours of the event start time, the condition judgment unit 431A determines, for each item of the date and time of the test of the entrance condition, that the test result satisfies the entrance condition.

For example, if the test result satisfies the entrance condition in all the items indicated as the entrance condition, the condition judgment unit 431A determines to permit the tested person entrance. In contrast, if the test result does not satisfy the entrance condition in at least of the items indicated as the entrance condition, the condition judgment unit 431A determines not to permit the tested person entrance. The condition judgment unit 431A outputs the determination result to the device control unit 432A.

Herein, the condition judgment unit 431A may cause the storage unit 42A to store the test ID for which it is determined to permit entrance.

The device control unit 432A performs overall control of the event site terminal 40A. For example, the device control unit 432A outputs image information on the digital certificate whose image is picked up by the imaging unit 46A to the acquisition unit 230. The device control unit 234A outputs a result of the determination of whether to permit the tested person entrance by the condition judgment unit 431A to a display unit 44A., thereby displaying an indication of whether to permit entrance.

The display unit 44A includes, for example, a display device such as a liquid crystal display and displays images according to control performed by the control unit 43A. The input unit 45A includes, for example, input devices such as a mouse, a keyboard, and a touch panel and outputs information input by operation by the person in charge of testing to the control unit 43A. The imaging unit 46A includes, for example, an imaging device such as a digital camera and captures images according to control performed by the control unit 43A. The imaging unit 46A outputs image information related to the captured image to the control unit 43A.

### (First modification)

The test terminal 10A and the user terminal 30A may be the same terminal. In this case, the test site is a site that is not a medical institution, such as a residence of the tested person. For example, in the registration image illustrated in FIG. 25, a private residence is checked as the test site.

### (Second modification)

The information processing server 20A may hold the test result for a certain period of time and delete the test result from the DB (database) when the period of time has elapsed. For example, the information processing server 20A may delete test information that has elapsed two weeks or more from the date and time of the test from the test result DB 221 A.
In this case, the information corresponding to the test ID corresponding to the information deleted from the test result DB 221 A is deleted from the test DB 220A. In addition, in the user DB 222A, among the test IDs stored as tests undergone by tested persons, the information corresponding to the test ID deleted this time is deleted. It is noted that the period of time for which the test result is held may be arbitrarily set depending on properties of the test such as long-term effectiveness of a test item, a period of time during which the test result is assumed as effective, or the like.

### (Third modification)

The test result presented from the user terminal 30A to the event site terminal 40A may be a test ID or a temporary number associated with the test ID. The event site terminal 40A may request the test result from the information processing server 20A based on the test ID or the temporary number presented from the user terminal 30A.

### (Fourth modification)

A plurality of entrance conditions may be set for one event site. If the test result satisfies any of the set plurality of entrance conditions, entrance may be permitted. Alternatively, only if the test result satisfies all of the set plurality of entrance conditions, entrance may be permitted.

As described above, the information processing system 1A of the embodiment includes the test terminal 10A, the information processing server 20A, and the user terminal 30A. The information processing system 1A is a system that processes information concerning a test using a test piece for testing whether a biological sample includes a test object. The test terminal 10A has the acquisition unit 130A and the registration unit 132A. The acquisition unit 130A acquires individual identification information and test information. The registration unit 132A transmits the individual identification information and the test information to the information processing server 20A. The user terminal 30A includes the acquisition unit 330A and the user registration unit 331A. The acquisition unit 330A acquires the individual identification information the user information. The user registration unit 331A transmits the individual identification information the user information to the information processing server 20A. The information processing server 20A includes the registration control unit 231A. The registration control unit 231A causes the test result DB 221 A to store the information obtained by associating the test information with the individual identification information based on the information received from the test terminal 10A. The registration control unit 231A causes the user DB 222A to store the information obtained by associating the user information with the individual identification information based on the information received from the user terminal 30A.

Herein, the test strip 100A is an example of a "test piece". The acquisition unit 130A is an example of a "first acquisition unit". The acquisition unit 330A is an example of a "second acquisition unit". The test result DB 221 A is an example of an "test result database". The user DB 222A is an example of a "user database".

Hence, the information processing system 1A of the embodiment can increase safety of data. The test result DB 221A and the user DB 222A are databases different from each other. That is, in the present embodiment, the test information and the user information are stored in different databases. Hence, the test result and an individual are not easily associated with each other. Not storing data which would enable the test result concerning the tested person to be easily found can increase safety of data.

It is noted that, in the embodiment described above, the indication that "the test result is negative" is illustrated as an example of the entrance condition. However, this is not limiting. For example, when the test object is a foreign substance (bacteria, virus, or the like) with respect to the biological body, a case in which the test object is not present in the biological sample (it is negative) as a result of the test is determined as "good". In this case, if the test result is negative, entering the event site is permitted. In contrast, when the test object is a substance (antibody or the like) having a role of eliminating foreign substances with respect to the biological body, a case in which the test object is present in the biological sample (positive) as a result of the test is determined as "good". In this case, if the test result is positive, entering the event site is permitted. In this case, the indication that "the test result is positive" is the entrance condition.

In addition, in the embodiment described above, a case is exemplified in which the test determination unit 131A of the test terminal 10A determines whether it is positive or negative. However, this is not limiting. A configuration may be provided in which all or part of the processing performed by the test determination unit 131A is performed by the information processing server 20A. In this case, for example, the test terminal 10A transmits a test image to the information processing server 20A. The information processing server 20A determines whether the result is positive or negative based on the test image received from the test terminal 10 A.

For example, when the test determination unit 131A uses AI (artificial intelligence) to determine whether it is negative or positive, causing the test terminal 10A to store a learned model is likely to consume the memory capacity of the test terminal 10A, which increases the load. In addition, it can be considered that, for example, when there are a plurality of test terminals 10A, management becomes complicated, for example, when the learned model is updated. As a measure against these problems, the information processing server 20A is caused to store the learned model, and the information processing server 20A is caused to determine whether it is negative or positive. According to the configuration, the load of the test terminal 10A is suppressed from increasing, and the management, for example, in the case in which the learned model is updated becomes easy.

In addition, in the embodiment described above, the method of logging in to the test management application may be optionally selected. For example, the login may be executed by inputting a login ID and a password. In this case, for example, when an account is created in the test management application, the login ID and the password of the tested person are registered. Then, if the test management application is started, a screen for inputting a login ID and a password is displayed, and inputting the previously registered login ID and password into the display performs the login. In this case, if the combination of the input login ID and password agrees with the combination of the previously registered input login ID and password, the login succeeds, and accessing the test management application is permitted.

Alternatively, login may be executed using biometric authentication such as fingerprint authentication and face authentication. In this case, in such a case that an account is created in the test management application, an image of a biological body (the fingerprint or the face) of the tested person is registered. Then, if the test management application is started, a screen for capturing an image of the biological body (the fingerprint or the face) is displayed, and capturing an image of the biological body (the fingerprint or the face) of the tested person according to the display performs the login. In this case, if it is determined that the person having the previously registered biological body (the fingerprint or the face) agrees with the person having the biological body whose image has been captured, the login succeeds, and accessing the test management application is permitted.

In addition, in the information processing system 1A, a configuration may be provided in which a user such as the tested person can log in to the test management application based on login information of another service. Herein, the other service is an application service assumed to be utilized by a relatively large number of users, for example, a membership reservation service such as Jalan (registered trademark), a membership information service such as Tabelog (registered trademark), an SNS (Social Networking Service) such as Facebook (registered trademark), and the like. For example, after logging in to the other service, the user can utilize the test management application by enabling functions to be connected to the test management application or selecting a button requiring login information for the other service in a login screen to the test management application. For cooperation between the test management application and the other service, for example, an API (Application Programming Interface) is utilized.

In addition, in FIG. 31 of the embodiment described above, a configuration may be provided in which when the button B32 is pressed without the individual identification information not being input to the input field N31, a list of the tests ID of the tests undergone by the tested person is displayed. In this case, the user terminal 30A transmits only the user information to the information processing server 20A. The information processing server 20A refers to the user DB 222A based on the user information received from the user terminal 30A to satisfy the user ID. The information processing server 20A refers to the test DB 220A based on the specified user ID to acquire the test ID associated with the user ID. The information processing server 20A transmits the acquired test ID to the user terminal 30A.

Alternatively, in FIG. 31, a configuration may be provided in which when the button B32 is pressed without the individual identification information being input to the input field N31, the test result of the latest test that the tested person has undergone is displayed. In this case, the user terminal 30A transmits only the user information to the information processing server 20A. The information processing server 20A refers to the user DB 222A based on the user information received from the user terminal 30A to specify the user ID. The information processing server 20A refers to the test DB 220A based on the specified user ID to acquire the test ID associated with the user ID. The information processing server 20A refers to the test result DB 221A based on the acquired test ID to specify the test ID on the last date and time of the test. The information processing server 20A transmits the test result of the specified test ID, the date and time of the test, and the like to the information processing server 20A.

In addition, in the information processing system 1A, the test result DB 221 A and the user DB 222A may be stored in the same database (hereinafter, referred to as an integration DB). Even in this case, services concerning the test management application can be implemented without changing the respective processes performed by the test terminal 10A, the user terminal 30A, and the event site terminal 40A.

In this case, for example, in step S14A in FIG. 13, the information processing server 20A causes the integration DB to store the test ID and the test result received from the test terminal 10A instead of causing the test result DB 221 A to store them. In addition, in step S18A in FIG. 13, the information processing server 20A causes the integration DB to store the test ID and the user information received from the user terminal 30A instead of causing the user DB 222A to store them.

In addition, in step S22A in FIG. 14, the information processing server 20A refers to the integration DB instead of referring to the user DB 222A based on the test ID received from the user terminal 30A to determine whether to inform the user terminal 30A of the test result. In addition, in step S23 in FIG. 14, the information processing server 20A refers to the integration DB instead of referring to the test result DB 221 A based on the test ID to acquire the test result.

As described above, even when test result DB 221 A and the user DB 222A are stored in the same database, the services concerning the test management application can be implemented by performing the processing as described above by the information processing server 20A.

That is, it is possible that (1) the test terminal 10A reads the two-dimensional code 102A and associates the individual identification information with information on the test institution, (2) a test using the test strip 100A is performed, and (3) the test terminal 10A associates a test result with the individual identification information. In addition, it is possible that (4) the person in charge of testing hands a sticker for the test to the tested person, and (6) the user terminal 30A inputs a test ID and receives information indicating the test result from the information processing server 20A. In addition, it is possible that (7) the tested person presents information indicating the test result displayed on the user terminal 30A to the event site, (8) the event site terminal 40A acquires the test result of the tested person, and (9) the event site terminal 40A determines whether the tested person satisfies the entrance condition for the event site based on the test result of the tested person.

In addition, in the embodiment described above, a case is exemplified in which the test result of the test using the test strip 100A is utilized. However, this is not limiting. As a matter of course, in the information processing system 1A, in addition to the test result or instead of the test result, data indicating a vaccination history may be used. A method similar to that for the data concerning the test can be also applied to data indicating vaccination history.

For example, the test terminal 10A transmits, instead of the test ID and the test result, the type of a vaccine (vaccine ID) and an inoculation history to the information processing server 20A. The information processing server 20A causes the test result DB 221A to store the vaccine ID and the inoculation history received from the test terminal 10A. The user terminal 30A transmits the vaccine ID to the information processing server 20A to request the inoculation history of the vaccine specified by the vaccine ID. The information processing server 20A refers to the test result DB 221A based on the vaccine ID to acquire the inoculation history and informs the user terminal 30A of the acquired inoculation history.

All or part of the information processing system 1A and the information processing server 20 in the embodiment described above may be implemented by a computer. This case may be implemented by storing a program for implementing the functions in a computer readable storage medium and causing a computer system to read the program stored in the storage medium to execute the program. It is noted that the "computer system" herein includes an OS and hardware such as peripheral devices. In addition, "computer readable storage medium" refers to a storage medium such as a portable medium, such as a flexible disk, a magneto-optical disk, a ROM, and a CD-ROM, and a hard disk installed in the computer system. Furthermore, the "computer readable storage medium" may include a medium that dynamically holds a program for a short time, such as a communication line that transmits a program via a network such as the Internet or a communication line such as a telephone line, and a medium that holds a program for a certain time, such as a volatile memory inside a computer system serving as a server or a client in that case. In addition, the program may be a program for implementing part of the function described above, furthermore, a program that can implement the function described above by the combination with the program previously stored in the computer system, or a program that is implemented by using a programmable logic device such as an FPGA.

### (Third embodiment)

Hereinafter, an embodiment of the present invention will be described with reference to the drawings. In the following embodiment, a different reference sign may be appended to even a similar component to that of the first embodiment.

### <Regarding information processing system 1>

FIG. 36 is a block diagram illustrating a configuration example of an information processing system 1B according to the embodiment. The information processing system 1B includes, for example, a test strip 100B, a user terminal 10B, and an information processing server 20B. The user terminal 10B and the information processing server 20B are communicably connected via the communication network NW.

As a configuration for transmitting and receiving information, for the communication network NW, for example, a LAN (Local Area Network), a WAN (Wide Area Network), a telephone network (mobile telephone network, fixed-line telephone network, or the like), a regional IP (Internet Protocol), the Internet, or the like may be applied.

In the information processing system 1B, the user terminal 10B captures an image of the test strip 100B. The user terminal 10B transmits the captured image (a test image GB described later) to the information processing server 20B. The information processing server 20B analyzes the test image GB to determine whether the test strip 100B indicates that the test object is present in the specimen (i.e., whether it is positive). The information processing server 20B informs the user terminal 10B of the determination result.

Alternatively, the user terminal may perform all the processes without using a telemedicine application described later. Specifically, an application program different from the telemedicine application (hereinafter, referred to as an analysis application) may be used to analyze the image by the user terminal 10B. In this case, the user terminal analyzes the image using the analysis application without transmitting the captured image to the information processing server 20B. Hence, the image can be analyzed without using the telemedicine, that is, without using the information processing server 20B, and it can be known whether it is likely to be positive before the user has a medical test at a medical institution.

### <Regarding test strip 100B>

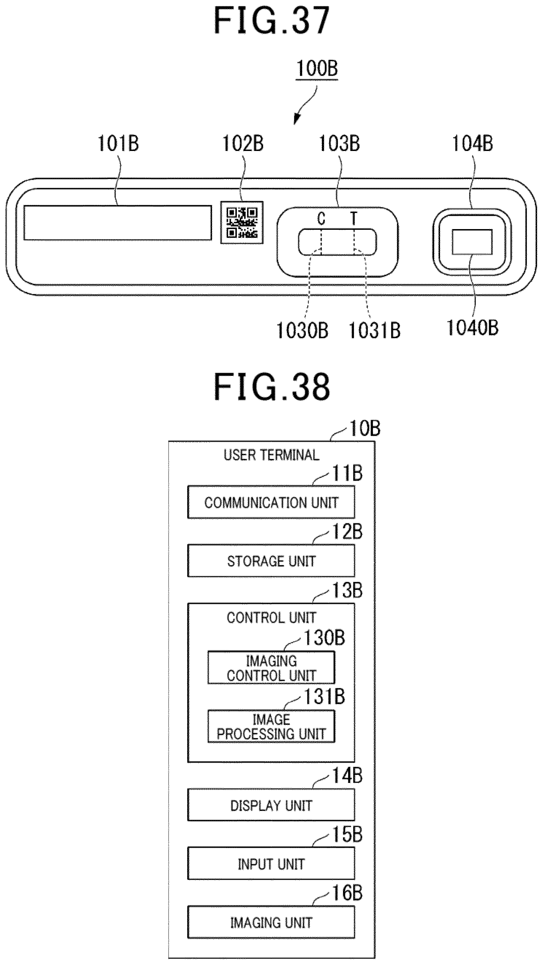

FIG. 37 is a diagram illustrating an example of the test strip 100B according to the embodiment. The test strip 100B is a test piece serving as a constituent element of a clinical test reagent. The clinical test reagent is used for verifying presence or absence of a disease. For example, a lateral flow assay (lateral flow method), an ELISPOT method, or the like is used to determine the presence or absence of a test object for diagnosing presence or absence of a disease. The lateral flow assay to which immunity assay is applied may be specifically referred to as immunochromatography. It is noted that the clinical test reagents may include a specimen collection jig for collecting a specimen, an extraction liquid, a detection reagent, a correction indicator, and the like.

FIG. 37 illustrates, as an example of the test strip 100B according to the embodiment, an example of a test strip used in the lateral flow assay. The test strip 100B includes, for example, a name tag 101B, a two-dimensional code 102B, a coloring part 103B, and a supply part 104B.

The name tag 101B is a column to which the full name or the like of a tested person is written. When a group medical test is performed in a hospital or the like, information such as the full name of the tested person is written on the name tag 101B with a pen, or a sticker on which the information such as the full name of the tested person is printed is attached to the name tag 101B, by, for example, medical personnel such as a nurse, a laboratory technician, or the like.

The two-dimensional code 102B is a two-dimensional code image in which individual identification information of the test strip 100B is embedded. The individual identification information is information uniquely identifying the test strip 100B and, for example, indicating the name of the manufacturer, the lot number, and the manufacturing number of the test strip 100B, the test object, a disease corresponding to the test object, items of the disease, and the like. It is noted that any method may be used to specify the individual identification information in the test strip 100B. For example, when the individual identification information is directly printed on test strip 100B, the two-dimensional code 102B may be omitted.

The coloring part 103B is an area on which a result of a color reaction is displayed. The color reaction is a chemical reaction that causes change in color or color development when the test object is present in a specimen. The coloring part 103B includes, for example, a control line 1030B and a test line 1031B. The control line 1030B is an area in which a line appears when a specimen supplied to the supply part 104B described later migrates to the coloring part 103B normally. The test line 1031B is an area in which a line appears when a virus, an antibody, or the like (test object) to be targeted is present in the specimen. It is noted when there are a plurality of types of test objects, a plurality of test lines 1031B corresponding to the respective test objects may be provided to the coloring part 103B.

The supply part 104B is an area to which the specimen is supplied. The supply part 104B includes, for example, a specimen supply window 1040B. For example, supplying the specimen of the tested person to the specimen supply window 1040B supplies the specimen to the supply part 104B.

### (Regarding specimen)

The specimen in the present embodiment is a specimen collected from a tested person, for example, mucus collected by rubbing a throat using a cotton swab or the like. The specimen is desirably a liquid, for example, peripheral blood, blood serum, blood plasma, ascites fluid, urine, cerebrospinal fluid, sputum, saliva, bone marrow, synovial fluid, aqueous humor, amniotic fluid, earwax, mother milk, bronchoalveolar lavage fluid, semen, prostatic fluid, Cowper's fluid or pre-ejaculation fluid, sweat, feces, hair, tears, cyst fluid, pleural fluid or ascites, pericardial fluid, lymph, chyme, chyle, bile, interstitial fluid, menstrual secretion, pus, sebum, vomit, vaginal secretion, secretion from mucous membranes, watery stools, pancreatic fluid, secretions from the nasal cavity, secretions from the pharynx, nasal cavity swab fluid, pharynx swab fluid, fluid from nasal irrigation, bronchoalveolar aspirates, blastocyst cavity fluid, umbilical cord blood, or the like, and includes a substance serving as an indicator when diseases are diagnosed.

### (Regarding test object)

The test object in the present embodiment is a substance to be tested with clinical test reagents. For example, the test object may be a cell, a bacterium, a virus, an exosome, a nucleic acid, polypeptide (including an antigen and an antibody), polynucleotide, lipid, phospholipids, carbohydrates, polysaccharide, glycoprotein, a low molecular weight compound, a metabolite from a cell or a bacterium, or a single substance (object) such as a fragment of a bacterium, a virus, or an exosome, or a compound substance (object) thereof.

### (Regarding diseases)

The disease are illnesses such as, as representative classifications, cancer, hypertension, diabetes, heart disease, cerebrovascular disease, neuropsychiatric disease, immunological/allergy disease, and infectious disease.

### (Regarding disease items)

Disease items further subdivide the above diseases and include a cause, a metabolite, and a phenomenon of the disease. For example, the disease items include coronavirus, influenza, adenovirus, RS virus, rotavirus, hepatitis B virus, hepatitis C virus, HIV, herpesvirus, norovirus, human metapneumovirus, group A beta haemolytic streptococcus, helicobacter pylori, treponema pallidum, mycoplasma, clostridium difficile, mycobacterium, escherichia coli 0157, escherichia coli verocytotoxin, pneumococcus, legionella, procalcitonin, chlamydia, gonococcus, allergic conjunctivitis, interstitial-cell-stimulating hormone (LH), human chorionic gonadotropin (HCG), BNP, NT-proBNP, CK-MB, myoglobin, troponin, D-dimer, H-FABP, granulocyte elastase, carcinoembryonic antigen (CEA), fecal occult blood, insulin-like growth factor-binding protein, fFN, allergy test, CRP, and anti-CCP antibodies.

### (Regarding user terminal 10B)

FIG. 3 is a block diagram illustrating a configuration example of the user terminal 10B according to the embodiment. The user terminal 10B is a computer of the tested person (user) and is operated by the user. The user may be a patient utilizing telemedicine. The user terminal 10B is implemented by, for example, a smartphone, a tablet terminal, a cellular phone, a PC (Personal Computer), or the like.

In the user terminal 10B, an application (referred to as telemedicine application) that the user operates when receiving a telemedicine service is installed. The telemedicine application is a program for implementing telemedicine by the information processing system 1B. It is noted that, as a matter of course, functions corresponding to the telemedicine application may be implemented by a Web browser. In this case, the user terminal 10B accesses a site specified by a predetermined URL (Uniform Resource Locator) or the like for performing telemedicine via the Web browser. Then, operations concerning telemedicine are performed according to guidance on a screen provided from the accessed site.

As illustrated in FIG. 3, the user terminal 10B includes, for example, a communication unit 11B, a storage unit 12B, a control unit 13B, a display unit 14B, an input unit 15B, and an imaging unit 16B. The communication unit 11B communicates with the information processing server 20B.

The storage unit 12B is configured by a storage medium such as an HDD (Hard Disk Drive), a flash memory, an EEPROM (Electrically Erasable Programmable Read Only Memory), a RAM (Random Access read/write Memory), and a ROM (Read Only Memory) or a combination thereof. The storage unit 12B stores a program for performing various processes of the user terminal 10B (e.g., a program concerning the telemedicine application) and temporary data used when the various processes are performed.

As the program stored in the storage unit 12B, an application program (analysis application) different from the telemedicine application may be included. Herein, it is assumed that the analysis application is a native application, that is, an application program that is installed in a terminal (the user terminal 10B or the like) to operate. However, this is not limiting.

The analysis application may be a program implemented by a Web application. Herein, the Web application is an application program operated by a Web browser.

The display unit 14B includes, for example, a display device such as a liquid crystal display and displays images according to control performed by the control unit 13B. The input unit 15B includes, for example, an input device such as a mouse and a keyboard and outputs information input by operation by the user to the control unit 13B. The imaging unit 16B includes, for example, an imaging device such as a digital camera and captures images according to control performed by the control unit 13B.

The control unit 13B is implemented by causing a CPU (Central Processing Unit) that the user terminal 10B includes as hardware to execute programs. The control unit 13B performs overall control of the user terminal 10B. The control unit 13B controls, for example, the display unit 14B and the imaging unit 16B according to the program concerning the telemedicine application.

The control unit 13B performs processing according to the program of the telemedicine application. The control unit 13B starts the telemedicine application, for example, in response to an input operation by the user, for example, when an icon of the telemedicine application is tapped. Hence, for example, an operation screen of the telemedicine application is displayed on the display unit 14B. On the operation screen, for example, a plurality of operation buttons which are labelled "Capture test image GB", "Log out", and the like are displayed. When the operation button which is labelled "Capture test image GB" is tapped by the user, the control unit 13B activates the imaging unit 16B to display an imaging screen for capturing the test image GB.

The control unit 13B includes, for example, an imaging control unit 130B and an image processing unit 131B. The imaging control unit 130B controls capturing the test image GB. Specifically, when the test image GB is captured, the imaging control unit 130B causes the display unit 14B to display the imaging area. For example, the user visually recognizes the imaging area displayed on the display unit 14B and adjusts the imaging direction and the distance to a subject so that the imaging area includes an area (coloring area) colored in the coloring part 103B. Hence, an image including the coloring area of the test strip 100B can be captured as the test image GB.

In addition, the imaging control unit 130B may specify the coloring area included in the imaging area to perform image processing of the imaging area based on the specified coloring area. For example, when an image of the test strip 100B is captured by a smartphone, image files are often stored in a red-green-blue (RGB) space. The imaging control unit 130B converts such an image file stored in the RGB space to a color space different from the RGB space. The color space different from the RGB space is, for example, a hue- saturation-lightness (HSL) space, a hue-saturation-value (HSV) space, white-black (gray scale) space, or the like. The imaging control unit 130B may convert the image file stored in the RGB space to any color space among the HSL space, the HSV space, and the gray scale space. For example, when the image file stored in RGB is converted to the HSL space or the HSV space, noise is appropriately removed depending on the imaging environment. For example, when the image file stored in RGB is converted to the gray scale space, influence of the color can be difficult to exert by simplifying color information on the image.

Alternatively, the imaging control unit 130B may specify a coloring area included in the imaging area to display a frame enclosing the specified coloring area. Hence, the coloring area in the test image GB can be easily specified, whereby it can be easily determined whether it is positive.

Alternatively, the imaging control unit 130B may cause the display unit 14B to display a guide indicating an outline of the coloring part 103B. Hence, orientations and sizes of the coloring areas of the test images GB can be made to coincide with each other, whereby it can be easily determined whether it is positive. In addition, since an imaging method used when a user captures an image of the test strip 100B, for example, variations in orientations and sizes of the coloring areas of the test images GB can be suppressed, it can be easily determined whether it is positive.

Herein, when an image of the test image GB is captured, a masking jig may be used. The masking jig is a jig physically covering part different from the coloring part 103B of the test strip 100B. In other words, the masking jig masks parts other than the coloring part 103B. For example, the masking jig is a paper board provided with a window. A user places the paper board on the test strip 100B so that the coloring part 103B can be seen through the window of the paper board, thereby physically masking the part different from the coloring part 103B. Then, in a state in which the masking jig is placed on the test strip 100B, the user operates imaging unit 16B to capture an image of the test strip 100B.

Hence, an image whose color of areas different from the coloring area is masked can be captured as the test image GB, color information of the areas different from the coloring area of the test image GB is simplified to make it difficult that the color of the coloring area is easily influenced by the color of areas different from the coloring area, whereby whether it is positive can be determined with high accuracy. In addition, when the test strip 100B includes the name tag 101B and the two-dimensional code 102B, information included therein is unique to each individual. In this case, various pieces of information indicated in areas different from the coloring area act as noise, which may lower the determination accuracy. As a measure for this, in the present embodiment, the configuration is provided in which the masking jig masks the information unique to each individual. Hence, the information indicated in areas different from the coloring area of the test image GB can be removed. Hence, influence of areas different from the coloring area can be difficult to exert, whereby whether it is positive can be determined with high accuracy.

In addition, a color correction indicator or an angle correction indicator may be printed on the masking jig.

The image processing unit 131B performs image processing on an image captured by the imaging unit 16B. The image processing unit 131B performs image processing so that the contents approach the contents learned by a determination model described later. For example, while the determination model is a model that has been learned from black and white images, if a color image is captured by the imaging unit 16B, the image processing unit 131B performs image processing for converting the color of the image to black and white.

For example, while the determination model is a model that has been learned from an image file in a first format, if an image in a second format is captured by the imaging unit 16B, the image processing unit 131B performs image processing for converting the file format of the image from the second format to the first format.

For example, while the determination model is a model that has been learned from an image including only an image of the coloring area, if an image including areas different from the coloring area is captured by the imaging unit 16B, the image processing unit 131B performs trimming processing for trimming the areas different from the coloring area. Herein, the image including the areas different from the coloring area is, for example, an image of the whole test strip 100B or an image including a stand on which the test strip 100B is placed or the like.

In addition, the image processing unit 131B may perform image processing that converts the shape of an image as required. The image processing that converts the shape of an image includes, for example, processing for rotating the image and processing for expanding the coloring area. In addition, the image processing that converts the shape of an image may include processing that shifts the color correction indicator in the horizontal direction and the vertical direction, horizontal reverse processing that reverses the image with respect to an axis of symmetry along the vertical direction, vertical reverse processing that reverses the image with respect to an axis of symmetry along the horizontal direction, and the like. In addition, the image processing that converts the shape of an image may include trapezoid correction and utilize the angle correction indicator.

The angle correction indicator may not only be directly applied to the test strip 100B but, when a test image is captured by the imaging unit, may use the color correction indicator whose image is included in the same test image. For example, when an image of the test strip is captured, paper on which the color correction indicator is printed is prepared, and an image of the color correction indicator on the paper is also simultaneously captured, to perform color correction.

In addition, the image processing unit 131B may perform image processing that converts the color of an image as required. In this case, for example, the image processing unit 131B may convert color information of each pixel in the image by using a prepared conversion table to convert the color of the image, or utilize the color correction indicator.

When the color correction indicator is used, for example, the test strip 100B is provided with the color correction indicator (e.g., a patch for color correction, a color code, or the like), and images are captured so that the color correction indicator is included in the test image G. As the color code used as the color correction indicator, for example, cyan, magenta, yellow, black, blue, green, red, a gray scale, or the like is used. Not only a single color but also a combination of a plurality of color codes may be used. Furthermore, a color gradient may be used and compared with tones of the control line 1030 and the test line 1031 to determine the density of the control line 1030 and the test line 1031.

The color correction indicator is not limited to be directly provided to the test strip 100B. When a test image is acquired by the imaging unit, the color correction indicator whose image is included in the same test image may be used. For example, when an image of the test strip is captured, paper on which the color correction indicator is printed is prepared, and an image of the color correction indicator on the paper is also simultaneously captured, to perform color correction.

In addition, as a component to be prepared in addition to the test strip 100B, other than the paper, a masking jig (a jig for covering or holding the test strip 100B to mask an area other than the display area whose image is required to be captured) or a holding jig may be used.

The control unit 13B transmits, as the test image GB, an image, which is obtained by subjecting the image captured by the imaging unit 16B to the required image processing by the image processing unit 131B, to the information processing server 20B. For example, the control unit 13B causes an operation button, which is labelled "Send image" or the like, to be displayed. When "Send image" is tapped by the user, the control unit 13B transmits the test image GB to the information processing server 20B via the communication unit 11B.

### (Regarding information processing server 20B)

The information processing server 20B is a computer of the tested person. The information processing server 20B is implemented by, for example, a cloud device, a server device, a PC, or the like. The information processing server 20B is, for example, a server device that manages a site concerning the telemedicine application.

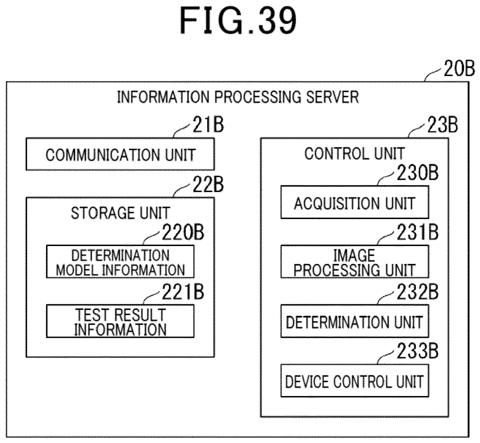

FIG. 39 is a block diagram illustrating a configuration example of the information processing server 20B according to the embodiment. The information processing server 20B includes, for example, a communication unit 21B, a storage unit 22B, and a control unit 23B. The communication unit 21B communicates with the user terminal 10B.

The storage unit 22B is configured by a storage medium such as an HDD, a flash memory, an EEPROM, a RAM, and a ROM or a combination thereof. The storage unit 22B stores a program for performing various processes of the information processing server 20B and temporary data used when the various processes are performed. The storage unit 22B stores determination model information 220B and test result information 221B.

The determination model information 220B is information indicating the determination model, which is a learned model. For example, if the determination model is a CNN, the determination model information 220B includes information indicating the number of units of each of an input layer, a hidden layer (intermediate layer), and an output layer, the number of hidden layers, an activation function, and the like.

The determination model is a learned model that has learned a correspondence relationship between an image and a positive result by performing machine learning using a training data set. The training data set is information of a set obtained by appending, to a training image, a sign that indicates whether the coloring area in the training image indicates a positive result. The training image of the present embodiment is an image of an unspecified test strip captured so as to include the coloring area of the test strip. The determination model is generated, for example, by repeatedly learning the correspondence relationship until the degree of positive indication in the input image can be estimated with high accuracy. If an unlearned image (e.g., the test image GB) is input, the determination model estimates the degree of positive indication in the image and outputs the estimated result.

The training image that has been learned by the determination model includes a plurality of images of the same subject captured in different imaging environments. For example, the training image includes respective images of the same test strip 100B captured in states in which illumination light is emitted from lighting units having different spectral radiation characteristics, for example, a fluorescent lamp and an incandescent lamp. In addition, the training image includes respective images of the same test strip 100B captured by different types of imaging devices having, for example, different color resolutions. In addition, the training image includes respective images of the same test strip 100B captured from different imaging directions. Using the determination model that has been learned by using the training image including a plurality of images of the same subject captured in different imaging environments can determine, when the test images GB captured in various imaging environments are input, the degree of positive indication in the test images GB with high accuracy.

In addition, the training images that the determination model has used for learning includes a plurality of images having different concentrations of the test object (hereinafter, referred to as object concentrations) supplied to the test strip. Typically, when a specimen having a high object concentration is supplied, a line indicating a positive result with a deep and clear color appears in the test line 1031B. In contrast, when a specimen having a low object concentration is supplied, a line indicating a positive result with a light and indistinct color appears, or the line indicating a positive result cannot be visually recognized with the naked eye.

It is noted that the plurality of images may include only one or a plurality of images having a certain concentration.

Herein, when the specimen is supplied to the test strip 100B, the test object in the specimen combines with colored particles. The colored particle is a particle that absorbs or reflects light so that the test object can be visually recognized as a line when the test object has migrated to the test line 1031B. Next, the specimen, which is a liquid, migrates within a membrane, which is a solid. At this time, the test object in the specimen migrates from the supply part 104B toward the coloring part 103B and reaches a position at which the control line 1030B and the test line 1031B are indicated. In addition, simultaneously, the colored particles that have not combined with the test object migrate from the supply part 104B toward the coloring part 103B while being absorbed to the membrane gradually, and reach an area beyond the control line 1030B. This means that the colored particles are absorbed to the whole coloring area gradually as a background. When a specimen having a high object concentration is supplied, a sufficient amount of test object reaches the test line 103 1B. Hence, a deep and clear line that can be visually recognized with the naked eye appears on the test line 1031B. In contrast, when a specimen having a low object concentration is supplied, only a small amount of test object reaches the test line 1031B. Hence, the clear and deep line does not appear on the test line 1031B, whereby it becomes difficult to distinguish the test line 1031B and an area therearound from each other. Hence, it becomes difficult to determine whether the specimen has migrated to the test line 1031B normally. It can be considered that such a situation is a factor for difficulty in determining whether it is positive when a specimen having a low object concentration is supplied.

It is desirable that even when the test object having a concentration low enough not to be visually recognized with the naked eye is supplied, a determination can be made with high accuracy. As a measure for this, in the present embodiment, a determination model is used which has been learned by using a training image including a plurality of images including test objects supplied to the test strip and having different concentrations. It is desirable that the plurality of images including test objects having different concentrations include, specifically, an image corresponding to a case in which the object concentration is low enough not to be able to be visually recognized as a line indicating positive with the naked eye. Hence, even when it is difficult to determine whether it is positive with the naked eye, the determination model can make a determination with high accuracy.

In addition, the breakdown of the training image may be determined depending on the object required to be determined by the determination model with high accuracy. For example, the number of low-concentration images included in the training image may be larger than the number of high-concentration images. The low-concentration image herein is an image corresponding to a case in which a specimen having an object concentration low enough not to be visually recognized with the naked eye is supplied. The high-concentration image is an image corresponding to a case in which a specimen having an object concentration high enough to be visually recognized with the naked eye is supplied. Hence, even in a case of a low-concentration image, a determination model can be generated which can determine whether the image indicates a positive result with high accuracy.

Herein, a situation will be described in which it is desired to determine whether a positive result is indicated with high accuracy even in a case of a low-concentration image. For example, when the test object is a virus having high infectivity, even if a small number of viruses are present inside the body of the user who has provided a specimen, other persons may be infected. Hence, in such a situation in which it is required to efficiently find a user who is likely to infect others, it is required that it is determined that a positive result is indicated, that is, the specimen includes the test object with high accuracy even in a case of a low-concentration image.

In addition, the determination model may not only determine whether the image indicates a positive result but also estimate an object concentration of the specimen. In this case, the determination model is a learned model that has learned a correspondence relationship between an image and an object concentration. The training data set includes information that is a set obtained by appending, to a training image, a sign that indicates the object concentration in the training image. The determination model may be a model that determines whether the image indicates a positive result and estimates the object concentration based on the image, and may be configured by two learned models, a first model that estimates whether it is positive and a second model that estimates the object concentration. In addition, the determination model may estimate the object concentration in addition to whether the image indicates a positive result, or may estimate only the object concentration.

The test result information 221B is information indicating a determination result based on the test image GB. The determination result is a result of a determination of whether the coloring part 103B whose image is captured as the test image GB indicates a positive result. The determination result may be information indicating the degree of positive indication in the test image GB.

For example, a configuration may be provided in which when the determination model is a model that estimates the degree of positive indication in the input test image GB, the information indicating the degree of positive indication in the test image GB output from the determination model is stored as the determination result. In addition, the test result information 221B may store the determination result and the test image GB in a state of being associated with each other.

The control unit 23B is implemented by causing a CPU that the information processing server 20B includes as hardware to execute the program. The control unit 23B performs overall control of the information processing server 20B. The control unit 23B includes, for example, an acquisition unit 230B, an image processing unit 231B, a determination unit 232B, and a device control unit 233B.

The acquisition unit 230B acquires the test image GB provided from the user terminal 10B via the communication unit 21B. The acquisition unit 230B outputs the acquired test image GB to the image processing unit 231B.

The image processing unit 231B performs image processing of the test image GB. Since the processing performed by the image processing unit 231B is similar to the processing performed by the image processing unit 131B, description thereof is omitted. For example, instead of the image processing unit 131B of the user terminal 10B, the image processing unit 231B performs the image processing of the test image GB.

Hence, a processing load of the user terminal 10B can be reduced. The image processing unit 231B outputs the test image GB subjected to image processing as required to the determination unit 232B. Specifically, if image processing is not required, the image processing unit 231B outputs the test image GB acquired from the image processing unit 231B to the determination unit 232B unchanged. If image processing is required, the image processing unit 231B outputs an image, which is obtained by subjecting the test image GB acquired from the image processing unit 231B to the required image processing, to the determination unit 232B.

The determination unit 232B determines whether the coloring area in the image acquired from the image processing unit 231B indicates a positive result, by using the determination model. The determination unit 232B determines whether the image indicates a positive result based on the estimated result acquired by inputting the image to the determination model. For example, if the estimated result output from the determination model is that the degree of positive indication in the image is a threshold value or more, the determination unit 232B determines that the coloring area in the image indicates a positive result. For example, if the estimated result output from the determination model is that the degree of positive indication in the image is less than the threshold value, the determination unit 232B determines that the coloring area in the image indicates negative.

The threshold value is set to, for example, a value such as 50%, 75%, 90%, 95%, 98%, and 99% depending on the situation. It is noted that, in the above description, a case is exemplified in which the determination unit 232B determines whether it is positive or negative based on the degree of positive indication in the input image. However, this is not limiting. The determination model may be configured to output any of a positive indication and a negative indication based on the degree of positive indication in the image. In this case, the determination unit 232B employs a result indicating any of a positive indication and a negative indication without change.

In addition, the determination unit 232B may determine, instead of whether it is positive, any of whether it is positive, whether it is negative, and whether it is neither positive nor negative, that is, whether a determination cannot be made. For example, if the estimated result output from the determination model is that the degree of positive indication in the image is a first threshold value or more, the determination unit 232B determines that the coloring area in the image indicates positive.
If the estimated result output from the determination model is that the degree of positive indication in the image is lower than a second threshold value, which is lower than the first threshold value, the determination unit 232B determines that the coloring area in the image indicates negative. If the estimated result output from the determination model is that the degree of positive indication in the image is the second threshold value or more and less than the first threshold value, the determination unit 232B determines that the coloring area in the image indicates neither positive nor negative, that is, a determination cannot be made. It is noted that the determination model may be configured to output any of a positive indication, a negative indication, and an indication that a determination cannot be made, based on the degree of positive indication in the image.

The determination unit 232B causes the test result information 221B to store the determination result. The determination unit 232B may cause the test result information 221B to store, together with the determination result, the test image GB and the degree of positive indication in the image. In addition, when the determination model estimates an object concentration, the determination unit 232B may cause the test result information 221B to store the test object concentration as the test result.

The device control unit 233B performs overall control of the information processing server 20B. For example, the device control unit 233B outputs the test image GB received by the communication unit 21B to the acquisition unit 230B. The device control unit 233B may transmit the determination result to the user terminal 10B via the communication unit 21B.

In the present embodiment, the estimated result of the estimation of the determination model, that is, not the degree of positive indication, any of "positive" and "negative" or any of "positive", "negative", and "determination not possible" is stored in the test result information 222B. Hence, not just medical personnel or an expert on AI but a general user can easily grasp the test result.

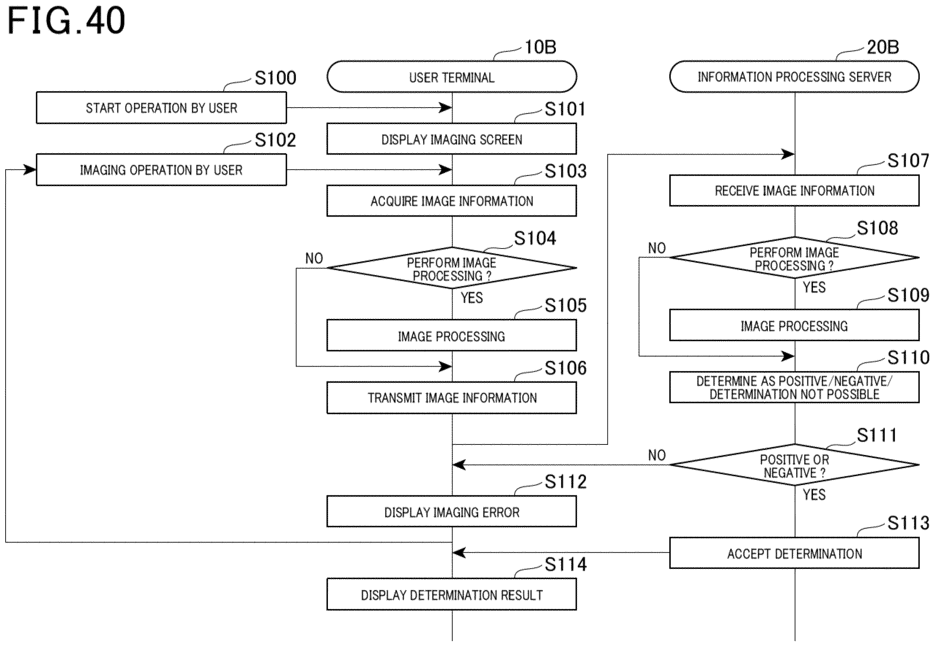

FIG. 40 is a sequence diagram illustrating a flow of processing performed by the information processing system 1B according to the embodiment. First, a user operates the user terminal 10B to start the telemedicine application (step S100). The user terminal 10B starts the telemedicine application in response to the start operation by the user and displays an imaging screen for capturing an image of the test strip 100B (step S101). The test strip 100B herein is a test strip after the test in which a specimen of the tested person is supplied. The user visually recognizes the imaging screen displayed on user terminal 10B and performs an adjustment so that the imaging area includes the coloring part 103B, and performs an operation for capturing an image (step S102). The user terminal 10B captures an image with the imaging unit 16B in response to an imaging operation by the user and acquires image information of the test image GB (step S103). The user terminal 10B determines whether to perform image processing of the test image GB (step S104). If determining to perform image processing of the test image GB, the user terminal 10B performs required image processing (step S105), and transmits the test image GB, which has undergone the image processing, to the information processing server 20B (step S106). In contrast, if determining not to perform image processing of the test image GB, the user terminal 10B does not perform image processing, and transmits the test image GB to the information processing server 20B.

The information processing server 20B receives the test image GB and the like (step S107). The information processing server 20B determines whether to perform image processing of the received test image GB (step S108). If determining to perform image processing of the test image GB, the information processing server 20B performs required image processing (step S109) and determines whether the test image GB that has undergone the image processing indicates positive, negative, or determination not possible by using a determination model (step S110). In contrast, if determining not to perform image processing of the test image GB, the information processing server 20B determines whether the test image GB indicates positive, negative, or determination not possible based on the test image GB, which is not changed, by using the determination model. If the brightness of the test image GB is less than a threshold value, the information processing server 20B may determine that determination is not possible. In this case, in step S107, the information processing server 20B determines whether the brightness of the received test image GB is the threshold value or more. The brightness of the test image GB herein is, for example, an average value of pixel values in the coloring area.

If the brightness of the test image GB is less than the threshold value, the information processing server 20B determines that determination is not possible. In this case, the information processing server 20B does not perform the processing indicated in steps S108 to S110.

The information processing server 20B determines whether the determination result is negative, positive or the other (determination not possible) (step S111). If the determination result is "determination not possible" the information processing server 20B informs the user terminal 10B of that effect. The user terminal 10B displays an imaging error in response to the notification from the information processing server 20B (step S112). For example, the imaging error indicates again a message for prompting capture of the image of the test strip 100B because it cannot be determined whether it is negative, positive or the other from the captured image. If the user visually recognizes the imaging error and captures an image again, returning to S102, the user performs an imaging operation. It is noted that, in step S112, the information processing server 20B may inform the user terminal 10B of, in addition to the indication that the determination result is "determination not possible", a suggestion concerning an imaging environment and the like. In this case, the user terminal 10B displays a message for simply prompting capture of the image again, or displays a message accompanied with the suggestion concerning an imaging environment, for example, a message such as "please capture image in a brighter place" if determination is not possible due to lack of brightness of the test image GB.

In contrast, in step S111, if the determination result is negative or positive, the information processing server 20B confirms the determination result (step S113). The information processing server 20B transmits the confirmed determination result to the user terminal 10B. The user terminal 10B displays the determination result received from the information processing server 20B (step S114).

### (First modification of embodiment)

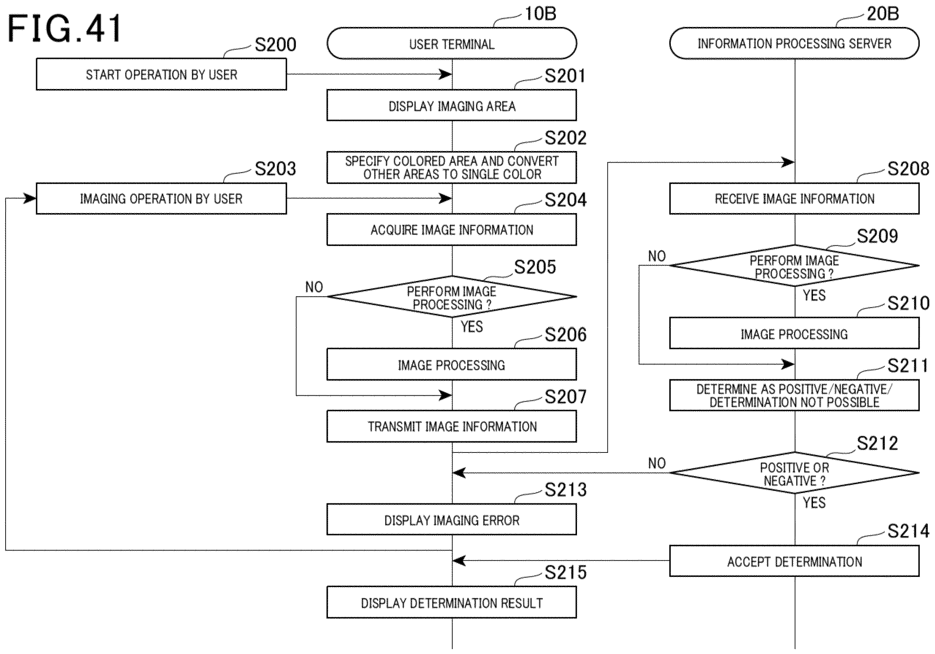

FIG. 41 is a sequence diagram illustrating a flow of processing performed by the information processing system 1B according to the first modification of the embodiment. The present modification differs from the embodiment described above in that image processing is performed when an image is captured, and the image that has undergone the image processing is captured. The image processing herein performed when an image is captured is processing by which a color in areas different from the coloring area is converted to a single color (e.g., white).

Since steps S200 to S201 illustrated in FIG. 41 are similar to steps S100 to S101 illustrated in FIG. 40, description thereof is omitted. Since steps S203 to S215 illustrated in FIG. 41 are similar to steps S102 to S114 illustrated in FIG. 40, description thereof is omitted. That is, step S202 will now be described.

The user terminal 10B displays an imaging area and acquires an image indicating the imaging area. The user terminal 10B specifies the coloring area included in the imaging area based on the acquired image and converts a color of areas different from the specified coloring area to a single color (e.g., white) (step S202). The user terminal 10B displays an image in which the color of areas different from the coloring area is converted to a single color.

It is noted that, in the present modification, a masking jig may be used. When the masking jig is used, the user captures an image of the test strip 100B in a state in which the masking jig is present. In this case, the user terminal 10B may omit the image processing when an image is captured. Alternatively, the user terminal 10B may determine whether the masking jig is present and display a warning indicating that the masking jig is not present. In addition, at a specific position on the masking jig, a two-dimensional code may be printed which includes an individual identification number as information. In addition, a color correction indicator may be printed on the test strip 100B. In this case, it can also be utilized for color correction.

### (Second modification of embodiment)

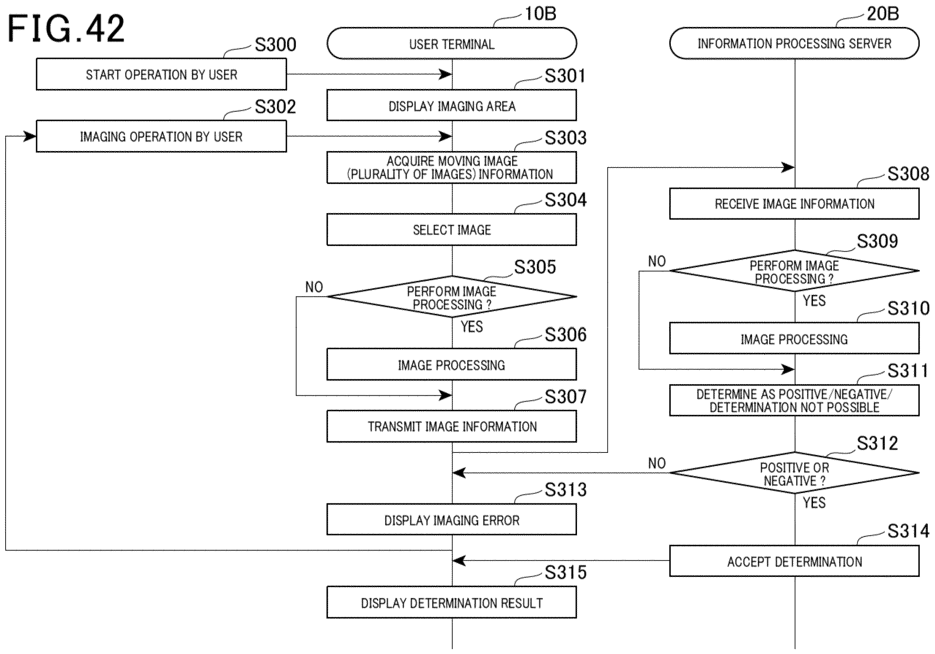

FIG. 42 is a sequence diagram illustrating a flow of processing performed by the information processing system 1B according to the second modification of the embodiment. The present modification differs from the embodiment described above in that a plurality of images (e.g., a moving image) are captured from different imaging positions, and the image that is selected from the plurality of images is transmitted to the information processing server 20B.

Since step S300 illustrated in FIG. 42 is similar to step S100 illustrated in FIG. 40, description thereof which is omitted. Since steps S305 to S315 illustrated in FIG. 42 are similar to steps S104 to S114 illustrated in FIG. 40, description thereof is omitted. That is, steps S301 to S302, S303 to S304 will now be described.

The user terminal 10B starts the telemedicine application in response to a start operation by a user and displays an imaging screen for capturing a moving image of the test strip 100B (step S301). Herein, the user terminal 10B may display a message such as "please capture moving image while changing imaging position". The user visually recognizes the imaging screen displayed on the user terminal 10B, and performs an operation for capturing a plurality of images, for example, while moving the user terminal 10B in the oblique direction from a position above test strip 100B (step S302). The user terminal 10B captures an image with the imaging unit 16B in response to the imaging operation for a moving image by the user to acquire image information related to the plurality of images (step S303). The user terminal 10B selects one image from the acquired plurality of images as the test image GB (step S304). The user terminal 10B selects an image by which determination can be made with high accuracy, for example, depending on states of the coloring areas whose images are included in the respective images. The user terminal 10B selects, as the test image GB, for example, an image which includes an image of the whole coloring area and in which the image of the coloring area is included with an appropriate color. For example, the user terminal 10B compares color information on the background (portion that is not colored) of the coloring parts 103B of the respective plurality of images with color information that is a predetermined criterion and selects, as the test image GB, an image having the smallest difference from the color information that is the criterion. The color information that is the criterion herein is, for example, color information of the background whose image is captured in a state in which an average light source is used whose spectral irradiance is known.

### (Third modification of embodiment)

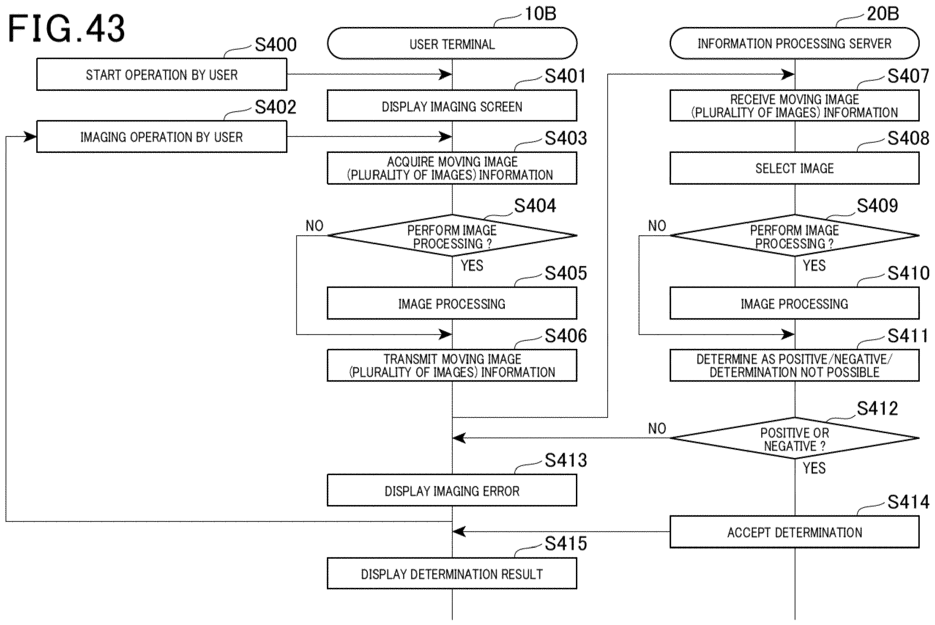

FIG. 43 is a sequence diagram illustrating a flow of processing performed by the information processing system 1B according to the third modification of the embodiment. The present modification differs from the embodiment described above in that a plurality of images (e.g., a moving image) are captured from different imaging positions, each of the plurality of images is transmitted to the information processing server 20B, and the image used for determination is selected in the information processing server 20B.

Since steps S400 to S403 illustrated in FIG. 43 are similar to steps S300 to S303 illustrated in FIG. 42, description thereof is omitted. Since steps S404 to S405, S409 to S415 illustrated in FIG. 43 are similar to steps S305 to S306, S309 to S315 illustrated in FIG. 42, description thereof is omitted. That is, steps S406 to S408 will now be described.

The user terminal 10B transmits each of the plurality of images captured in step S403 to the information processing server 20B (step S406). The information processing server 20B receives the plurality of images from the user terminal 10B (step S407) and selects one image from among the received plurality of images as the test image GB (step S408). Herein, since the method of selecting one image by the information processing server 20B is similar to the method performed in step S304 by the user terminal 10B, description thereof is omitted.

### (Fourth modification of embodiment)

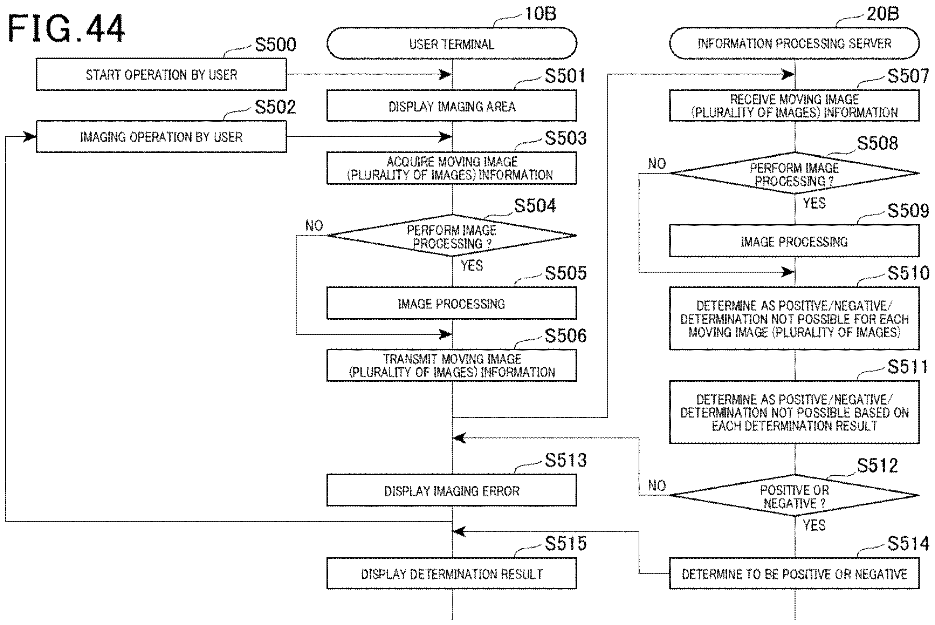

FIG. 44 is a sequence diagram illustrating a flow of processing performed by the information processing system 1B according to the fourth modification of the embodiment. The present modification differs from the embodiment described above in that a plurality of images (e.g., a moving image) are captured from different imaging positions, and determination is made for each of the plurality of images.

Since steps S500 to S507 illustrated in FIG. 44 are similar to steps S400 to S407 illustrated in FIG. 43, description thereof is omitted. Since steps S512 to S515 illustrated in FIG. 44 are similar to steps S412 to S415 illustrated in FIG. 43, description thereof is omitted. That is, steps S508 to S511 will now be described.

The information processing server 20B determines whether to perform image processing of each of the plurality of images received from the user terminal 10B (step S508). If determining to perform image processing, the information processing server 20B performs required image processing (step S509) and determines whether the image that has undergone image processing indicates positive, negative, or determination not possible (step S510).

In contrast, if determining not to perform image processing, the information processing server 20B determines whether positive, negative, or determination not possible is indicated based on the image that has undergone the image processing and is not changed. The information processing server 20B determines whether the subject (test strip 100B) indicates positive, negative, or determination not possible based on determination results of the respective plurality of images. For example, the information processing server 20B determines the largest number of determination results among the determination results of the respective plurality of images as the determination result of the subject. For example, if 13 of 15 images are determined to be positive, the information processing server 20B determines that the subject indicates positive.

Alternatively, if the ratio of the largest number of determination results with respect to the number of the determination results of the respective plurality of images is less than a threshold value, the information processing server 20B may determine the determination result of that subject as "determination not possible". In this case, for example, if the threshold value is set to 1/2, and 7 of 15 images are determined as positive, 5 of 15 images are determined as determination not possible, and 3 of 15 images are determined as negative, the information processing server 20B determines that the ratio of the number of images that have determined as positive with respect to the number of all the images (7/15) is less than the threshold value (1/2), and determines the determination result of the subject as "determination not possible".

As described above, the information processing system 1B of the embodiment is a system that processes information concerning the test using a test piece that develops a color depending on whether the specimen includes the test object.

The information processing system 1B includes, for example, the imaging unit 16B and the determination unit 232B. The imaging unit 16B captures an image of the test strip 100B, which is a subject, so as to include the coloring part 103B (colored coloring area) of the test strip 100B. The determination unit 232B determines whether the coloring part 103B indicates positive based on the image captured by the imaging unit 16B. The determination model is a learned model that has learned a correspondence relationship between an image and positive by performing machine learning using a training data set in which the training image captured so as to include the coloring part 103B of the unspecified test strip 100B is appended with a sign representing whether the coloring part 103B in the training image indicates positive. The determination model estimates the degree of positive indication in the input images based on a previously learned correspondence relationship and outputs the estimated result. The training image learned by the determination model includes a plurality of images of the same subject captured in different imaging environments. Hence, in the information processing system 1B of the embodiment, when the test images GB captured in various imaging environments are input, the degree of positive indication in the images can be determined with high accuracy.

Herein, the test strip 100B is an example of a "test piece". The coloring part 103B is an example of a "coloring area".

In addition, in the information processing system 1B of the embodiment, the training image that the determination model has learned includes a plurality of images including the test objects having different concentrations and supplied to the test strip 100B. Hence, in the information processing system 1B of the embodiment, even when it is difficult to determine whether it is positive with the naked eye, the determination mode can make a determination with high accuracy.

In addition, in the information processing system 1B of the embodiment, the image processing unit (at least one of the image processing unit 131B and the image processing unit 231B) may be configured to perform processing for converting information indicating a color of the image captured by imaging unit 16B from information using a first color space to information using a second color space different from the first color space. In addition, the image processing unit may be configured to perform processing for converting a file format of an image captured by the imaging unit 16B. In addition, in the information processing system 1B of the embodiment, the image processing unit (at least one of the image processing unit 131B and the image processing unit 231B) may be configured to perform image processing for trimming the coloring part 103B in the image captured by the imaging unit 16B. Hence, in the information processing system 1B of the embodiment, when the image learned by the determination model and the test image GB are different from each other, the contents can approach the contents learned by the determination model, whereby whether it is positive can be determined with high accuracy.

In addition, in the information processing system 1B of the embodiment, the determination unit 232B determines whether the image indicates positive, negative, or neither positive nor negative, that is, determination not possible. If determination is not possible, the determination unit 232B causes the imaging unit 16B to capture an image of the subject again. Hence, in the information processing system 1B of the embodiment, if determination is not possible, imaging can be performed again.

In addition, in the information processing system 1B of the embodiment, the determination model may output information indicating whether the input image indicates a positive result. Hence, the result output from the determination model can be easily grasped by a general user.

In addition, in the information processing system 1B of the embodiment, the training data set may be configured so that the training image is appended with a sign indicating whether it is positive and a concentration of the test object present in a subject in the training image. In this case, the determination model is a learned model that has learned a correspondence relationship between the image and the concentration. The determination model estimates the concentration of the test object present in the subject in the input image based on the learned correspondence relationship. That is, the determination model estimates a concentration of the test object present in the subject supplied to the test strip 100B in the input image. Hence, in the information processing system 1B of the embodiment, the concentration of the test object present in the subject can be estimated. It is noted that the input image includes a color correction indicator or an angle correction indicator.

In addition, in the information processing system 1B of the embodiment, the training image includes a plurality of images captured by emitting illumination lights having different spectral radiation characteristics. Hence, in the information processing system 1B of the embodiment, when the test images GB captured in environments in which various illumination lights are emitted are input, the degree of positive indication in the images can be determined with high accuracy.

In addition, in the information processing system 1B, before determining whether it is positive, a prior confirmation may be performed which determines whether determination is not possible depending on the imaging environment in which the image is captured. For example, the control unit 13B determines whether the image captured by the imaging unit 16B is difficult to use for determining whether it is positive. The control unit 13B determines, for example, an image not including an image of the coloring part 103B, an image in which the coloring part 103B is out of focus, or the like as an image difficult to use for determining whether it is positive. If the image captured by the imaging unit 16B is an image difficult to use for determining whether it is positive, the control unit 13B displays a message prompting capture of the image again.
Alternatively, the information processing server 20B may be configured to perform a prior confirmation. In this case, the user terminal 10B transmits an image captured by the imaging unit 16B to the information processing server 20B. The user terminal 10B performs a prior confirmation concerning the image received from the user terminal 10B. If the image captured by the imaging unit 16B is an image difficult to use for determining whether it is positive, the user terminal 10B transmits a notification prompting capture of the image again to the user terminal 10B.

When the user captures an image of the test strip 100B with a smartphone, the control unit 13B may prompt the user to capture an image again according to the state of the captured image (e.g., in a case in which the captured image includes a shadow, or the like). Hence, the control unit 13B can determine, from a finger or the background in the image captured by the user, whether the image is an image that has already been captured in the past and transmitted to the information processing server. If the image is an image that has been transmitted in the past, the control unit 13B can prompt capture of the image again.

In addition, the determination model may be caused to learn with images captured by a plurality of smartphones produced by different device manufacturers or include OSs of different versions serving as user terminals (e.g., smartphones) and input the captured image to the determination model to determine whether positive is indicated by using the determination model.

In addition, in the information processing system 1B according to the first modification of the embodiment, the imaging control unit 130B displays the imaging area whose image is captured by the imaging unit 16B. The imaging control unit 130B specifies the coloring part 103B included in the imaging area and converts a color of areas different from the specified coloring part 103B to a single color. The imaging unit 16B captures an image that has undergone image processing by the imaging control unit 130B. Hence, in the information processing system 1B according to the first modification of the embodiment, the color of areas different from the coloring area is simplified to avoid the color of the coloring area from being influenced by the color of areas different from the coloring area.

In addition, in the information processing system 1B according to the second modification of the embodiment, the imaging unit 16B captures images of the same subject from respective different imaging positions. The image processing unit 131B selects one image based on the respective colors of the plurality of images captured by the imaging unit 16B from different imaging positions. Hence, in the information processing system 1B according to the second modification of the embodiment, the image used for determination can be selected from the plurality of images. Hence, even in a case in which an image is captured in a state in which the color of coloring part 103B is separated from a standard color depending on a relative positional relationship between the imaging position and the installation position of illumination light, an image that is slightly separated from the standard color can be selected. Hence, whether it is positive can be determined with high accuracy.

In addition, in the information processing system 1B according to the fourth modification of the embodiment, the imaging unit 16B captures images of the same subject from respective different imaging positions. The determination unit 232B determines whether the respective plurality of images captured by the imaging unit 16B from the different imaging positions indicate positive. The determination unit 232B determines whether positive is indicated based on the respective determination results of the plurality of images. Hence, in the information processing system 1B according to the fourth modification of the embodiment, it can be determined whether positive is comprehensively indicated by using the respective determination results of the plurality of images.

All or part of the information processing system 1B and the information processing server 20B in the embodiment described above may be implemented by a computer. This case may be implemented by storing a program for implementing the functionality in a computer readable storage medium and causing a computer system to read the program stored in the storage medium to execute the program. The "computer system" herein includes an OS and hardware such as peripheral devices. In addition, the "computer readable storage medium" refers to a storage medium such as a portable medium, such as a flexible disk, a magneto-optical disk, a ROM, and a CD-ROM, and a hard disk and the like installed in the computer system. Furthermore, the "computer readable storage medium" may include a medium that dynamically holds a program for a short time, such as a communication line that transmits a program via a network such as the Internet or a communication line such as a telephone line, and a medium that holds a program for a certain time, such as a volatile memory inside a computer system serving as a server or a client in that case. In addition, the program may be a program for implementing part of the function described above, furthermore, a program that can implement the function described above by the combination with the program previously stored in the computer system, or a program that is implemented by using a programmable logic device such as an FPGA.

In the above, an embodiment of the present invention is described in detail with reference to the drawings. However, specific configurations are not limited to the embodiment and include design and the like within a range that does not deviate from the gist of the present invention.

It is noted that the present invention also includes the following inventions.

### (Invention 1)

An information processing method that is performed by an information processing system including a test piece, a first device, and a second device communicably connected with the first device and processes information concerning a test using the test piece, the method including:
a coloring step of applying individual identification information for identifying an individual of the test piece to the test piece, and supplying a biological sample, which is collected from a tested person, to the test piece, to develop a color depending on whether the biological sample includes a test object;
an imaging step of capturing, by the first device, a digital image including at least an area colored with the color of the test piece in a state of being colored with the color;
   a first communication step of transmitting, by the first device, the digital image and the individual identification information to the second device communicably connected with the first device;
a determination step of determining, by the second device, whether color information indicating the color in the digital image has the test object, based on the digital image received from the first device and determination criterion information for determining presence or absence of the test object; and
a storage control step of storing, by the second device, the digital image received from the first device in a state of being associated with the individual identification information.

### (Invention 2)

The information processing method according to invention 1, further including an acquisition step of acquiring, by the first device, tested person information that is information concerning the tested person, the tested person information including at least one piece of information selected from a group including information uniquely indicating the tested person, information indicating an attribute of the tested person, and information indicating a location at which the test for the tested person has been performed,
in the first communication step, the tested person information is transmitted to the second device in a state of being associated with the digital image, and
in the storage control step, the tested person information is stored in a state of being associated with the individual identification information.

### (Invention 3)

The information processing method according to claim 1 or 2, further including:
a second communication step of transmitting, by the second device, a determination result of a determination made in the determination step to the first device via a communication network; and
a display step of displaying the determination result received from the second device.

### (Invention 4)

The information processing method according to any of inventions 1 to 3, including a second communication step of associating, by the second device, information used for the determination in the determination step and the determination result of the determination made in the determination step with the individual identification information and transmitting them to a third device.

### (Invention 5)

The information processing method according to invention 4, further including a third communication step of associating, by the third device, a verification result of the determination result of a verification by a verifying person who verifies the determination result based on information received from the second device with the individual identification information and transmitting them to the second device.

### (Invention 6)

The information processing method according to any of inventions 1 to 5, wherein,
in the storage control step, the determination result of the determination in the determination step is stored in a state of not being associated with tested person information that is information concerning the tested person but being associated with the individual identification information, and the tested person information is stored in a state of not being associated with the determination result but being associated with the individual identification information.

### (Invention 7)

The information processing method according to any of inventions 1 to 6, wherein
the individual identification information is information embedded in a code provided to the test piece,
in the imaging step, the digital image is captured so as to include an area, to which the code is provided, of the test piece, and
in the first communication step, the digital image including an area indicating the code is transmitted to transmit the individual identification information to the second device.

### (Invention 8)

The information processing method according to invention 7, wherein
the information embedded in the code includes, as test piece description information describing the test piece, information indicating at least one selected from a group including a type of the test object, a type of disease indicated by the test object, and a manufacturer of the test piece.

### (Invention 9)

The information processing method according to invention 8, wherein
in the imaging step, the digital image is captured so that the individual identification information and the test piece description information are included in one image.

### (Invention 10)

The information processing method according to any of inventions 1 to 9, wherein
the test piece is a lateral flow test strip.

### (Invention 11)

The information processing method according to any of inventions 1 to 10, wherein
in the imaging step, when an image of the test piece is captured, a guide representing an outline of the test piece is displayed on a display unit of the first device.

### (Invention 12)

The information processing method according to any of inventions 1 to claim 11, wherein
a color correction indicator is prepared,
in the imaging step, the digital image is captured so as to include an area, to which the color correction indicator is provided, of the test piece, and
the method further includes a correction step of using, by the second device, the color correction indicator indicated in the digital image received from the first device to perform color correction of the digital image.

### (Invention 13)

The information processing method according to any of inventions 1 to claim 12, wherein
an angle correction indicator is prepared,
in the imaging step, the digital image is captured so as to include an area, to which the angle correction indicator is provided, of the test piece, and
the method further includes a correction step of using, by the second device, the angle correction indicator indicated in the digital image received from the first device to perform angle correction of the digital image.

### (Invention 14)

The information processing method according to invention 12 or claim 13, wherein
the individual identification information is information embedded in a code provided to the test piece,
in the imaging step, the digital image is captured so as to include an area, to which the code is provided, of the test piece, and
in the correction step, the code indicated in the digital image received from the first device is used to perform at least one of color correction and angle correction of the digital image.

### (Invention 15)

An information processing system that includes a test piece, a first device, and a second device communicably connected with the first device and processes information concerning a test using the test piece, wherein
the test piece has a coloring unit that, when a biological sample collected from a tested person is supplied to the test piece, develops a color depending on whether the biological sample includes a test object,
the test piece is applied with individual identification information for identifying an individual of the test piece,
the first device has:
an imaging unit that captures a digital image including at least an area colored with the color of the test piece in a state of being colored with the color; and
a first communication unit that transmits the digital image and the individual identification information to an information processing server, and
the second device has:
   a determination unit that determines whether color information indicating the color in the digital image has the test object, based on the digital image received from the first device and determination criterion information for determining presence or absence of the test object; and
   a storage control unit that causes the digital image received from the first device to be stored in a stated of being associated with the individual identification information.

### (Invention 16)

An information processing system that includes a test terminal, a user terminal, and an information processing server and processes information concerning a test using a test piece for testing whether a biological sample includes a test object, wherein
the test terminal has:
a first acquisition unit that acquires individual identification information for identifying an individual of the test piece used for a test of a tested person and test information indicating a test result of the tested person; and
a registration unit that transmits the individual identification information and the test information acquired by the first acquisition unit to the information processing server,
the user terminal has:
   a second acquisition unit that acquires the individual identification information of the test piece used for the test of the tested person and user information concerning the tested person; and
   a user registration unit that transmits the individual identification information and the user information acquired by the second acquisition unit to the information processing server, and
   the information processing server has a registration control unit that causes a test result database to store information in which the test information is associated with the individual identification information based on the information received from the test terminal, and stores information in which the user information is associated with the individual identification information in a user database based on the information received from the user terminal.

### (Invention 17)

The information processing system according to claim 16, wherein
the test piece has a coloring unit that indicates a color depending on whether a biological sample includes a test object when the biological sample, which is collected from a tested person, is supplied to the test piece,
the test terminal further has a test determination unit that determines whether the biological sample includes the test object based on the color indicated on the coloring unit after the test to which the biological sample, which is collected from the tested person, is supplied, and
the first acquisition unit acquires a test result of the test determination unit as the test information indicating the test result.

### (Invention 18)

The information processing system according to invention 16 or 17, wherein
the user terminal further has a test result request unit that transmits the individual identification information to the information processing server to request the determination result of the test using the test piece corresponding to the individual identification information from the information processing server,
the information processing server further has:
   a test result extraction unit that extracts the test result associated with the individual identification information by referring to the test result database based on the individual identification information provided from the user terminal; and
   a device control unit that transmits the test result extracted by the test result extraction unit to the user terminal.

### (Invention 19)

The information processing system according to invention 18, wherein
the test result request unit transmits, together with the individual identification information, the user information to the information processing server to request the test result from the information processing server.

### (Invention 20)

The information processing system according to invention 19, wherein
the information processing server further has a notification determination unit that determines whether to provide the test result to the user terminal based on the user information provided from the user terminal and information stored in the user database, and
if the notification determination unit determines to provide the test result to the user terminal, the test result extraction unit extracts the test result.

### (Invention 21)

The information processing system according to invention 19 or 20, further including an event site terminal having:
a third acquisition unit that acquires the test result associated with the individual identification information from the user terminal; and
a condition judgment unit that determines whether the test result acquired by the third acquisition unit satisfies an entrance condition.

### (Invention 22)

The information processing system according to invention 21, wherein
the device control unit transmits a bar code or a two-dimensional code, in which the test result extracted by the test result extraction unit is embedded, to the user terminal, and
the third acquisition unit reads the bar code or the two-dimensional code to acquire the test result associated the individual identification information from the user terminal.

### (Invention 23)

The information processing system according to invention 21, wherein
the device control unit transmits a password, which is used when the test result extracted by the test result extraction unit is displayed, to the user terminal,
the user terminal transmits the individual identification information and the password to the event site terminal, and
the third acquisition unit transmits the individual identification information and the password to the information processing server to acquire the test result associated with the individual identification information.

### (Invention 24)

An information processing method that is an information processing system that includes a test terminal, a user terminal, and an information processing server and processes information concerning a test using a test piece for testing whether a biological sample includes a test object, wherein
the test terminal
acquires individual identification information for identifying an individual of the test piece used for a test of a tested person and test information indicating a test result of the tested person, and
transmits the acquired individual identification information and test information to the information processing server,
the user terminal
acquires the individual identification information of the test piece used for the test of the tested person and user information concerning the tested person, and
transmits the acquired individual identification information and user information to the information processing server, and
the information processing server causes a test result database to store information in which the test information is associated with the individual identification information based on the information received from the test terminal, and stores information in which the user information is associated with the individual identification information in a user database based on the information received from the user terminal.

### (Invention 25)

An information processing method that is performed by an information processing system that processes information concerning a test using a test piece that develops a color depending on whether a specimen includes a test object, the method including:
capturing, by an imaging unit, an image of the test piece that is a subject so as to include a coloring area, which is colored with the color, of the test piece; and
determining, by a determination unit, whether the image captured by the imaging unit indicates a positive result indicating that the coloring area has the test object, by using a determination model, wherein
the determination model is a learned model that learns a correspondence relationship between an image and the positive by performing machine learning using a training data set, which is obtained by appending a sign, which indicates whether the coloring area in a training image, which has been captured so as to include the coloring area in the unspecified test piece, indicates the positive, to the training image, and that estimates a degree of positive indication in an input image based on the correspondence relationship, to output a result of the estimation, and
the training image includes a plurality of images of the same subject captured in different imaging environments.

### (Invention 26)

The information processing method according to invention 25, wherein
the determination model outputs information indicating whether the input image indicates the positive.

### (Invention 27)

The information processing method according to invention 25 or claim 26, wherein
in the training data set, the training image is applied with the sign and a concentration of the test object included in the specimen in the training image, and
the determination model estimates the concentration of the test object included in the specimen in the input image and outputs a result of the estimation.

### (Invention 28)

The information processing method according to any of inventions 25 to 27, wherein
the training image includes a plurality of images in which concentrations of the test object supplied to the test piece are different.

### (Invention 29)

The information processing method according to any of inventions 25 to 28, wherein
the training image includes a plurality of images captured in different imaging environments in which illumination lights having different spectral radiation characteristics are emitted.

### (Invention 30)

The information processing method according to any of inventions 25 to claim 29, wherein
an image processing unit performs image processing that is at least one of processing for converting information on a color of an image captured by the imaging unit from information using a first color space to information using a second color space different from the first color space and processing for converting a file format of the image captured by the imaging unit, and
the determination unit makes a determination by using an image that has been subjected to the image processing by the image processing unit.

### (Invention 31)

The information processing method according to any of inventions 25 to 30, wherein
the image processing unit performs image processing for trimming the coloring part in the image captured by the imaging unit, and
the determination unit makes a determination by using an image that has been subjected to the image processing by the image processing unit.

### (Invention 32)

The information processing method according to any of inventions 25 to 31, wherein
the imaging unit captures images of the same subject from respective different image positions, and
an image processing unit selects one image based on respective colors of the plurality of images captured by the imaging unit from the different imaging positions, and
the determination unit makes a determination by using the image selected by the image processing unit.

### (Invention 33)

The information processing method according to any of inventions 25 to 31, wherein
the imaging unit captures images of the same subject from respective different image positions, and
the determination unit makes a determination for each of the plurality of images captured by the imaging unit from the different image positions and determines whether the positive is indicated based on respective determination results of the plurality of images.

### (Invention 34)

The information processing method according to any of inventions 25 to claim 33, wherein
an imaging control unit displays an imaging area whose image is captured by the imaging unit, specifies the coloring area included in the imaging area, and converts a color of an area different from the specified coloring part to a single color,
the imaging unit captures an image that has undergone image processing by the imaging control unit, and
the determination unit makes a determination using the image captured by the imaging unit.

### (Invention 35)

The information processing method according to inventions 25 to 34, wherein
the determination unit determines, based on the degree of positive indication obtained by inputting an image to the determination model, whether the image indicates the positive, indicates negative indicating that the coloring area does not have the test object, or indicates neither the positive nor the negative, which indicates that determination is not possible, and causes the imaging unit to capture an image of the subject again if it is determined that determination is not possible.

### (Invention 36)

An information processing system that processes information concerning a test using a test piece that develops a color depending on whether a specimen includes a test object, the system including:
an imaging unit that captures an image of the test piece that is a subject so as to include a coloring area, which is colored with the color, of the test piece; and
a determination unit that determines whether the image captured by the imaging unit indicates a positive result indicating that the coloring area has the test object, by using a determination model, wherein
the determination model is a learned model that learns a correspondence relationship between an image and the positive by performing machine learning using a training data set, which is obtained by appending a sign, which indicates whether the coloring area in a training image, which has been captured so as to include the coloring area in the unspecified test piece, indicates the positive, to the training image, and that estimates a degree of positive indication in an input image based on the correspondence relationship, to output a result of the estimation, and
the training image includes a plurality of images of the same subject captured in different imaging environments.

### (Invention 37)

The information processing system according to invention 36, including:
a user terminal that has the imaging unit; and
an information processing server that has the determination unit, wherein
the user terminal transmits an image captured by the imaging unit to the information processing server.

### [Industrial Applicability]

According to the present invention, a test using a manner having high reliability can be performed by an aspect that is difficult to leak information having high confidentiality.

Furthermore, according to the present invention, safety of data can be increased when a test result is utilized. Specifically, a tested person who has undergone a test and the test result thereof are stored in separate databases. Hence, the test result and a person cannot be easily associated with each other, whereby privacy of the tested person can be protected.

Furthermore, according to the present invention, when a determination is made using an image captured by a user, images captured in various imaging environments can be determined with high accuracy.

### [Reference Signs List]

- 1, 1A, 1B: Information processing system
- 10: User terminal (first device)
- 10A: Test termina
- 10B: User terminal
- 11, 11A, 11B: Communication unit (first communication unit)
- 12, 12A, 12B: Storage unit
- 13, 13A, 13B: Control unit
- 130: Imaging control unit
- 130A: Acquisition unit (first acquisition unit)
- 130B: Imaging control unit
- 131A: Test determination unit
- 131B: Image processing unit
- 132A: Registration unit
- 14, 14A, 14B: Display unit
- 15, 15A, 15B: Input unit
- 16, 16A, 16B: Imaging unit
- 20, 20A, 20B: Information processing server (second device)
- 21, 21A, 21B: Communication unit (second communication unit)
- 22, 22A, 22B: Storage unit
- 220: Individual information
- 220B: Determination model information
- 221: Tested person information
- 221B: Test result information
- 222: Test result information
- 23: Control unit
- 23B: Control unit
- 230: Acquisition unit
- 230A: Acquisition unit
- 230B: Acquisition unit
- 231: Correction unit
- 231A: Registration control unit
- 231B: Image processing unit
- 232: Determination unit
- 232A: Notification determination unit
- 232B: Determination unit
- 233: Storage control unit
- 233A: Test result extraction unit
- 233B: Device control unit
- 234A: Device control unit
- 30: Institution server (third device)
- 30A: User terminal
- 31A: Communication unit
- 32A: Storage unit
- 33A: Control unit
- 330A: Acquisition unit (second acquisition unit)
- 331A: User registration unit
- 332A: Test result request unit
- 333A: Device control unit
- 34A: Display unit
- 35A: Input unit
- 40A: Event site terminal
- 41A: Communication unit
- 42A: Storage unit
- 420A: Entry condition information section
- 43A: Control unit
- 430A: Acquisition unit (third acquisition unit)
- 431A: Condition judgment unit
- 432A: Device control unit
- 44A: Display unit
- 45A: Input unit
- 46A: Imaging unit

## Claims

1. An information processing method that is performed by an information processing system including a test piece, a first device, and a second device communicably connected with the first device and processes information concerning a test using the test piece, the method comprising:
a coloring step of applying individual identification information for identifying an individual of the test piece to the test piece, and supplying a biological sample, which is collected from a tested person, to the test piece, to develop a color depending on whether the biological sample includes a test object;
an imaging step of capturing, by the first device, a digital image including at least an area colored with the color of the test piece in a state of being colored with the color;
a first communication step of transmitting, by the first device, the digital image and the individual identification information to the second device communicably connected with the first device;
a determination step of determining, by the second device, whether color information indicating the color in the digital image has the test object, based on the digital image received from the first device and determination criterion information for determining presence or absence of the test object; and
a storage control step of storing, by the second device, the digital image received from the first device in a state of being associated with the individual identification information.

2. The information processing method according to claim 1, further comprising an acquisition step of acquiring, by the first device, tested person information that is information concerning the tested person, the tested person information including at least one piece of information selected from a group including information uniquely indicating the tested person, information indicating an attribute of the tested person, and information indicating a location at which the test for the tested person has been performed,
in the first communication step, the tested person information is transmitted to the second device in a state of being associated with the digital image, and
in the storage control step, the tested person information is stored in a state of being associated with the individual identification information.

3. The information processing method according to claim 1 or claim 2, further comprising:
a second communication step of transmitting, by the second device, a determination result of a determination made in the determination step to the first device via a communication network; and
a display step of displaying the determination result received from the second device.

4. The information processing method according to any one of claim 1 to claim 3, further comprising a second communication step of associating, by the second device, information used for the determination in the determination step and the determination result of the determination made in the determination step with the individual identification information and transmitting them to a third device.

5. The information processing method according to claim 4, further comprising a third communication step of associating, by the third device, a verification result of the determination result of a verification by a verifying person who verifies the determination result based on information received from the second device with the individual identification information and transmitting them to the second device.

6. The information processing method according to any one of claim 1 to claim5, wherein
in the storage control step, the determination result of the determination in the determination step is stored in a state of not being associated with tested person information that is information concerning the tested person but being associated with the individual identification information, and the tested person information is stored in a state of not being associated with the determination result but being associated with the individual identification information.

7. The information processing method according to any one of claim 1 to claim 6, wherein
the individual identification information is information embedded in a code provided to the test piece,
in the imaging step, the digital image is captured so as to include an area, to which the code is provided, of the test piece, and
in the first communication step, the digital image including an area indicating the code is transmitted to transmit the individual identification information to the second device.

8. The information processing method according to claim 7, wherein
the information embedded in the code includes, as test piece description information describing the test piece, information indicating at least one selected from a group including a type of the test object, a type of disease indicated by the test object, and a manufacturer of the test piece.

9. The information processing method according to claim 8, wherein
in the imaging step, the digital image is captured so that the individual identification information and the test piece description information are included in one image.

10. The information processing method according to any one of claim 1 to claim 9, wherein
the test piece is a lateral flow test strip.

11. The information processing method according to any one of claim 1 to claim 10, wherein
in the imaging step, when an image of the test piece is captured, a guide representing an outline of the test piece is displayed on a display unit of the first device.

12. The information processing method according to any one of claim 1 to claim 11, wherein
a color correction indicator is prepared,
in the imaging step, the digital image is captured so as to include an area, to which the color correction indicator is provided, of the test piece, and
the method further comprises a correction step of using, by the second device, the color correction indicator indicated in the digital image received from the first device to perform color correction of the digital image.

13. The information processing method according to any one of claim 1 to claim 12, wherein
an angle correction indicator is prepared,
in the imaging step, the digital image is captured so as to include an area, to which the angle correction indicator is provided, of the test piece, and
the method further comprises a correction step of using, by the second device, the angle correction indicator indicated in the digital image received from the first device to perform angle correction of the digital image.

14. The information processing method according to claim 12 or claim 13, wherein
the individual identification information is information embedded in a code provided to the test piece,
in the imaging step, the digital image is captured so as to include an area, to which the code is provided, of the test piece, and
in the correction step, the code indicated in the digital image received from the first device is used to perform at least one of color correction and angle correction of the digital image.

15. An information processing system that includes a test piece, a first device, and a second device communicably connected with the first device and processes information concerning a test using the test piece, wherein
the test piece has a coloring unit that, when a biological sample collected from a tested person is supplied to the test piece, develops a color depending on whether the biological sample includes a test object,
the test piece is applied with individual identification information for identifying an individual of the test piece,
the first device has:
an imaging unit that captures a digital image including at least an area colored with the color of the test piece in a state of being colored with the color; and
a first communication unit that transmits the digital image and the individual identification information to an information processing server, and
the second device has:
a determination unit that determines whether color information indicating the color in the digital image has the test object, based on the digital image received from the first device and determination criterion information for determining presence or absence of the test object; and
a storage control unit that causes the digital image received from the first device to be stored in a stated of being associated with the individual identification information.

16. An information processing system that includes a test terminal, a user terminal, and an information processing server and processes information concerning a test using a test piece for testing whether a biological sample includes a test object, wherein
the test terminal has:
a first acquisition unit that acquires individual identification information for identifying an individual of the test piece used for a test of a tested person and test information indicating a test result of the tested person; and
a registration unit that transmits the individual identification information and the test information acquired by the first acquisition unit to the information processing server,
the user terminal has:
a second acquisition unit that acquires the individual identification information of the test piece used for the test of the tested person and user information concerning the tested person; and
a user registration unit that transmits the individual identification information and the user information acquired by the second acquisition unit to the information processing server, and
the information processing server has a registration control unit that causes a test result database to store information in which the test information is associated with the individual identification information based on the information received from the test terminal, and stores information in which the user information is associated with the individual identification information in a user database based on the information received from the user terminal.

17. The information processing system according to claim 16, wherein
the test piece has a coloring unit that indicates a color depending on whether a biological sample includes a test object when the biological sample, which is collected from a tested person, is supplied to the test piece,
the test terminal further has a test determination unit that determines whether the biological sample includes the test object based on the color indicated on the coloring unit after the test to which the biological sample, which is collected from the tested person, is supplied, and
the first acquisition unit acquires a test result of the test determination unit as the test information indicating the test result.

18. The information processing system according to claim 16 or claim 17, wherein
the user terminal further has a test result request unit that transmits the individual identification information to the information processing server to request the determination result of the test using the test piece corresponding to the individual identification information from the information processing server,
the information processing server further has:
a test result extraction unit that extracts the test result associated with the individual identification information by referring to the test result database based on the individual identification information provided from the user terminal; and
a device control unit that transmits the test result extracted by the test result extraction unit to the user terminal.

19. The information processing system according to claim 18, wherein
the test result request unit transmits, together with the individual identification information, the user information to the information processing server to request the test result from the information processing server.

20. The information processing system according to claim 19, wherein
the information processing server further has a notification determination unit that determines whether to provide the test result to the user terminal based on the user information provided from the user terminal and information stored in the user database, and
if the notification determination unit determines to provide the test result to the user terminal, the test result extraction unit extracts the test result.

21. The information processing system according to claim 19 or claim 20, further comprising an event site terminal having:
a third acquisition unit that acquires the test result associated with the individual identification information from the user terminal; and
a condition judgment unit that determines whether the test result acquired by the third acquisition unit satisfies an entrance condition.

22. The information processing system according to claim 21, wherein
the device control unit transmits a bar code or a two-dimensional code, in which the test result extracted by the test result extraction unit is embedded, to the user terminal, and
the third acquisition unit reads the bar code or the two-dimensional code to acquire the test result associated the individual identification information from the user terminal.

23. The information processing system according to claim 21, wherein
the device control unit transmits a password, which is used when the test result extracted by the test result extraction unit is displayed, to the user terminal,
the user terminal transmits the individual identification information and the password to the event site terminal, and
the third acquisition unit transmits the individual identification information and the password to the information processing server to acquire the test result associated with the individual identification information.

24. An information processing method that is an information processing system that includes a test terminal, a user terminal, and an information processing server and processes information concerning a test using a test piece for testing whether a biological sample includes a test object, wherein
the test terminal
acquires individual identification information for identifying an individual of the test piece used for a test of a tested person and test information indicating a test result of the tested person, and
transmits the acquired individual identification information and test information to the information processing server,
the user terminal
acquires the individual identification information of the test piece used for the test of the tested person and user information concerning the tested person, and
transmits the acquired individual identification information and user information to the information processing server, and
the information processing server causes a test result database to store information in which the test information is associated with the individual identification information based on the information received from the test terminal, and stores information in which the user information is associated with the individual identification information in a user database based on the information received from the user terminal.

25. An information processing method that is performed by an information processing system that processes information concerning a test using a test piece that develops a color depending on whether a specimen includes a test object, the method comprising:
capturing, by an imaging unit, an image of the test piece that is a subject so as to include a coloring area, which is colored with the color, of the test piece; and
determining, by a determination unit, whether the image captured by the imaging unit indicates a positive result indicating that the coloring area has the test object, by using a determination model, wherein
the determination model is a learned model that learns a correspondence relationship between an image and the positive by performing machine learning using a training data set, which is obtained by appending a sign, which indicates whether the coloring area in a training image, which has been captured so as to include the coloring area in the unspecified test piece, indicates the positive, to the training image, and that estimates a degree of positive indication in an input image based on the correspondence relationship, to output a result of the estimation, and
the training image includes a plurality of images of the same subject captured in different imaging environments.

26. The information processing method according to claim 25, wherein
the determination model outputs information indicating whether the input image indicates the positive.

27. The information processing method according to claim 25 or claim 26, wherein
in the training data set, the training image is applied with the sign and a concentration of the test object included in the specimen in the training image, and
the determination model estimates the concentration of the test object included in the specimen in the input image and outputs a result of the estimation.

28. The information processing method according to any one of claim 25 to claim 27, wherein
the training image includes a plurality of images in which concentrations of the test object supplied to the test piece are different.

29. The information processing method according to any one of claim 25 to claim 28, wherein
the training image includes a plurality of images captured in different imaging environments in which illumination lights having different spectral radiation characteristics are emitted.

30. The information processing method according to any one of claim 25 to claim 29, wherein
an image processing unit performs image processing that is at least one of processing for converting information on a color of an image captured by the imaging unit from information using a first color space to information using a second color space different from the first color space and processing for converting a file format of the image captured by the imaging unit, and
the determination unit makes a determination by using an image that has been subjected to the image processing by the image processing unit.

31. The information processing method according to any one of claim 25 to claim 30, wherein
the image processing unit performs image processing for trimming the coloring part in the image captured by the imaging unit, and
the determination unit makes a determination by using an image that has been subjected to the image processing by the image processing unit.

32. The information processing method according to any one of claim 25 to claim 31, wherein
the imaging unit captures images of the same subject from respective different image positions, and
an image processing unit selects one image based on respective colors of the plurality of images captured by the imaging unit from the different imaging positions, and
the determination unit makes a determination by using the image selected by the image processing unit.

33. The information processing method according to any one of claim 25 to claim 31, wherein
the imaging unit captures images of the same subject from respective different image positions, and
the determination unit makes a determination for each of the plurality of images captured by the imaging unit from the different image positions and determines whether the positive is indicated based on respective determination results of the plurality of images.

34. The information processing method according to any one of claim 25 to claim 33, wherein
an imaging control unit displays an imaging area whose image is captured by the imaging unit, specifies the coloring area included in the imaging area, and converts a color of an area different from the specified coloring part to a single color,
the imaging unit captures an image that has undergone image processing by the imaging control unit, and
the determination unit makes a determination using the image captured by the imaging unit.

35. The information processing method according to any one of claim 25 to claim 34, wherein
the determination unit determines, based on the degree of positive indication obtained by inputting an image to the determination model, whether the image indicates the positive, indicates negative indicating that the coloring area does not have the test object, or indicates neither the positive nor the negative, which indicates that determination is not possible, and causes the imaging unit to capture an image of the subject again if it is determined that determination is not possible.

36. An information processing system that processes information concerning a test using a test piece that develops a color depending on whether a specimen includes a test object, the system comprising:
an imaging unit that captures an image of the test piece that is a subject so as to include a coloring area, which is colored with the color, of the test piece; and
a determination unit that determines whether the image captured by the imaging unit indicates a positive result indicating that the coloring area has the test object, by using a determination model, wherein
the determination model is a learned model that learns a correspondence relationship between an image and the positive by performing machine learning using a training data set, which is obtained by appending a sign, which indicates whether the coloring area in a training image, which has been captured so as to include the coloring area in the unspecified test piece, indicates the positive, to the training image, and that estimates a degree of positive indication in an input image based on the correspondence relationship, to output a result of the estimation, and
the training image includes a plurality of images of the same subject captured in different imaging environments.

37. The information processing system according to claim 36, further comprising:
a user terminal that has the imaging unit; and
an information processing server that has the determination unit, wherein
the user terminal transmits an image captured by the imaging unit to the information processing server.
